# EUROPEAN PATENT APPLICATION

(11) **EP 2 058 306 A1**
(43) Date of publication of application: **13.05.2009**
(21) Application number: 07021687.4
(22) Date of filing: 08.11.2007
(51) Int. Cl.: C07D 401/12, C07D 401/14, A61K 31/4427, A61P 25/00

(54) **Heteroaryl-substituted 2-pyridinyl-methylamine derivatives**

(71) Applicant: SCHWARZ PHARMA AG, 40789 Monheim/Rhld. (DE)
(72) Inventor: The designation of the inventor has not yet been filed
(74) Representative: Dressen, Frank

(57) **Abstract**

The present disclosure relates to compounds having the general formula I 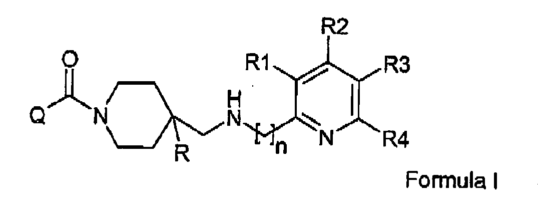 wherein Q represents a five membered heteroaryl group. The compounds are selective 5-HT1a modulators and are useful for treating several diseases.

## Description

The 5-HT1A receptor is a major target for neurobiological research and drug development due to its implication in many (patho)physiological processes. 5-HT1A ligands have been proven in various clinical studies to be effective in pain, anxiety and depression.

5-HT1A ligands may also find use in the treatment of several other diseases such as compulsive-obsessive disorders (Am. J. Psychiatry (1991), 148, 127), schizophrenia, sexual dysfunction (Behavioural Pharmacol. (1995), 6, 276), nausea and vomiting (Pharmacol. Biochem. Behaviour (1989), 33, 627), sleep disorders (Psychopharmacol. (1995), 117, 186), obesity (Int. Clin. Psychopharmacol. (1994), 9, 7), addiction/withdrawal (J. Clin. Psychopharmacol. (1989), 9, 379) and in the treatment of incontinence.

In addition to therapeutic applications in the field of psychiatry, more recent studies have suggested that 5-HT1A receptor ligands have also pronounced neuroprotective properties (EP A 01 544 201, EP A 05 707 057).

Buspirone and tandospirone are launched for the treatment of anxiety disorders in the USA, Western Europe, Japan and China, respectively. PRX-00023 is in advanced clinical studies in anxiety disorders and depression. These three compounds do all belong to the chemical family of arylpiperazines.

F-13640 is a very selective 5-HT1 a agonist which is in clinical studies for pain (Colpaert, Curr Opin. Invest. Drugs (2006), 7, 40), and has the following formula:

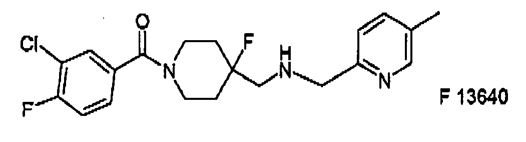

F-13640 is disclosed, inter alia, in EP A 946 546. This document is disclosing 2-pyridinylmethylamine structures of a following general formula which may be summarized as follows: 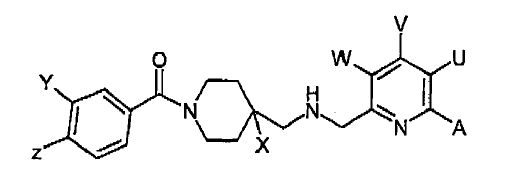 wherein
X can be hydrogen or fluoro,

A, inter alia, can be hydrogen, F, Cl, (C1-C5)alkyl, (C1-C5)alkyloxy, (C1-C5)alkylthio, (C1-C5) mono- or dialkylamino, fluoroalkyl, mono- or trifluormethyloxy, mono- or trifluoromethylthio, (C3-C5)cycloalkyl, (C3-C5)cycloalkyloxy, (C3-C5)cycloalkylthio, alkyloxycarbonyl, or may be selected from a specific list of heteroaryls; as further defined, for example, in claim 1 of EP A 946546.

Vacher et al. (J. Med. Chem. (1998), 41, 5070) have disclosed a series of substituted 2-pyridinylmethylamine derivatives, for example of the following general formula (Table 1 of Vacher et al, supra) 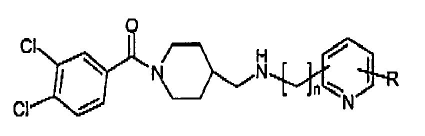 wherein n can be 1-3, and R is a substituent in position 4 or 6 of the pyridine.

Vacher et al disclosed in J. Med. Chem. (1999), 42, 1648, a series of derivatives of 2-pyridinylmethylamine derivatives, for example of the following general formula (Table 7 of Vacher et al, supra) 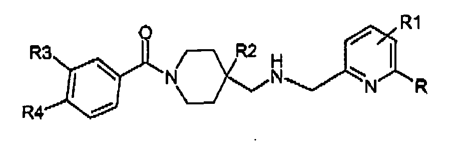 wherein

R can be inter alia hydrogen, certain alkylamines, furanyl, thienyl, oxazol, thiazol, or pyrazol;

R1 can be e.g. hydrogen, fluoro, methyl, ethyl or isopropyl;

R2 may be hydrogen, fluoro, methyl, cyano, methoxy, hydroxyl, or fluoromethyl; and

R3 and R4 may be fluoro, chloro, or methyl.

Besides the high affinity to the 5-HT1 a receptor, the compounds disclosed by Vacher et al also show remarkable specificity over other receptors such as the dopamine D2 or adrenergic alpha 1 receptor (table 5 of Vacher et al (1998); table 2 of Vacher et al (1999); supra).

WO 03/106449 discloses heteroarylmethylaminomethylpiperidin-1-yl-phenyl-methanone derivatives of the following general formula 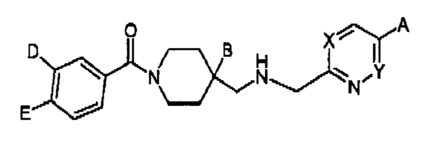 wherein
X and Y are selected from CH and N,
B may be Cl or F,
E is H, F or Cl, and

A and D are selected from a variety of options as disclosed e.g. in claim 1 of WO 03/106449.

Maurel et al (J. Med. Chem. (2007), 50, 5024) very recently disclose further structural derivatives of F-13640. According to the introduction of this publication, the aim of this study was to explore compounds with similar pharmacodynamics but altered pharmacokinetic profiles. The derivatives are based on the variation of the pyridyl ring of F-13640, namely by the further introduction of ring forming nitrogens to form, e.g. a pyrimidine ring. The possibly most general structure proposed by Maurel et al is depicted on page 5026, as follows:

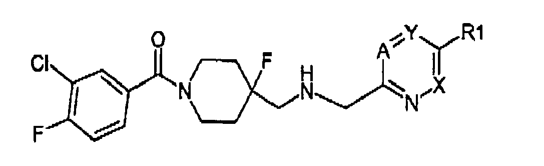

Maurel et al surprisingly found that some derivatives exhibited potential differences compared to F-13640 in terms of stereotypic behaviors and possible serotonin syndrome. They concluded that "there was no simple correlation between affinity, functional activity, and occurrence of these behavioral signs in rats".

In addition to potential differences in affinity, functional activity, occurrence of behavioral signs, and side effects such as the serotonin syndrome, different serotonin agonists may also distinguish with respect to their physicochemical properties, pharmacokinetic profiles, efficacy in certain diseases, toxicity, and/or unwanted side effects. For example, it is well known in the art that some patients do not tolerate certain compounds, and may wish to switch to alternative drugs. Also, dependent on the severity of a given disease, a more or less potent drug may be better suited, respectively. Moreover, some patients may require drugs with a shortened or prolonged elimination half-life. For example, F13640 is disclosed to have a haff-life elimination from plasma of 35 hours (Maurel et al, supra, page 5032, reference (14)) which may be too long in certain cases.

Given the significant therapeutic potential of compounds having 5-HT1 a agonist effects and the surprisingly few 5-HT1 a agonists that are available on the market, or which are at least in advanced clinical trials, the discovery of further 5HT1a agonists is thus highly desirable.

It has been now found, surprisingly, that methylaminomethylpiperidin-1-ylmethanones are still selective and efficient serotonin agonists, if the phenyl group is exchanged by a 5-membered heteroaryl ring.

One aspect of the present disclosure is therefore directed to a compound having the general formula I 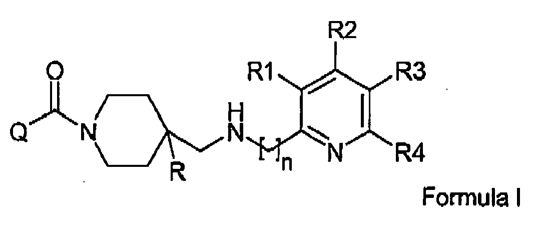 wherein:

n is 1,2 or 3;

R is hydrogen, fluoro, chloro, hydroxyl, cyano;

R1 and R2 are independently hydrogen, fluoro, chloro, methyl;

R3 is hydrogen, (C1-C3)alkyl preferably methyl, fluoromethyl, cyano, hydroxyl, methoxy, fluoro, chloro;

R4 is selected from hydrogen, (C1-C5)alkyl, fluoro(C1- C5)alkyl, cyclo(C3- C5)alkyl, (C1-C5)alkoxy, (C1-C5)alkylthio, (C3-C5)cycloalkyloxy, (C3-C5)cycloalkylthio, (C1-C5)alkoxy, which may contain one or more fluoro substituents; (C1-C5)alkylthio, which may contain one or more fluoro substituents; a group -NRxRy, wherein Rx and Ry are independently hydrogen, (C1-C5)alkyl, (C5-C6)cycloalkyl or (C1-C3)alkyloxycarbonyl; orwherein Rx and Ry are alkyles, which together with the nitrogen to which they are attached form a four to six membered saturated ring;

or a five membered heteroaryl group chosen from furanyl, pyrrolyl, thienyl, pyrazolyl, imidazolyl, oxazolyl, isoxazolyl, thiazolyl, isothiazolyl, triazolyl, oxadiazolyl, tetrazolyl;

### Q represents a

five membered heteroaryl group, which is
(i) substituted with one, two or three residues R5, R6 and R7; or
(ii) substituted with one residue R5, and annelated to a second five or six membered ring, which is aromatic or heteroarylic, wherein said second ring is substituted with one or two residues R8 and R9; or

(i) substituted with one residue R5, and is annelated to a second five, six or seven membered ring, which is non-aromatic, may contain one or two heteroatoms, and is substituted with one or two residues R10 and R11;
wherein every substituent R5, R6, R7, R8, R9, R10 and R11 is bound to a ring forming carbon atom;
and wherein any heteroarylic or non-aromatic ring which contains one or more ring forming nitrogens, may be substituted at one or more of said ring forming nitrogens with a residue R12.

R5, R6, R7, R8 and R9 are independently of each other selected from the group consisting of hydrogen, hydroxyl, formyl, oxime, cyano, nitro, NR13R14, carboxy, carbamoyl, alkyl preferably (C1-C6)alkyl, cycloalkyl preferably (C3-C6)cycloalkyl, alkyloxy preferably (C1-C6)alkyloxy, alkylthio preferably (C1-C6)alkylthio, alkenyl preferably (C2-C6)alkenyl, alkynyl preferably (C2-C6)alkynyl, phenyl, heteroaryl, phenoxy, halogen, trifluoromethyl, alkylcarbonyl preferably (C1-C6)alkylcarbonyl, alkylaminocarbonyl preferably (C1-C6)alkylaminocarbonyl, dialkylaminocarbonyl preferably di(C1-C6)alkylaminocarbonyl, phenylcarbonyl, phenylalkyl preferably phenyl(C1-C6)alkyl, phenylalkyloxy preferably phenyl(C1-C6)alkyloxy, phenylalkylcarbonyl preferably phenyl(C1-C6)alkylcarbonyl, phenylalkyloxycarbonyl preferably phenyl(C1-C6)alkyloxycarbonyl, alkyloxycarbonyl preferably (C1-C6)alkyloxycarbonyl, alkylsulfonyl preferably (C1-C6)alkylsulfonyl, phenylsulfonyl, sulfamoyl, alkylaminosulfonyl preferably (C1-C6)alkylaminosulfonyl, dialkylaminosulfonyl preferably di(C1-C6)alkylaminosulfonyl, phenylsulfonylamino, and alkylsulfonylamino preferably (C1-C6)alkylsulfonylamino;
wherein each alkyl, alkenyl, or alkynyl may be unsubstituted or substituted with one or more residues selected from among hydroxyl, (C1-C6)alkyloxy preferably (C1-C3)alkyloxy, halogen, and NR13R14; and wherein each heteroaryl is a monocyclic ring and wherein each phenyl or heteroaryl can be unsubstituted or substituted with one or more residues selected from among hydroxyl, (C1-C6)alkyloxy preferably (C1-C3)alkyloxy, halogen, (C1-C6)alkyl preferably (C1-C3)alkyl, (C3-C6)cycloalkyl preferably (C5-C6)cycloalkyl, carboxy, NR13R14, cyano, trifluoromethyl and nitro;

R10 and R11 are independently selected from the group consisting of hydrogen, hydroxyl, alkyl preferably (C1-C6)alkyl, phenyl, halogen, NR13R14 and oxo, wherein each alkyl may be unsubstituted or substituted with one or more residues selected from among hydroxyl, (C1-C6)alkyloxy preferably (C1-C3)alkyloxy, halogen, and NR13R14;

R12 is hydrogen, alkyl preferably (C1-C6)alkyl, cycloalkyl preferably (C3-C6)cycloalkyl, phenyl, phenylalkyl preferably phenyl(C1-C3)alkyl, alkylcarbonyl preferably (C1-C5)alkylcarbonyl, phenylcarbonyl, phenylalkyloarbonyl preferably phenyl(C1-C3)alkylcarbonyl and phenylsulfonyl, wherein each phenyl ring may be substituted with one or two substituents selected from among fluoro, chloro, methyl and methoxy;

R13 and R14 are independently selected from hydrogen, alkyl preferably (C1-C5)alkyl, (C3-C6)cycloalkyl preferably (C5-C6)cycloalkyl and (C1-C6)alkyloxycarbonyl preferably (C1-C3)alkyloxycarbonyl or R13 and R14 are alkylenes, which together with the nitrogen to which they are attached form a four to six membered saturated ring,

and pharmaceutically acceptable salts and/or isomers or racemates thereof..

In one embodiment of the present disclosure, in a compound of formula I, R is preferably fluoro.

Another embodiment of the present disclosure is a compound of formula I, wherein R1, R2 and R4 are all hydrogen.

Another embodiment of the present disclosure is a compound of formula I, wherein R3 is preferably methyl or chloro.

Another embodiment of the present disclosure is a compound of formula I, wherein R is fluoro, R1 and R2 are both hydrogen, R3 is methyl or chloro, and R4 is hydrogen or (C1-C3)alkyl.

Another embodiment of the present disclosure is a compound of formula I and having the general formula Ib 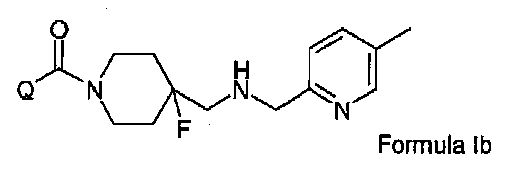 wherein Q is as defined further above, and a pharmaceutically acceptable salt and/or isomer or racemate thereof.

Another aspect of the present disclosure is a compound of formula I or Ib as described above, wherein Q is a five membered heteroaryl selected from among furanyl, pyrrolyl, thienyl, pyrazolyl, imidazolyl, oxazolyl, isoxazolyl, thiazolyl, isothiazolyl, triazolyl, oxadiazolyl, thiadiazolyl and tetrazolyl, which is substituted with one or two residues R5 and R6 which are independently selected from hydrogen, hydroxyl, formyl, cyano, oxime, (C1-C6 alkyl) preferably (C1-C4)alkyl, (C1-C6)alkyloxy preferably (C1-C4)alkyloxy, (C1-C6)alkylthio preferably (C1-C4)alkylthio, carboxy, (C1-C5)alkylcarbonyl preferably (C1-C3)alkylcarbonyl, (C1-C5)alkyloxycarbonyl preferably (C1-C3)alkyloxycarbonyl, chloro, bromo, iodo, hydroxymethyl, trifluoromethyl, (C1-C6)alkylaminocarbonyl preferably (C1-C4)alkylaminocarbonyl, di(C1-C6)alkylaminocarbonyl preferably di(C1-C4)alkylaminocarbonyl, nitro, NR13R14; and wherein in the case of pyrrolyl, pyrazolyl, imidazolyl and triazolyl one nitrogen atom is substituted with R12 as defined in claim 1, and wherein R13 and R14 are independently selected from hydrogen, (C1-C3)alkyl, (C5-C6)cycloalkyl and (C1-C3)alkyloxycarbonyl or R13 and R14 are alkylenes, which together with the nitrogen to which they are attached form a four to six membered saturated ring. In these compounds, R12 is in one preferred aspect hydrogen.

Another aspect of the present disclosure is a compound of formula I or Ib as described above, wherein Q is a five membered heteroaryl selected from among furanyl, pyrrolyl, thienyl, pyrazolyl, imidazolyl, oxazolyl, isoxazolyl, thiazolyl, isothiazolyl, triazolyl, oxadiazolyl, thiadiazolyl and tetrazolyl, which is substituted with one or two residues R5 and R6 which are independently selected from hydrogen, hxdroxyl, formyl, cyano, oxime, (C1-C3)alkyl, (C1-C3)alkyloxy, (C1-C3)alkylthio, (C1-C3)alkylcarbonyl, (C1-C3)alkyloxycarbonyl, chloro, bromo, iodo, hydroxymethyl, trifluoromethyl, (C1-C3)alkylaminocarbonyl, di(C1-C3)alkylaminocarbonyl, nitro, NR13R14; and wherein in the case of pyrrolyl, pyrazolyl, imidazolyl and triazolyl one nitrogen atom is substituted with R12 and wherein
- R12 is selected from hydrogen, (C1-C3)alkyl, phenyl(C1-C3)alkyl, (C1- C3)alkylcarbonyl, phenylcarbonyl and phenylsulfonyl; and
- R13 and R14 are independently selected from hydrogen, (C1-C3)alkyl, (C5-C6)cycloalkyl and (C1-C3)alkyloxycarbonyl or R13 and R14 are alkylenes, which together with the nitrogen to which they are attached form a four to six membered saturated ring. In these compounds, R12 is in one preferred aspect hydrogen.

Another embodiment of the present disclosure is a compound according to formula I or Ib, wherein Q represents a group Q2: 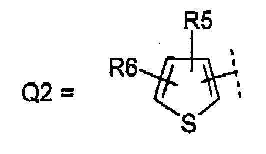 wherein
the bond crossed by the dotted line indicates the attachment position such that the compounds have the general formula II or IIb: 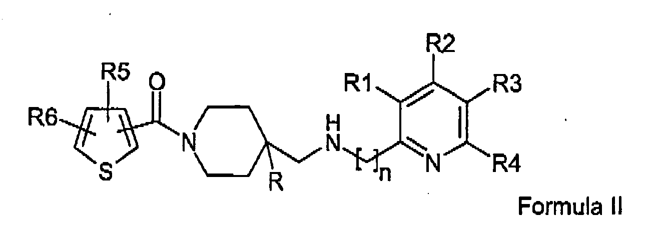 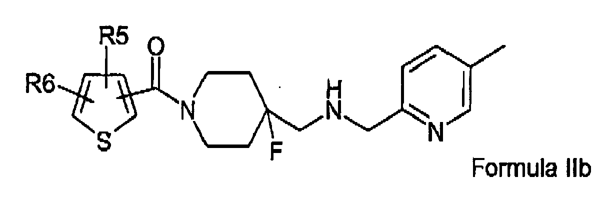 wherein
n, R, R1, R2, R3, R4, R5 and R6 are as defined for formula I and Ib further above; and
wherein R5 and R6 are in a particular embodiment independently selected from hydrogen, fluoro, chloro, bromo, iodo, (C1-C3)alkyl, (C1-C3)alkyloxy, methylthio, and phenyl.

Another embodiment of the present disclosure relates to compounds of formula I or Ib,
wherein Q is a five membered heteroaryl selected from among furanyl, pyrrolyl, thienyl, pyrazolyl, imidazolyl, oxazolyl, isoxazolyl, thiazolyl, isothiazolyl and triazolyl, which is annelated to a second five or six membered ring, which is aromatic or heteroarylic,
wherein said second ring is substituted with one or two residues R8 and R9, as further defined elsewhere in this specification.

Another aspect of the present disclosure is a compound of formula I or Ib, wherein Q is a thienyl, furanyl, pyrrolyl, or pyrazolyl, which carries one substituent R5, and which is annelated to a second ring selected from the group phenyl, thienyl, pyridyl, which second ring is substituted with one or two residues R8 and R9, wherein R5, R8 and R9 are independently selected from hydrogen, hydroxyl, formyl, cyano, oxime, (C1-C4)alkyl, (C2-C4)alkenyl, (C2-C4)alkynyl, (C1-C4)alkyloxy, (C1-C4)alkylthio, carboxy, (C1-C3)alkylcarbonyl, (C1-C3)alkyloxycarbonyl, chloro, bromo, iodo, hydroxymethyl, trifluoromethyl, (C1-C4)alkylaminocarbonyl, di(C1-C5)alkylaminocarbonyl, nitro, and NR13R14, wherein R13 and R14 are independently selected from hydrogen, (C1-C3)alkyl, (C5-C6)cycloalkyl and (C1-C3)alkyloxycarbonyl or R13 and R14 are alkylenes, which together with the nitrogen to which they are attached form a four to six membered saturated ring.

Another embodiment of the present disclosure relates to compounds of formula I or Ib,
wherein Q is thienyl or furanyl which is annelated to a second aromatic or heteroarylic ring, and forming a ring system selected from one of the formula Q3-Q5: 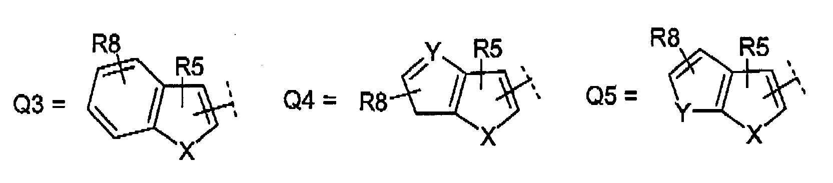 such that the compounds have the general formula III, IIIb, IV, IVb, V and Vb, respectively. 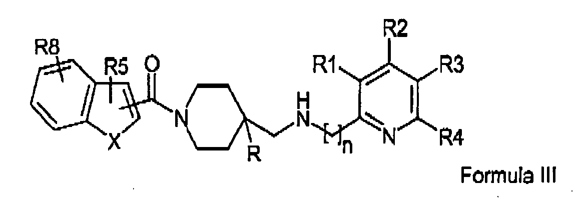 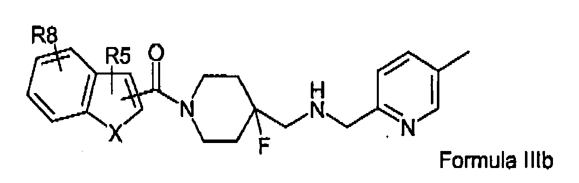 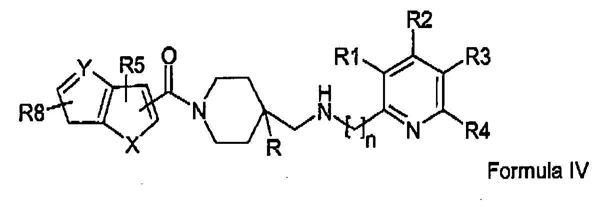 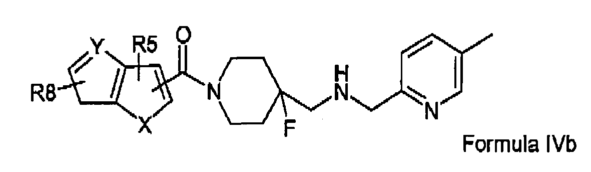 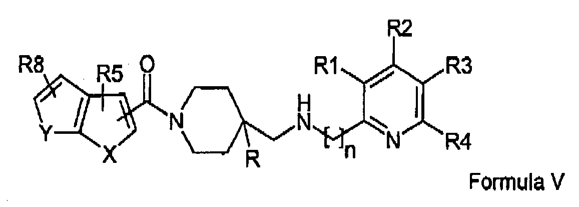 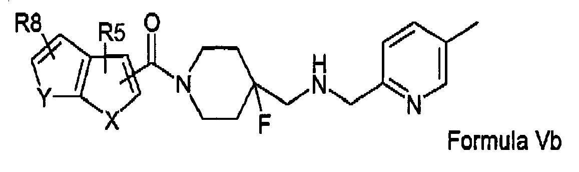 wherein:
n, R, R1, R2, R3, R4, R5 and R8 are as defined for formula I and Ib further above,
   X is S or O; and
   Y is S or O, and is preferably S;
wherein in one embodiment R5 and R8 are preferably independently selected from hydrogen, fluoro, chloro, and bromo.

Another embodiment of the present disclosure relates to compounds of formula I or Ib,
wherein Q is a thienyl or furanyl which is annelated with a phenyl and in which Q is attached in position 3 of the heteroarene, thus forming a ring system according to formula
Q6: 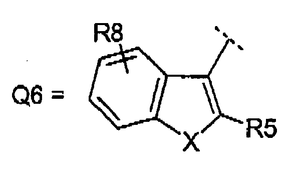 such that the compounds have the general formula VI or VIb as follows: 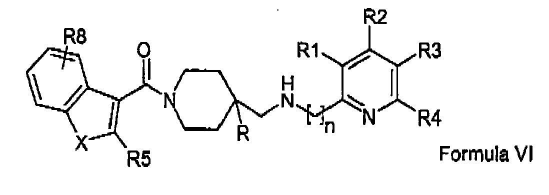 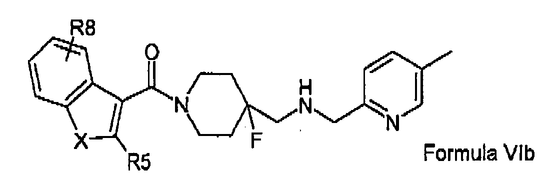 wherein:
n, R, R1, R2, R3, R4, R5 and R8 are as defined for formula I and Ib further above, and X is S or O;
wherein in one embodiment R5 and R8 are preferably independently selected from hydrogen, fluoro, chloro, and bromo, wherein in one particular embodiment R5 is hydrogen.

Another embodiment of the present disclosure relates to compounds of formula I or Ib,
wherein Q is a thienyl or furanyl, which carries one substituent R5, and which is annelated to a second ring selected from the group cyclopentyl, cyclohexyl, or a saturated five or six membered ring which contains one or more heteroatoms selected from N, S and O,
wherein said N is substituted with R12, and wherein
- said second ring carries one substituent R10;
- R5 is selected from hydrogen, hydroxyl, formyl, cyano, oxime, (C1-C4)alkyl, (C2-C4)alkenyl, (C2-C4)alkynyl, (C1-C4)alkyloxy, (C1-C4)alkylthio, carboxy, (C1-C3)alkylcarbonyl, (C1-C3)alkyloxycarbonyl, chloro, bromo, iodo, hydroxymethyl, trifluoromethyl, (C1-C4)alkylaminocarbonyl, di(C1-C4)alkylaminocarbonyl, nitro, and NR13R14;
- R10 is selected from the group consisting of hydrogen, hydroxyl, (C1-C3)alkyl, phenyl, halogen, NR13R14 and oxo, wherein each alkyl may be unsubstituted or substituted with one or more residues selected from among hydroxyl, (C1-C3)alkyloxy, halogen, and NR13R14
- R12 is selected from hydrogen, (C1-C5)alkyl, phenyl(C1-C3)alkyl, (C1-C5)alkylcarbonyl, phenylcarbonyl and phenylsulfonyl; and
- R13 and R14 are Independently selected from hydrogen, (C1-C3)alkyl, (C5-C6)cycloalkyl and (C1-C3)alkyloxycarbonyl or R13 and R14 are alkylenes, which together with the nitrogen to which they are attached form a four to six membered saturated ring.

Another embodiment of the present disclosure relates to compound of formula I or Ib, wherein Q is a thienyl or furanyl which is annelated to a second ring, thus forming a ring system according to formula Q7. 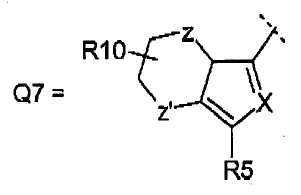 such that the compounds have the general formula VII or Vllb as follows: 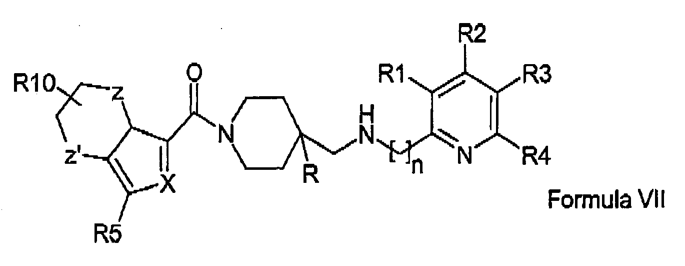 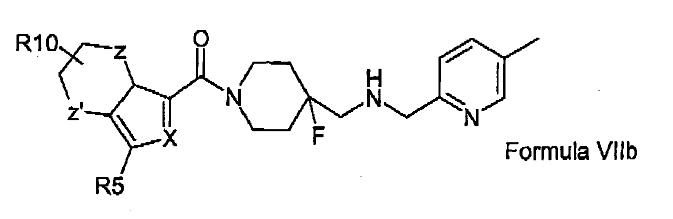 wherein:
n, R, R1, R2, R3, R4, R5 and R10 are as defined for formula I and Ib further above,
X is S or O, and
Z and Z' are independently CH₂ or O;
wherein in one embodiment R5 and R10 are preferably hydrogen, chloro or bromo, and preferably chloro and hydrogen.

The compounds of the present disclosure may exist as optical isomers such as enantiomers or, in the case of two or more stereocenters, as diasteromers, or mixtures of optical isomers, including racemates. Unless expressly stated otherwise, all possible optical forms are considered to be part of the present disclosure.

Particular compounds according to the present disclosure are:
- [4-Fluoro-4-[(5-methylpyridin-2-ylmethyl)amino]methyl]piperidin-1-yl]-thien-2-yl-methanone
- [4-Fluoro-4-[(5-methylpyridin-2-ylmethyl)amino]methyl]piperidin-1-yl]-5-fluorothien-2-yl-methanone
- 3-Chlorothien-2-yl-[4-fluoro-4-[[(5-methylpyridin-2-ylmethyl)amino]methyl]-piperidin-1-yl]-methanone
- 5-Chlorothien-2-yl-[4-fluoro-4-[[(5-methylpyridin-2-ylmethyl)amino]methyl]-piperidin-1-yl]-methanone
- 3-Bromothien-2-yl-[4-fluoro-4-[[(5-methylpyridin-2-ylmethyl)amino]methyl]-piperidin-1-yl]-methanone
- 4-Bromothien-2-yl-[4-fluoro-4-[[(5-methylpyridin-2-ylmethyl)amino]methyl]-piperidin-1-yl]-methanone
- 5-Bromothien-2-yl-[4-fluoro-4-[[(5-methylpyridin-2-ylmethyl)amino]methyl]-piperidin-1-yl]-methanone
- 4,5-Dibromothien-2-yl-[4-fluoro-4-[[(5-methylpyridin-2-ylmethyl)amino]methyl]-piperidin-1-yl]-methanone
- [4-Fluoro-4-[[(5-methylpyridin-2-ylmethyl)amino]methyl]piperidin-1-yl]-3-methylthien-2-yl-methanone
- [4-Fluoro-4-[[(5-methylpyridin-2-ylmethyl)amino]methyl]piperidin-1-yl]- 4-methylthien-2-yl-methanone
- [4-Fluoro-4-[[(5-methylpyridin-2-ylmethyl)amino]methyl]piperidin-1-yl]-5-methylthiophen-2-yl-methanone
- [4-Fluoro-4-[[(5-methylpyridin-2-ylmethyl)aminolmethyl]piperldin-1-yl]-5-methylthiothien-2-yl-methanone
- [4-Fluorp-4-[[(5-methylpyridin-2-ylmethyl)amino]methyl]piperidin-1-yl]-5-phenylthien-2-yl-methanone
- 14-Fluoro-4-[[(5-methylpyridin-2-ylmethyl)amino]methyl]piperidin-1-yl]-5-pyridin-2-ylthien-2-yl-methanone
- [2,2']Bithien-5-yl-[4-fluoro-4-[[(5-methylpyridin-2-ylmethyl)amino]methyl]-piperidin-1-yl]-methanone
- [4-Fluoro-4-[[(5-methylpyridin-2-ylmethyl)amino]methyl]piperidin-1-yl]-[5-(2-methylthiazol-4-yl)thien-2-yl]-methanone
- [4-Fluoro-4-[[(5-methylpyridin-2-ylmethyl)amino]methyl]piperidin-1-yl]-3-pyrrol-1-ylthien-2-yl-methanone
- [4-Fluoro-4-[[(5-methylpyridin-2-ylmethyl)amino]methyl]piperidin-1-yl]-thien-3-yl-methanone
- 2-Bromothien-3-yl-[4-fluoro-4-[[(5-methylpyridin-2-ylmethyl)amino]methyl]-piperidin-1-yl]-methanone
- [4-Fluoro-4-[[(5-methylpyridin-2-ylmethyl)amino]methyl]piperidin-1-yl]-4-methoxythien-3-yl-methanone
- 5-Chloro-4-methoxythien-3-yl-[4-fluoro-4-[[(5-methylpyridin-2-ylmethyl)amino]methyl]piperidin-1-yl]-methanone
- 5-Bromo-4-methoxythien-3-yl-[4-fluoro-4-[[(5-methylpyridin-2-ylmethyl)amino]methy]piperidin-1-yl]-methanone
- Benzo[b]thien-2-yl-[4-fluoro-4-[[(5-methylpyddin-2-ylmethyl)amino]methyl]-piperidin-1-yl]-methanone
- 5-Bromobenzo[b]thien-2-yl-[4-fluoro-4-[[(5-methylpyridin-2-ylmethyl)amino]methyl]piperidin-1-yl]-methanone
- Benzo[b]thien-3-yl-[4-fluoro-4-[[(5-methylpyridin-2-ylmethyl)amino]methyl]piperidin-1-yl]-methanane
- [4-Fluoro-4-[[(5-methylpyridin-2-ylmethyl)amino]methyl]piperidin-1-yl]-thieno[3,2-b]thien-2-yl-methanone
- [4-Fluoro-4-[[(5-methylpyridin-2-ylmethyl)amino]methyl]piperdin-1-yl]-thieno[2,3-b]thien-2-yl-methanone
- 3-Chlorothieno[2,3-b]thien-2-yl-[4-fluoro-4-[[(5-methylpyridin-2-ylmethyl)amino]methyl]piperidin-1-yl]-methanone
- [4-Fluoro-4-[[(5-methylpyridin-2-ylmethyl)amino]methyl]piperidin-1-yl]-4,5,6,7-tetrahydrobenzo[c]thien-1-yl-methanone
- 7-Bromo-2,3-dihydrothieno[3,4-b][1,4]dioxin-5-yl-[4-fluoro-4-[[(5-methylpyridin-2-ylmethyl)amino]methyl]piperidin-1-yl]-methanone
- [4-Fluoro-4-[[(5-methylpyridin-2-ylmethyl)amino]methyl]pipeddin-1-yl]-furan-2-yl-methanone
- 4,5-Dibromofuran-2-yl-[4-fluoro-4-[[(5-methylpyridin-2-ylmethyl)amino]methyl]pipeddin-1-yl]-methanone
- [4-Fluoro-4-[[(5-methylpyridin-2-ylmethyl)amino]methyl]piperidin-1-yl]-furan-3-yl-methanone
- 5-Bromofuran-3-yl-[4-fluoro-4-[[(5-methylpyridin-2-ylmethyl)amino]methyl]piperidin-1-yl]-methanone
- Benzo[b]furan-2-yl-[4-fluoro-4-[[(5-methylpyridin-2-ylmethyl)amino]methyl]piperidin-1-yl]-methanone
- Benzo[b]furan-3-yl-[4-fluoro-4-[[(5-methylpyridin-2-ylmethyl)amino]methyl]piperidin-1-yl]-methanone
- [4-Fluoro-4-[[(5-methylpyridin-2-ylmethyl)amino]methyl]piperidin-1-yl]-pyrrol-2-yl-methanone
- [4-Fluoro-4-[[(5-methylpyridin-2-ylmethyl)amino]methyl]piperidin-1-yl]-pyrazol-3-yl-methanone
- [4-Fluoro-4-[[(5-mathylpyridin-2-ylmethyl)amino]methyl]piperidin-1-yl]-indol-2-yl-methanone
- [4-Fluoro-4-[[(5-methylpyridin-2-ylmethyl)amino]methyl]piperidin-1-yl]-pyrazolo[1,5-a]pyridin-2-yl-methanone
- [4-Fluoro-4-[[(5-methylpyridin-2-ylmethyl)amino]methyl]piperidin-1-yl]-pyrazolo[1,5-a]pyridin-3-yl-methanone

Another aspect of the present disclosure is a compound selected from the following group:
- [4-Fluoro-4-[(5-methylpyridin-2-ylmethyl)amino]methyl]piperidin-1-yl]-thien-2-yl-methanone
- [4-Fluoro-4-[(5-methylpyridin-2-ylmethyl)amino]methyl]piperidin-1-yl]-5-fluorothien-2-yl-methanone
- 3-Chlorothien-2-yl-[4-fluoro-4-[[(5-methylpyridin-2-ylmethyl)amino]methyl]-piperidin-1-yl]methanone
- 5-Chlorothien-2-yl-[4-fluoro-4-[[(5-methylpyridin-2-ylmethyl)amino]methyl]-piperidin-1-yl]-methanone
- 3-Bromothien-2-yl-[4-fluoro-4-[[(5-methylpyridin-2-ylmethyl)amino]methyl]-piperidin-1-yl]-methanone
- 4-Bromothien-2-yl-[4-fluoro-4-[[(5-methylpyridin-2-ylmethyl)amino]methyl]-piperidin-1-yl]-methanone
- 5-Bromothien-2-yl-[4-fluoro-4-[[(5-methylpyridin-2-ylmethyl)amino]methyl]-piperidin-1-yl]-methanone
- 4,5-Dibromothien-2-yl-[4-fluoro-4-[[(5-methylpyridin-2-ylmethyl)amino]methyl]-piperidin-1-yl]-methanone
- [4-Fluoro-4-[[(5-methylpyridin-2-ylmethyl)amino]methyl]piperidin-1-yl]-3-methylthien-2-yl-methanone
- [4-Fluoro-4-[[(5-methylpyridin-2-ylmethyl)amino]methyl]piperidin-1-yl]- 4-methylthien-2-yl-methanone
- [4-Fluoro-4-[[(5-methylpyridin-2-ylmethyl)amino]methyl]piperidin-1-yl]-5-methylthiophen-2-y1-methanone
- [4-Fluoro-4-[[(5-methylpyridin-2-ylmethyl)amino]methyl]piperidin-1-yl]- 5-methylthiothien-2-yl-methanone
- [4-Fluoro-4-[[(5-methylpyridin-2-ylmethyl)amino]methyl]piperidin-1-yl]-thien-3-yl-methanone
- 2-Bromothien-3-yl-[4-fluoro-4-[[(5-methylpyridin-2-ylmethyl)amino]methyl]-piperidin-1-yl]-methanone
- 5-Chloro-4-methoxythien-3-yl-[4-fluoro-4-[[(5-methylpodin-2-ylmethyl)amino]methyl]piperidin-1-yl]-methanone
- 6-Bromo-4-methoxythien-3-yl-[4-fluoro-4-[[(5-methylpyridin-2-ylmethyl)amino]methyl]piperidin-1-yl]-methanone
- Benzo[b]thien-3-yl-[4-fluoro-4-[[(5-methylpyridin-2-ylmethyl) amino]methyl]piperidin-1-yl]-methanone
- [4-Fluoro-4-[[(5-methylpyridin-2-ylmethyl)amino]methyl]piperidin-1-yl]-thieno[3,2-b]thien-2-yl-methanone
- [4-Fluoro-4-[[(5-methylpyridin-2-ylmethyl)amino]methyl]piperidin-1-yl]-thieno[2,3-b]thien-2-yl-methanane
- 3-Chlorothieno[2,3-b]thion-2-yl-[4-fluoro-4-[[(5-methylpyridin-2-ylmethyl)amino]methyl]piperidin-1-yl]-methanone
- [4-Fluoro-4-[[(5-methylpyridin-2-ylmethyl)amino]methyl]pipeddin-1-yl]-4,5,6,7-tetrahydrobenzo[c]thien-1-yl-methanone
- 7-Bromo-2,3-dihydrothieno[3,4-b][1,4]dioxin-5-yl-[4-fluoro-4-[[(5-methylpyridin-2-ylmethyl)amino]methyl]pipeddin-1-yl]-methanone
- 4,5-Dibromofuran-2-yl-[4-fluoro-4-[[(5-methylpyridin-2-ylmethyl)amino]methyl]piperidin-1-yl]-methanone
- 5-Bromofuran-3-yl-[4-fluoro-4-[[(5-mothylpyridin-2-ylmethyl)amino]methyl]piperidin-1-yl]-methanone and
- Benzo[b]furan-3-yl-[4-fluoro-4-[[(5-methylpyridin-2-ylmethyl)amino]methyl]piperidin-1-yl]-methanone.

Other examples of compounds of the current inventions are:
- 5-Bromobenzo[b]thien-3-yl-[4-fluoro-4-[[(5-methylpyridin-2-ylmethyl)amino]methyl]piperidin-1-yl]-methanone
- 5-Chlorobenzo[b]thien-3-yl-[4-fluoro-4-[[(5-methylpyridin-2-ylmethyl)amino]methyl]piperidin-1-yl]-methanone
- 5-Ethoxy-2-methylbenzo[b]furan-3-yl-[4-fluoro-4-[[(5-methylpyridin-2-ylmethyl)amino]methyl]piperidin-1-yl]-methanone
- [4-Fluoro-4-[[(5-methylpyridin-2-ylmethyl)amino]methyl]piperidin-1-yl]-5-methoxybenzo[b]furan-3-yl-methanone
- [4-Fluoro-4-[[(5-methylpyridin-2-ylmethyl)amino]methyl]piperidin-1-yl]-5-phenylsulfonyloxybenzo[b]thiophene-3-yl-methanone
- [4-Fluoro-4-[[(5-methylpyridin-2-ylmethyl)amino]methyl]piperidin-1-yl]-indol-3-yl-methanone
- 4-Bromoindol-3-yl-[4-fluoro-4-[[(5-methylpyridin-2-ylmethyl)amino]methyl]piperidin-1-yl]-methanone
- 5-Chloroindol-3-yl-[4-fluoro-4-[[(5-methylpyridin-2-ylmethyl)amino]methyl]piperidin-1-yl]-methanone
- 6-Bromoindol-3-yl-[4-fluoro-4-[[(5-methylpyridin-2-ylmethyl)amino]methyl]piperidin-1-yl]-methanone
- [4-Fluoro-4-[[(5-methylpyridin-2-ylmethyl)amino]methyl]piperidin-1-yl]-5-methylindol-3-yl-methanone
- [4-Fluoro-4-[[(5-methylpyridin-2-ylmethyl)amino]methyl]piperidin-1-yl]-5-methoxylindol-3-yl-methanone
- 6-Fluoroindol-3-yl-[4-fluoro-4-[[(5-methylpyridin-2-ylmethyl)amino]methyl]piperidin-1-yl]-methanone
- 7-Bromoindol-3-yl-[4-fluoro-4-[[(5-methylpyridin-2-ylmethyl)amino]methyl]piperidin-1-yl]-methanone
- [4-Fluoro-4-[[(5-mothylpyridin-2-ylmethyl)aminolmethyl]piperdin-1-yl]-7-methylindol-3-yl-methanone
- 7-Azaindol-3-yl-[4-fluoro-4-[[(5-methylpyridin-2-ylmethyl)amino]methyl]piperidin-1-yl]-methanone
- [4-Fluoro-4-[[(5-methylpyridin-2-ylmethyl)amino]methyl]piperidin-1-yl]-indolizin-1-yl-methanone
- [4-Fluoro-4-[[(5-methylpyridin-2-ylmethyl)amino]methyl]piperidin-1-yl]-indolizin-2-yl-methanone
- [4-Fluoro-4-[[(5-methylpyridin-2-ylmethyl)amino]methyl]piperidin-1-yl]-imidazol-2-yl-methanone
- [4-Fluoro-4-[[(6-methylpyridin-2-ylmethyl)amino]methyl]piperidin-1-yl]-4-methyl-1 H-imidazol-2-yl-methanone
- [4-Fluoro-4-[[(5-methylpyridin-2-ylmethyl)amino]methyl]piperidin-1-yl]-imidazol-4-yl-methanone
- 6-Chloroimidazo[1,2-a]pyridin-2-yl-[4-fluoro-4-[[(5-methylpyridin-2-ylmethyl)amino]methyl]piperidin-1-yl]-methanone
- 6-Chloro-2-methylimidazo[1,2-a]pyridin-3-yl-[4-fluoro-4-[[(5-methylpyridin-2-ylmethyl)amino]methyl]piperidin-1-yl]-methanone
- [4-Fluoro-4-[[(5-methylpyridin-2-ylmethyl)amino]methyl]piperidin-1-yl]-oxazol-2-yl-methanone
- 4-Bromooxazol-2-yl-[4-fluoro-4-[[(5-methylpyridin-2-ylmethyl)amino]methyl]pipeddin-1-yl]-methanone
- [4-Fluoro-4-[[(5-methylpyridin-2-ylmethyl)amino]methyl]piperidin-1-yl]-oxazol-5-yl-methanone
- [4-Fluoro-4-[[(5-methylpyridin-2-ylmethyl)amino]methyl]piperidin-1-yl]-4-methyloxazol-5-yl-methanone
- [4-Fluoro-4-[[(5-methylpyridin-2-ylmethyl)amino]methyl]piperidin-1-yl]-thiazol-2-yl-methanone
- [4-Fluoro-4-[[(5-methylpyridin-2-ylmethyl)amino]methyl]piperidin-1-yl]-4-methylthiazol-2-yl-methanone
- [4-Fluoro-4-[[(5-methylpyridin-2-ylmethyl)amino]methyl]piperidin-1-yl]-4-isopropylthiazol-2-yl-methanone
- [4-Fluoro-4-[[(5-mothylpyridin-2-ylmethyl)amino]methyl]piperidin-1-yl]-4-trifluoromethylthiazol-2-yl-methanone
- [4-Fluoro-4-[[(5-methylpyridin-2-ylmethyl)amino]methyl]piperidin-1-yl]-thiazol-5-yl-methanone
- 2-Bromo-4-methylthiazol-5-yl-[4-fluoro-4-[[(5-methylpyridin-2-ylmethyl)amino]methyl]piperidin-1-yl]-methanone
- 5-Cyanoisothiazol-3-yl-[4-fluoro-4-[[(5-methylpyridin-2-ylmethyl)amino]methyl]piperidin-1-yl]-methanone
- 1,2-Benzisothiazol-3-yl-[4-fluoro-4-[[(5-methylpyridin-2-ylmethyl)amino]methyl]piperidin-1-yl]-methanone
- [4-Fluoro-4-[[(5-methylpyridin-2-ylmethyl)amino]methyl]piperidin-1-yl]-isoxazol-5-yl-methanone
- 1,2-Benzisoxazol-3-yl-[4-fluoro-4-[[(5-methylpyridin-2-ylmethyl)amino]methyl]piperidin-1-yl]-methanone
- [4-Fluoro-4-[[(6-methylpyridin-2-ylmethyl)amino]methyl]piperidin-1-yl]-1,2,5-thiadiazol-3-yl-methanone
- [4-Fluoro-4-[[(5-methylpyridin-2-ylmethyl)amino]methyl]piperidin-1-yl]-4-methyl-1,2,5-thiadiazol-3-yl-methanone
- [4-Fluoro-4-[[(5-methylpyridin-2-ylmethyl)amino]methyl]piperidin-1-yl]-1,2,5-oxadiazol-3-yl-methanone
- [4-Fluoro-4-[[(5-methylpyridin-2-ylmethyl)amino]methyl]piperidin-1-yl]-4-methylfurazan-3-yl-methanone
- [4-Fluoro-4-[[(5-methylpyridin-2-ylmethyl)amino]methyl]piperidin-1-yl]-1,3,4-oxadiazol-2-yl-methanone
- [4-Fluoro-4-[[(5-methylpyridin-2-ylmethyl)amino]methyl]piperidin-1-yl]-5-methyl-1,3,4-oxadiazol-2-yl-methanone
- [4-Fluoro-4-[[(5-methylpyridin-2-ylmethyl)amino]methyl]piperidin-1-yl]-5-trifluoromethyl-1,3,4-oxadiazol-2-yl-methanone
- [4-Fluoro-4-[[(5-methylpyridin-2-ylmethyl)amino]methyl]piperidin-1-yl]-5-methyl-1,3,4-thiadiazol-2-yl-methanone
- [4-Fluoro-4-[[(5-methylpyridin-2-ylmethyl)amino]methyl]piperidin-1-yl]-3H-1,2,3-triazol-4-yl-methanone
- [4-Fluoro-4-[[(5-methylpyridin-2-ylmethyl)amino]methyl]piperidin-1-yl]-5-methyl-2H-1,2,3-triazol-4-yl-methanone
- [4-Fluoro-4-[[(5-methylpyridin-2-ylmethyl)amino]methyl]piperidin-1-yl]-4H-1,2,4-triazol-3-yl-methanone

The compounds according to the present disclosure can be produced as follows:

A compound of formula I can be synthesized starting with the benzoylation of 1,4-dioxa-8-azaspiro[4.5]decane (formula A1) and subsequent deprotection of the acetale (formula A2) to get the precursor 1-benzoyl-4-piperidinone (formula A3), which is reacted to the oxirane derivative as a common intermediate according to formula A4.

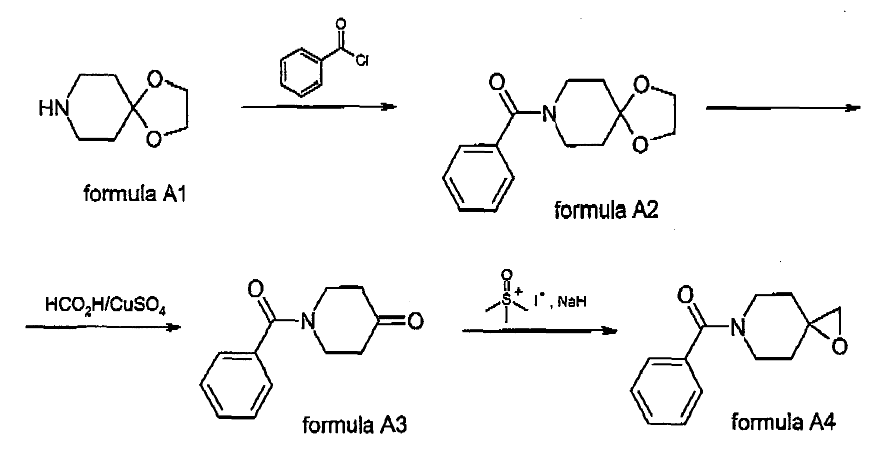

The oxirane ring system of the common intermediate according formula A4 can be opened by reaction with a nucleophilic reagent of type X⁺R⁻ or X-R to obtain a primary alcohol (A5a), which can be activated as a trifluoromethane sulfonic acid ester (A5b) and subsequently reacted with sodium azide to get an azide derivative according to formula A5 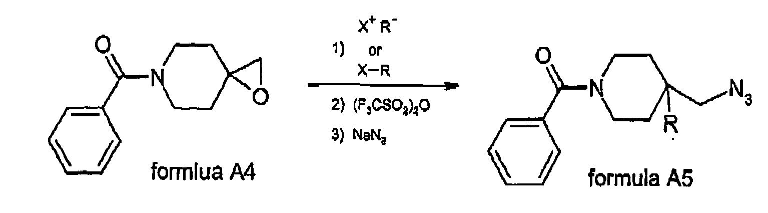 wherein the nucleophilic reagent of type X⁺R⁻ or X-R is selected from poly(hydrogene fluorid)pyridine, sodium azide, hydrogene chloride or solutions of acids.

Compounds according to formula A6 can be obtained by reaction of an intermediate according to formula A5 with pyridine derivatives according to formula B under reductive conditions:

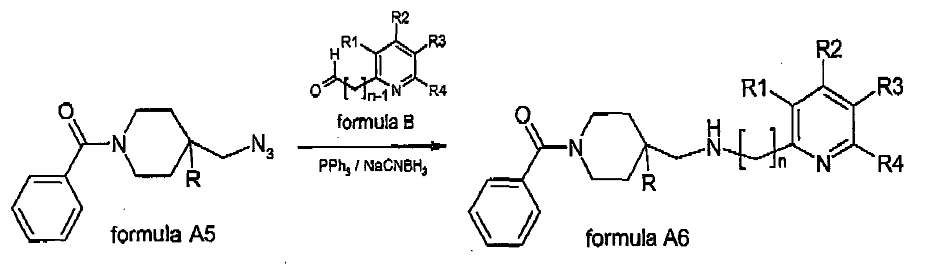

The pyridine derivatives according to formula B are available by oxidation of the appropriate alcohol precursors according to formula B1 when using oxidants like IBX (iodoxybenzoic acid) (for n = 1, 2 or 3) or MnO₂ (for n = 2 or 3). Alternatively, formyl pyridines according to formula B with n = 1 can be synthesized starting with bromo pyridines according to formula B2 which are activated by halogen metal exchange and subsequently reacted with formyl equivalents such as DMF.

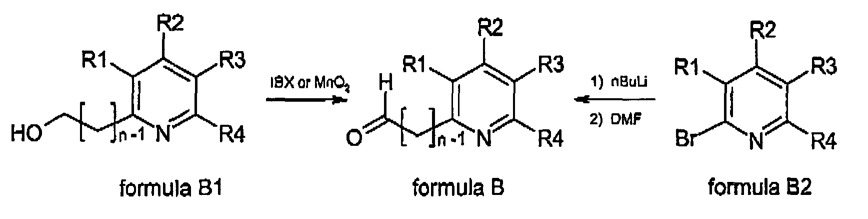

The synthesis of piperidine derivatives according to formula A7 as central intermediates in the preparation of the embodiments of this invention can be achieved in two further steps.

At first, a deprotecting reaction of the compounds according to formula A6 under acidic conditions leads to the piperidine bases according to formula A7.

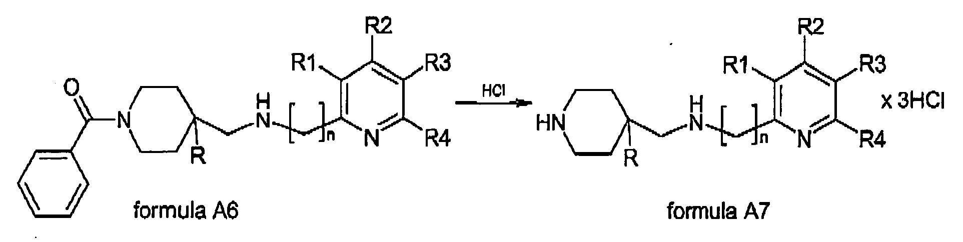

Secondly, a coupling reaction of the piperidine derivatives according to formula A7 with different acid derivatives of type C reveals the final compounds as embodiments of this disclosure. For this coupling reaction the acid derivatives according to formula C are used in an activated form as acid chlorides, acid bromides or acid anhydrids or, alternatively, as free acid compounds in the presence of an appropriate activating reagent typically used for amid coupling. 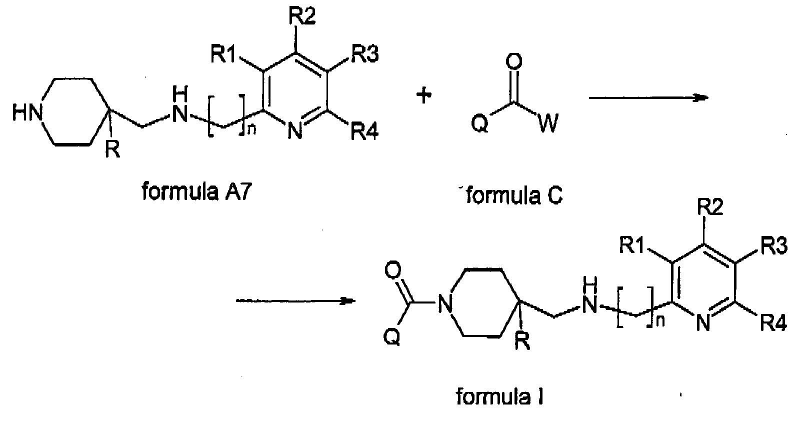 wherein in anyone of the formulas I, A5, A6, A7, B, B1, B2 and C

Q, n, R, R1, R2, R3 and R4 are as defined further above and in the disclosures for compounds of the formulas I, Ib, II, IIb, III, IIIb, IV, IVb, V, Vb, VI, VIb, VII or VIIb; and W is selected of hydroxyl, chloro, bromo or alkylcarbonyloxy;

and if W is hydroxyl, the corresponding acid derivative is activated by addition of an acid specific activating reagent like hydroxybenzotriazole, hydroxyazabenzotriazole, HATU (O-(7-azabenzotriazol-1-yl)-1,1,3,3-tetramethyluronium hexafluorophosphate) orTBTU (O-benzotriazolyl tetramethylisouronium tetrafluoroborate).

The above disclosed synthesis as well as the respective novel intermediates are also considered to be part of the present invention.

The production of individual compounds of the present invention is described in more detail in the experimental part of the present application.

In various embodiments, the compounds of the present invention are useful medicines, and may be used for the treatment of various diseases of the CNS system. One embodiment of the present disclosure is thus a compound as described herein for use as a medicine, in particular for use as a medicine for the treatment of a disease which is associated with a malfunction of the serotonin signaling system, examples of which are further disclosed below.

Because of their affinity to the serotonin 5-HT1a receptors, the present compounds in one embodiment can be used for the production of a medicament for the treatment of a variety of diseases such as
- pain, particularly chronic pain (nociceptive and neuropathic), including for example
   ■ neuropathic pain (central and peripheral) including mononeuropathies and polyneuropathies, which may be associated with diseases such as diabetic neuropathy, herpetic or other infections, AIDS, or cancer
   ■ trigeminal neuralgia
   ■ postoperative pain
   ■ treatment and prophylaxis of migraine
- depression, such as for example
   ■ endogenic depressions including major depression and depressive phases of bipolar disorders
   ■ somatogenic depressions
   ■ psychogenic depressions
- anxiety disorders, such as generalized anxiety, panic disorders, certain kinds of phobia such as e.g. social phobia, and post-traumatic disorders
- compulsive disorders and/or aggressive disorders
- a psychotic disease including manic phases of bipolar disorder, acute idiopathic psychotic illnesses, psychoses associated with other diseases, drug-induced psychoses, and particularly schizophrenia; attention deficit hyperactivity disorder (ADHD);
- idiopathic or drug-induced movement disorders such as tremors, akinesia, dyskinesia, idiopathic Parkinson's disease and it's associated motor disturbances, Segawa syndrome, or Tourette's syndrome,;
- addiction disorders such as e.g. cocaine, alcohol, opiate and nicotine addiction
- sexual dysfunction
- amnesic and/or cognitive disorders,
- autism, or disorders associated with autism,
- stroke,
- urinary incontinence

A further therapeutic application that can be mentioned is the treatment and/or prevention of neurodegenerative diseases, since due to the neuroprotective effect of 5-HT1a agonists, the substances may delay or stop the destruction or loss of neurones as the cause or result of a pathophysiological episode. Such illnesses are for example amyotrophic lateral sclerosis, Alzheimer's disease, Huntington's chorea, epilepsy, Parkinson's disease or synucleopathias, e.g. of the Parkinson-plus-syndrome type.

Another embodiment of the present disclosure is a method of treating a subject having a disease as descrbed above by administering a compound as described herein. According to one aspect, the subject to be treated with the presently disclosed compounds is determined to be in need of a treatment of one or more of the above diseases based on a prior diagnosis of the disease or diseases.

Prior to administration, the presently disclosed compounds are normally combined with ingredients to provide suitable dosage forms for administration. The other ingredients are well known and are variously characterized as carriers, excipient, adjurants, functional additives, and the like. For convenience of reference, "carrier" or "excipient" will be used to stand for these ingredients. Accordingly one aspect of the present invention is a pharmaceutical composition comprising a compound as described in this specification, and a pharmaceutically acceptable carrier. Hereinafter, some formulating methods and kinds of excipients will be described, but the present invention is not limited to them.

The pharmaceutical composition of the present invention can be administered to a mammalian subject such as domestic animals or human beings via various routes. The methods of administration include oral, buccal, sublingual, nasal, pulmonal, and rectal administration; intravenous, intramuscular, subcutaneous, and intracerebroventricular injections, wherein oral delivery is preferred.

The oral administration may be performed by providing the compounds of the present invention in the form of a tablet, a capsule, a drage', a powder, a granulate, or in form of a liquid or a semi-solid, by way of non-limiting example. A compound of formula (I), (Ib), (II), (IIb), (III), (IIIb), (IV), (IVb), (V), (Vb), (VI), (VIb), (VII) or (VIIb), including optical isomers or a pharmaceutically acceptable salt thereof according to the present invention can be prepared as a pharmaceutical composition containing pharmaceutically acceptable carriers, adjuvants, diluents and the like.

For instance, the compounds of the present invention can be dissolved in oils, propylene glycol or other solvents which are commonly used to produce an injection. Suitable examples of the carriers include, but not limited to, physiological saline, polyethylene glycol, ethanol, vegetable oils, isopropyl myristate, etc. The compounds of the present invention may be formulated into injections by dissolving, suspending or emulsifying in water-soluble solvent such as saline and 5% dextrose, or in water-insoluble solvents such as vegetable oils, synthetic fatty acid glyceride, higher fatty acid esters and propylene glycol. The formulations of the invention may include any of conventional additives such as dissolving agents, isotonic agents, suspending agents, emulsifiers, stabilizers and preservatives.

Oral formulations may contain, without limitation, sustained release agents, disintegrants, fillers, lubricants, stabilizers, antioxidants, stabilizers, flavours, dispersion agents, electrolytes, buffers or conservation agents. Suitable excipients and formulations are known to those skilled in the art and are disclosed in standard monographs such as like Remington ("The science and practice of pharmacy", Lippincott, Williams & Wilkins, 2000). Typical sustained release agents are for example those that swell upon contact with water such as polyvinylpyrrolidone, hydroxyethylcellulose, hydroxypropylcellulose, other cellulose ethers, starch, pregelatinised starch, polymethacrylate, polyvinylacetate, microcrystalline cellulose, dextrans, and mixtures of these. Non-limiting examples of disintegrants include pregelatinised starch, sodium starch glycolate, microcrystalline cellulose, carboxymethylcellulose sodium (CMC-Na), cross-linked CMC-Na, and low-substituted hydroxypropylcellulose, as well as mixtures thereof. Suitable fillers and binders include without limitation microcrystalline cellulose, powdered cellulose, lactose (anhydrous or monohydrate), compressible sugar, starch (e.g. corn starch or potato starch), pregelatinised starch, fructose, sucrose, dextrose, dextrans, other sugars such as mannitol, maltitol, sorbitol, lactitol and saccharose, siliconised microcrystalline cellulose, calcium hydrogen phosphate, calcium hydrogen phosphate dihydrate, dicalciumphosphate dihydrate, tricalciumphophate, calcium lactate or mixtures thereof. Lubricants, antiadherents and/or glidants include stearic acid, magnesium stearate, calcium stearate, sodium lauryl sulphate, hydrogenated vegetable oil, hydrogenated castor oil, sodium stearyl fumarate, macrogols, glycerol dibehenate, talc, corn starch, silicon dioxide, and the like, including mixtures.

The preferable dose level of the compounds according to the present invention depends upon a variety of factors including the condition and body weight of the patient, severity of the particular disease, dosage form, and route and period of administration, but may appropriately be chosen by those skilled in the art. In various embodiments, the compounds are administered in an amount ranging from 0.001 to 10 mg/kg of body weight per day, or from 0.03 to 1 mg/kg of body weight per day. Individual doses may range from about 0.1 to 500 mg of active ingredient per day, from about 0.2 to 100 mg/day, or from about 0.5 to 100 mg/day. Doses may be administered once a day, or several times a day with each divided portions.

Another aspect of the present invention is a Kit comprising a medicine or a pharmaceutical composition as described above, and instructions for its use.

The medicine according to the present invention may comprise one of the presently disclosed compounds as "stand alone" treatment of a pain condition such as e.g. neuropathic pain or fibromyalgia.

In one embodiment, a presently disclosed compound is administered together with other useful drugs in a combination therapy. In a non-limiting example, a compound according to the present invention is combined with another pain medicament having a different mode of action. Likewise a compound of the present invention can be combined with an anti-inflammatory drug if a painful inflammatory disease is to be treated. Also, a compound of the present disclosure may be used in combination with a dopaminergic agonist to treat a movement disorder such as e.g. idiopathic Parkinson's disease. In combination therapies the two or more active principles may be provided via the same formulation or as a "kit of parts", i.e. in separate galenic units. Also, the two or more active principles may be administered to the patient at the same time or subsequently, e.g. in an interval therapy.

### Definitions:

"Alkenyl" includes monovalent olefinically unsaturated hydrocarbyl groups being straight-chained or branched and having at least 1 double bond. In various embodiments, "Alkenyl" has 2-10 carbon atoms ("C2-C10 alkenyl"), 2-8 carbon atoms ("C2-C8 alkenyl") 2-6 carbon atoms ("C2-C6 alkenyl"), 2-5 carbon atoms ("C2-C5 alkenyl"), 2-4 carbon atoms ("C2-C4 alkenyl"), or only 2-3 carbon atoms ("C2-C3 alkenyl"). Particular alkenyl groups include ethenyl (vinyl, -CH=CH₂), n-propenyl (allyl, -CH₂CH=CH₂), isopropenyl (C(CH₃)=CH₂), and the like.
"alkynyl" includes acetylenically unsaturated hydrocarbyl groups being straight-chained or branched and having at least 1 triple bond. In various embodiments, "Alkynyl" has 2-10 carbon atoms ("C2-C10 alkynyl"), 2-8 carbon atoms ("C2-C8 alkynyl") 2-6 carbon atoms ("C2-C6 alkynyl"), 2-5 carbon atoms ("C2-C5 alkynyl"), 2-4 carbon atoms ("C2-C4 alkynyl"), or only 2-3 carbon atoms ("C2-C3 alkynyl"). A preferred alkynyl group is ethynyl (acetylenyl).
"Alkyl" includes monovalent saturated aliphatic hydrocarbyl groups. The hydrocarbon chain may be either straight-chained or branched. Examples of "alkyl" include those with 1-10 carbon atoms ("C1-C10 alkyl"), those with 1-8 carbon atoms ("C1-C8 alkyl"), those with 1-6 carbon atoms ("C1-C6 alkyl"), those with 1-5 carbon atoms ("C1-C5 alkyl"), 1-4 carbon atoms ("C1-C4 alkyl"), or only 1-3 carbon atoms ("C1-C3 alkyl"). This term is exemplified by groups such as methyl, ethyl, n-propyl, isopropyl, n-butyl, iso-butyl, tert-butyl, t-amyl, and the like.
"Alkylaminocarbonyl" includes the groups -C(O)-NH-alkyl wherein "alkyl" is defined above. An example of "alkylaminocarbonyl" is -C(O)-NH-(C1-C6)alkyl or -C(O)-NH-(C1-C4)alkyl. "Alkylaminosulfonyl" includes the group -SO₂-NH-alkyl, wherein "alkyl" is preferably selected from the groups specified in the definition of "alkyl" further above. Most preferably "alkyl" in "alkylaminosulfonyl" is a (C1-C6)alkyl group Examples of "alkylaminosulfonyl" are e.g. methylaminosulfonyl, ethylaminosulfonyl or butylaminosulfonyl.

"Alkylcarbonyl" includes the group -C(O)-alkyl, wherein alkyl is defined above. An example of "Alkylcarbonyl" is -C(O)-(C1-C6)alkyl, -C(O)-(C1-C5)alkyl or -C(O)-(C1-C3)alkyl and is illustrated by acetyl, propionyl and butyryl.
"Alkyloxy" or "alkoxy" includes the group -OR wherein R is "alkyl" as defined and exemplified further above. Particular alkyloxy groups include, by way of example, methoxy, ethoxy, n-propoxy, isopropoxy, n-butoxy, tert-butoxy, sec-butoxy, n-pentoxy, 1,2-dimethylbutoxy, and the like.
"Alkyloxycarbonyl" refer to the radical -C(=O)-O-R, wherein R is an alkyl group as defined herein. In various embodiments, "alkyloxycarbonyl" is a (C1-C6)alkyloxycarbonyl group, (C1-C5)alkyloxycarbonyl group or a (C1-C3)alkyloxycarbonyl group.
"Alkylsulfonyl" includes a radical-S(O)₂R, wherein R is an alkyl group as defined herein. Representative examples include, but are not limited to, methanesulfonyl, ethylsulfonyl, propylsulfonyl, butylsulfonyl and the like.
"Alkylsulfonylamino" includes the group -NH-SO₂-alkyl, wherein alkyl is defined above. In a non-limiting embodiment, "alkyl" in "alkylsulfonylamino "is a (C1-C6)alkyl group, such as in methanesulfonylamino.
"Alkylthio" includes a radical -S-R wherein R is an alkyl group as defined herein that may be optionally substituted as defined herein. Preferably, "alkylthio" is a (C1-C6)alkyl-S-group, (C1-C5)alkyl-S-group or a (C1-C4)alkyl-S-group. Representative examples include methylthio, ethylthio, propylthio, butylthio, and the like.
"Carbamoyl" refers to the group -C(=O)-NH₂.
"Carboxy" refers to the radical -C(=O)OH.
"Cyano" refers to the radical -C≡N.
"Cycloalkyl" refers to cyclic saturated aliphatic hydrocarbyl groups. The numbers of C-atoms referenced in connection with a given cycloalkyl group corresponds to the number of ring forming carbon atoms, e.g. "(C3-C6)cycloalkyl" refers to a cycloalkyl with between three and six ring-forming C atoms. Examples of "cycloalkyl" are (C3-C6)cycloalkyls, (C3-C5) cycloalkyls, or more specifically (C3-C4)cycloalkls or (C5-C6)cycloalkyls such as e.g. cyclopropyl, cyclobutyl, cyclopentyl, cyclohexyl etc. If indicated, a "cycloalkyl" group may be unsubstituted or substituted with one or more of hydroxyl, alkyloxy, halogen, phenyl, amino, dialkylamino, and alkyl groups. The latter particularly those selected from C1-C6 alkyl groups and C1-C3 alkyl groups, especially methyl groups. If a "cycloalkyl" carries more than one substituent, such as one or more alkyl substituent, these substituents may be attached to the same or to different ring-forming carbon atoms.
"Cycloalkyloxy" refers to the group -OR, wherein R is "cycloalkyl" group as defined further above.
"Cycloalkylthio" refers to the group -SR, wherein R is "cycloalkyl" group as defined further above.

"Dialkylaminocarbonyl" includes the group -CO-N-dialkyl, where "dialkyl" indicates two alkyl groups (defined above) bonded to the N atom. A non-limiting example of "dialkylaminocarbonyl" is -C(O)-N-di(C1-C6)alkyl.
"Dialkylaminosulfonyl" includes the group -SO₂-N-dialkyl, wherein "alkyl" is preferably selected from the groups specified in the definition of "alkyl" further above. An exemplary "alkyl" in "dialkylaminosulfonyl" is a (C1-C6)alkyl group. Examples of "alkylaminosulfonyl" include N,N-dimethylaminosulfonyl, N-methyl-N-ethylaminosulfonyl, and N-methyl-N-butylaminosulfonyl.
"Fluoralkyl" includes an "alkyl" group as defined above, which is substituted one or more times by a fluoro radical. Examples of "fluoroalkyl" are (mono)fluoromethyl or trifluoromethyl.
"Formyl" refers to the group -CH=O
"Halo" or "halogen" refers to fluoro, chloro, bromo and iodo. Preferred halogen groups are either fluoro or chloro.
"Heteroaryl" refers to aromatic ring systems containing at least one heteroatom such as O, S or N. Examples of heteroaryl radicals are furanyl, thienyl, pyrollyl, thiazolyl, oxazolyl, imidazolyl, pyrazolyl, isoxazolyl, isothiazolyl, triazolyl, oxadiazolyl, thiadiazolyl, pyranyl, pyridinyl, pyridazinyl, pyrimidinyl, pyrazinyl, triazinyl, tetrazinyl, indolinyl, indolyl, isoindolyl, benzofuranyl, benzothiophenyl, benzoimidazolyl, benzthiazolyl, purinyl, quinazolinyl, quinolinyl, isoquinolinyl, quinolizinyl, pteridinyl, carbazolyl, wherein one-ring sytems, and in particular pyridinyl, and imidazolyl are preferred.
"Hydroxy" refers to the radical -OH.
"Hydroxyalkyl" includes an "alkyl" group as defined further above which is substituted with one or more hydroxy groups.
"Nitro" refers to the radical-NO₂.
"Oxime" refers to the group -CH=N-OH.
"Oxo" refers to =O.
"Phenyl" is the aromatic radical -C₆H₅. Whether a phenyl group is substituted with one or more substituents, is specified throughout this specification and the claims.

"phenylalkyl" is an "alkyl" group substituted with a phenyl group, wherein "alkyl" is as defined further above. For example, phenyl(C1-C6)alkyl refers to a (C1-C6)alkyl which is substituted with a phenyl group. Examples of phenylalkyl groups are phenylethyl and benzyl, wherein benzyl is a particularly preferred phenylalkyl group.
"Phenylalkyloxy" is an alkyloxy group substituted with a phenyl. Examples of phenylalkyloxy groups are phenylethyloxy and benzyloxy.
"Phenylcarbonyl" is -C(O)-phenyl, wherein phenyl" has the meaning as defined further above.
"Phenylalkylcarbonyl" is -C(O)-alkylene-phenyl, wherein "phenyl" is defined above and "alkylene" is a divalent aliphatic derived by substitution from an alkyl group as defined above.
"Phenylalkyloxycarbonyl" is the group -C(O)-O-alkylene-phenyl, wherein "phenyl" and "alkylene" have the meaning as defined further above.
"Phenoxy" comprises the group -O-phenyl, wherein "phenyl" has the meaning as defined further above.
"Phenylsulfonyl" is -SO₂-phenyl, wherein "phenyl" has the meaning as defined further above.
"Phenylsulfonylamino" is -NH-SO₂-phenyl, wherein "phenyl" has the meaning as defined further above.
"Sulfamoyl" includes the group -SO₂-NH₂.
"Trifluoromethyl" refers to the group -CF₃.

Unless expressly specified otherwise, any "alkyl", "alkenyl", "alkynyl", "phenyl", or "heteroaryl" is meant to be unsubstituted. If any "alkyl", "alkenyl", "alkynyl", "phenyl", or "heteroaryl", is expressly stated to be substituted, this usually also refers to the respective "alkyl", "alkenyl", "alkynyl", "phenyl", or "heteroaryl" partial structures of more complex structures such as "alkyloxy", "alkylsulfonyl", "alkenyloxy", "phenoxy", "heteroaryloxy", etc.

"Isomer" includes especially optical isomers (for example pure enantiomers, pure diastereomers, and mixtures thereof) as well as conformation isomers (i.e. isomers that differ only in their angles of at least one chemical bond), position isomers (particularly tautomers), and geometric isomers (e.g. cis-trans isomers). According to the present disclosure, an enantiomer or diasteromer is "pure" if it present to over 90mol%, preferably to over 95mol%, especially preferably to over 99mol % as enantiomers in the respectively specified optical structure. This means that less than 10%, preferably less than 5%, especially preferably less than 1% of the respective compound is present in one or more optical forms.

"Pharmaceutically acceptable" means safe for use in pharmaceutical preparations, generally considered as safe for such use, officially approved by a regulatory agency of the Federal or a state government for such use, or being listed in the U. S. Pharmacopoeia or other generally recognized pharmacopoeia for use in animals, and more particularly in humans.

"Pharmaceutically acceptable salt" refers to a salt of a compound of the invention that is pharmaceutically acceptable and that possesses the desired pharmacological activity of the parent compound. Such salts include: (1) acid addition salts, formed with inorganic acids such as hydrochloric acid, hydrobromic acid, sulfuric acid, nitric acid, phosphoric acid, and the like; or formed with organic acids such as acetic acid, propionic acid, hexanoic acid, cyclopentanepropionic acid, glycolic acid, pyruvic acid, lactic acid, malonic acid, succinic acid, malic acid, maleic acid, fumaric acid, tartaric acid, citric acid, benzoic acid, 3-(4-hydroxybenzoyl) benzoic acid, cinnamic acid, mandelic acid, methanesulfonic acid, ethanesulfonic acid, 1,2-ethane-disulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, 4-methylbicydo [2.2.2]-oct-2-ene-1-carboxyic acid, glucoheptonic acid, 3-phenylpropionic acid, trimethylacetic acid, tertiary butylacetic acid, lauryl sulfuric acid, gluconic acid, glutamic acid, hydroxynaphthoic acid, salicylic acid, stearic acid, and muconic acid. Other salts include 2,2-dichloroacetate, adipate, alginate, ascorbate, aspartate, 2-acetamidobenzoate, caproate, caprate, camphorate, cyclamate, laurylsulfate, edisilate, esylate, isethionate, formate, galactarate, gentisate, gluceptate, glucuronate, oxoglutarate, hippurate, lactobionate, napadisilate, xinafoate, nicotinate, oleate, orotate, oxalate, palmitate, embonate, pidolate, p-aminosalicylate, sebacate, tannate, rhodanide, undecylenate, and the like; or (2) salts formed when an acidic proton present in the parent compound is replaced, such as with ammonia, arginine, benethamine, benzathine, calcium, choline, deanol, diethanolamine, diethylammonium, ethanolamine, ethylendiamine, meglumine, hydrabamine, imidazole, lysine, magnesium, hydroxyethylmorpholine, piperazine, potassium, epolamine, sodium, trolamine, tromethamine or zinc.

"Pharmaceutically acceptable carrier" refers to a diluent, adjuvant, excipient, or carrier, or other ingredient with which a compound of the invention is administered.

"Preventing" or "prevention" refers to a reduction in risk of acquiring a disease or disorder (i.e., causing at least one of the clinical symptoms of the disease not to develop in a subject that may be exposed to or predisposed to the disease but does not yet experience or display symptoms of the disease).
"Subject" includes humans. The terms "human," "patient" and "subject" are used interchangeably herein.
"Therapeutically effective amount" means the amount of a compound that, when administered to a subject for treating a disease, is sufficient to effect such treatment for the disease. The "therapeutically effective amount" can vary depending on the compound, the disease and its severity, and the age, weight, etc., of the subject to be treated. "Treating" or "treatment" of any disease or disorder refers, in one embodiment, to ameliorating the disease or disorder (i. e., arresting or reducing the development of the disease or at least one of the clinical symptoms thereof). In another embodiment "treating" or "treatment" refers to ameliorating at least one physical parameter, which may not be discernible by the subject. In yet another embodiment, "treating" or "treatment" refers to modulating the disease or disorder, either physically, (e. g., stabilization of a discernible symptom), physiologically, (e. g., stabilization of a physical parameter), or both. In yet another embodiment, "treating" or "treatment" refers to delaying or preventing the onset of the disease or disorder.

### Modes for carrying out the invention:

### 1. Synthesis of the piperidine components according to formula A7

### 1a) 1,4-Dioxa-8-aza-spiro[4.5]dec-8-yl-phenyl-methanone (A2)

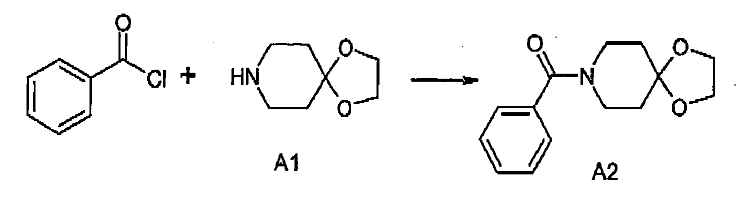

The synthesis of compound A2 started by adding 5.0 g (35.6 mmol) benzoylchloride to a cooled (0 °C) solution of 10.2 g (71.1 mmol) dioxaazaspirodecane (A1) in 500 ml dichloromethane and 59 ml triethylamine. The mixture was warmed up to room temperature and stirred for 15 hrs. The reaction mixture was concentrated by evaporation, washed with 2n HCl and water, tried over Na₂SO₄ and purified by flash chromatography using hexane-ethyl acetate (6:4)
Yield: 7.2 g (90%) brown oil,
MS (EI): m/z 248 (M+1)⁺.
Further spectroscopic data are identical with literature (Kobashi, S., Biol. Pharm. Bull. (2002), 25, 1030).

### 1b) 1-Benzoylpiperidin-4-one (A3)

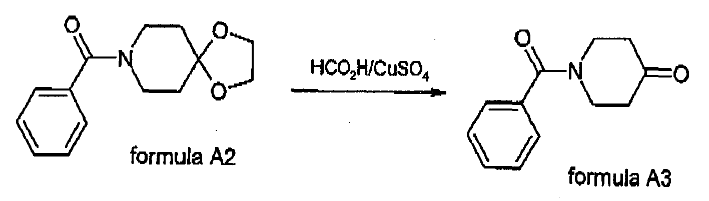

1-Benzoylpiperidin-4-one (A3) was synthesized according to literature (Vacher et al. J. Med. Chem., (1999), 42, 1648). In detail, the reaction of 6.87 g (27.8 mmol) 1,4-dioxa-8-aza-spiro[4.5]dec-8-yl-phenyl-mothanone (A2) with 33.7 ml aqueous formic acid (80%) and 15.2 mg (0.09 mmol) CuSO₄ at 80 °C for 16 hrs and subsequent evaporation of the formic acid by aceotropic distillation gave the crude product.

Yield: 5.4 g (96 %) brown oil.

MS (APCI): m/z 204 (M+1)⁺. ¹H NMR (CDCl₃, 600 MHz) δ (ppm): 2.28-2.75 (m, 4H, piperidine); 3.61-4.20 (m, 4H, piperidine); 7.43-7.52 (m, 5H, phenyl).

### 1c) 1-Oxa-6-aza-spiro[2.5]oct-6-yl-phenyl-methanone (A4)

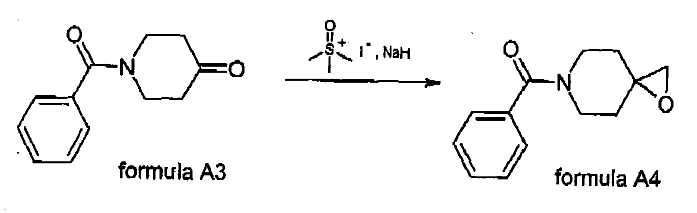

1-Oxa-6-aza-spiro[2.5]oct-6-yl-phenyl-methanone (A4) was synthesized according to literature (Vacher et al. J Med Chem, (1999), 42, 1648). In detail, 19.6 g (490 mmol) NaH (60% in oil) was suspended in 600 ml DMSO and stirred at 65° C for 2 hrs. After cooling to room temperature 108 g (490 mmol) trimethylsulfoxonium iodide was added and the mixture was stirred for 15 min at room temperature. Then, 95 g (470 mmol) 1-benzoylpiperidin-4-one dissolved in 300 ml DMSO was added to the mixture and the solution was stirred for 45 min. The reaction mixture was poured in water and extracted with ethyl acetate for three times. The combined organic layers were washed with water and brine, dried over Na₂SO₄ and evaporated in vacuo to yield the product.
Yield: 84 g (83 %) yellow oil.
MS (APCI): m/z 218 (M+1)⁺. ¹H NMR (CDCl₃, 600 MHz) δ (ppm): 1.35-1.60 (m, 2H, piperidine); 1.76-2.68 (m, 2H, piperidine); 2.74 (bs, 2H, oxirane); 3.43-3.72 (m, 3H, piperidine); 4.18-4.36 (m, 1H, piperidine); 7.37-7.46 (m, 5H, phenyl).

### 1d) Synthesis of 4-substituted piperidine intermediates according to formula (A5)

The intermediate A5 can be synthesized in a three step reaction starting with the ring opening of the oxirane system by reaction with different nucleophile reagent of type X⁺R⁻ or X-R to obtain the primary alcohol (A5a), which is activated as a trifluoromethane sulfonic acid ester (A5b) and subsequently reacted with sodium azide to get the azide derivative A5.

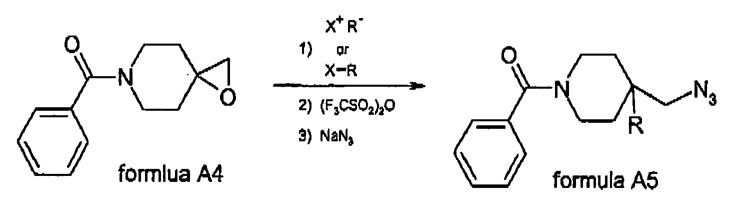

*4-Fluoro-4-hydroxymethylpiperidin-4-yl-phenyl-methanone* (A5a; R = F)

To a cooled (-10° C) solution of compound A4 (84 g, 385 mmol) in 200 ml dichloromethane 100 ml (1160 mmol) poly(hydrogene fluorid)pyridine was added drop wise in 3hrs at -10° C. The solution was warmed to room temperature, poured in water, which was neutralised with 50% K₂CO₃ to pH = 7 and extracted with dichloromethane for three times. The combined organic layers were washed with water, 1n HCl and brine, dried over Na₂SO₄ and concentrated in vacuo to get crude brown oil which was purified by flash chromatography with dichloromethane-methanol (98:2).
Yield: 53 g (60 %) white solid.
MS (APCI): m/z 238 (M+1)⁺. ¹H NMR (CDCl₃, 360 MHz) δ (ppm): 1.46-2.13 (m, 4H, piperidine); 2.96-3.53 (m, 3H, piperidine); 3.64 (dd, J=19.6 Hz, 4.2 Hz, 2H, piperidine-CH₂); 4.42-4.76 (m, 1H, piperidine); 7.37-7.51 (m, 5H, phenyl).

*Trifluoromethane sulfonic acid 1-benzoyl-4-fluoropiperidin-4-ylmethyl ester* (A5b; R = F) A solution of 71 g (300 mmol) 4-fluoro-4-hydroxymethylpiperidin-1-yl-phenyl-methanone (A5a; R = F) in 1400 ml pyridine was cooled to 0 °C. Trifluoromethane sulfonic acid anhydride (75 ml, 450 mmol) was added drop wise within 70 min, the mixture was stirred for 1 h at room temperature and then poured in water. The aqueous layer was extracted with dichloromethane for three times, the combined organic layers were washed with 5n HCl, dried over Na₂SO₄ and concentrated under reduced pressure to get 78 g (yield: 60%) of a white solid.
Yield: 78 g (60%).
MS (APCI): m/z 370 (M+1)⁺.¹H NMR (CDCl₃, 360 MHz) δ (ppm): 1.63-2.13 (m, 4H, piperidine); 3.10-4.02 (m, 3H, piperidine); 4.48 (d, J=18.9 Hz, 2H, piperidine-CH₂); 4.58-4.87 (m, 1H, piperidine); 7.39-7.48 (m, 5H, phenyl).

*4-Azidomethyl-4-fluoropiperidin-1-y*/*-phenyl-methanone* (A5; R = F)
To a solution of 78 g (212 mmol) trifluoromethane sulfonic acid 1-benzoyl-4-fluoropiperidin-4-ylmethyl ester (A5b) in 1000 ml DMF was added 110 g (1700 mmol) NaN₃ and the mixture was heated at 80° C for 15 hrs. The solvent of the mixture was reduced by evaporation in vacuo and 1000 ml dichloromethane was added. Then, the organic layer was washed with brine for three times, dried over Na₂SO₄ and concentrated in vacuo to yield a dark brown oil, which was purified by flash chromatography with heptane (supplemented with ethyl acetate from 0 to 40%).
Yield: 28 g (36 %) white solid.
MS (EI): m/z 263 (M+1)⁺. ¹H NMR (CDCl₃, 600 MHz) δ (ppm): 1.48-2.14 (m, 4H, piperidine); 3.05-3.48 (m, 4H, piperidine, pipertdine-CH₂); 3.58-3.83 (m, 1H, piperidine); 4.48-4.79 (m, 1H, piperidine); 7.38-7.47 (m, 5H, phenyl).

### 1e) Synthesis of 4-substituted piperidine intermediates according to formula (A7)

The synthesis of the central intermediate in the preparation of the embodiments of this invention according to formula A7 can be achieved by coupling reaction of piperidines according to formula A5 with aldehyde precursors of the pyridine derivatives according to formula B under reductive conditions and a subsequent acidic hydrolysis of the protecting benzoyl moiety yielding the free piperidines according to formula A7.

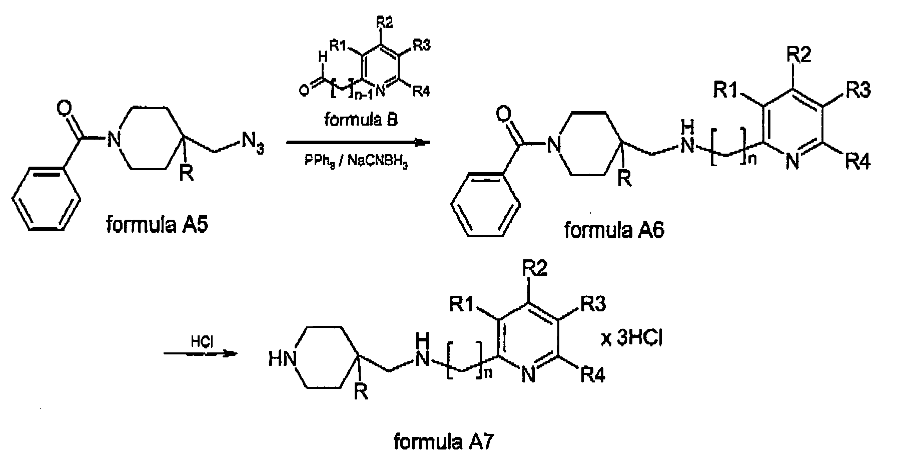

*4-Fluoro-4-[[(5-methylpyridin-2-ylmethyl)amino]methyl]piperidin-1-yl-phenyl-methanone* (A6; n =1; R = F; R1, R2, R4 = H; R3 = Me)
A solution of 41 g (156 mmol) 4-azidomethyl-4-fluoropiperidin-1-yl-phenyl-methanone (A5; R = F), 19 g (160 mmol) 5-methylpyridin-2-carbaldehyd (B; n = 1; R1, R2, R4 = H; R3 = Me) and 41 g (160 mmol) PPh₃ in 2800 ml MeOH was heated to reflux for 3 hrs. After cooling to room temperature 31 g (50 mmol) NaCNBH₃ was added and the solution was stirred at room temperature for 15 hrs. The solvent was evaporated in vacuo and the remaining residue was poured in water. The aqueous layer was extracted with dichloromethane for three times, the combined organic layers were washed with water, brine (1 x 1 L), dried over Na₂SO₄ and concentrated in vacuo to obtain 101 g of a brown oil. The product was purified by flash chromatography with dichloromethane (supplemented with MeOH from 0 to 4%).
Yield: 44 g (82%) brown oil.
MS (APCl): m/z 342 (M+1)⁺. ¹H NMR (CDCl₃, 360 MHz) δ (ppm): 1.48-2.18 (m, 4H, piperidine); 2.35 (s, 3H, pyridine-CH₃); 2.82 (d, J=20.2 Hz, 2H, piperidine-CH₂NHCH₂): 3.11-3.78 (m, 2H, piperidine); 3.93 (s, 2H, piperidine-CH₂NHCH₂); 4.44-4.70 (m, 2H, piperidine); 7.23 (d, J=8.0 Hz, 1H, pyridine H3): 7.38-7.46 (m, 5H, phenyl); 7.48 (dd, J=7.8 Hz, 1.7 Hz, 1 H, pyridine H4); 8.37-8.43 (m, 1 H, pyridine H6).

*(4-Fluoropiperidin-4-ylmethyl)-(5-methylpyridin-2-ylmethyl)amine trihydrochloride* (A7; n = 1; R = F; R1, R2, R4 = H; R3 = Me)
A solution of 43 g (125 mmol) 4-fluoro-4-[[(5-methylpyridin-2-ylmethyl)amino]methyl] piperidin-1-yl-phenyl-methanone (A6; n = 1; R = F; R1, R2, R4 = H; R3 = Me) in 4800 ml 6n HCl was heated to reflux for 15 hrs. The reaction mixture was washed with diethylether for three times, the aqueous layer was evaporated under reduced pressure to get a thick oil, which was washed with acetonitrile for several times to obtain a solid. The solid was washed with diethylether, then suspended in a small amount of MeOH. Addition of diethylether induced the formation of a yellow solid, which was rinsed with diethylether and tried.
Yield: 31 g (89%) yellow solid.
MS (EI): m/z 342 (M+1)⁺. ¹H NMR (CDCl₃, 360 MHz) 6 (ppm): 1.98-2.25 (m, 4H, piperidine); 2.37 (s, 3H, pyridine-CH₃); 2.93-3.04 (m, 2H, piperidine); 3.20-3.30 (m, 2H, piperidines); 3.37 (d, J=21.5 Hz, 2H, pipefldine-CH₂NHCH₂); 4.39 (s, 2H, piperidine-CH₂NHCH₂); 7.72 (d, J=7.9 Hz, 1H, pyridine H3); 7.91 (d, J=7.6 Hz, 1H, pyridine H4); 8.58 (s, 1H, pyridine H6); 9.45 (bs, 2H, pyridine NH, piperidine NH).¹³C NMR (CDCl₃, 360 MHz) δ (ppm): 18.1; 29.3; 29.5; 39.1; 50.1; 52.7; 52.8; 90.3; 91,5; 125.1; 134.9; 140.4; 147.7.

### 2. Synthesis of pyridine derivatives according to formula B

### 2a) 5-Methylpyridin-2-carbaldehyde (B; n = 1; R1, R2, R4 = H; R3 = Me)

The formyl pyridine derivatives according to formula B (n = 1) can be prepared starting from the appropriate 2-bromo substituted pyridines (B2), which were treated with n-butyl lithium to achieve a halogen metal exchange. Further reaction with formylating reagents led to formyl pyridine derivatives according to formula B (n = 1).

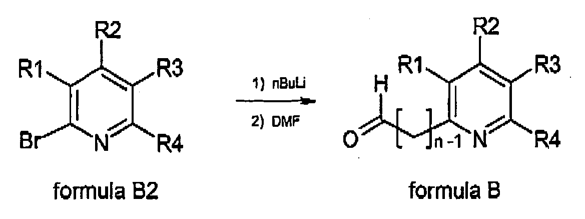

For the synthesis of 5-methylpyridin-2-carbaldehyde (B; n = 1; R1, R2, R4 = H; R3 = Me) 50 g (290 mmol) 2-bromo-5-methylpyridine (B2; R1, R2, R4 = H; R3 = Me) was dissolved in 2500 ml diethylether and cooled to -78°C. Then, 150 ml (380 mmol) nBuLi was added drop wise and the reaction mixture was stirred for 2hrs at -78° C. DMF (31.5 ml, 407 mmol) was added drop wise and after the reaction mixture was allowed to warm to room temperature, water was added to the reaction mixture. Both, organic and aqueous layer were separated and the water layer was extracted with ethyl acetate for three times. All organic layers were combined, washed with brine, dried over Na₂SO₄ and the solvent was evaporated in vacuo yielding a brown oil. This residue was purified by flash chromatography with heptane (supplemented with ethyl acetate from 0 to 25%).
Yield: 19 g (39%) brown solid.
MS (APCI): m/z 122 (M+1)⁺. ¹H NMR (CDCl₃, 360 MHz) δ (ppm): 2.48 (s, 3H, CH₃); 7.70 (dd, J=7.9 Hz, 2.0 Hz, 1H, pyridine H4); 7.91 (d, J=7.7 Hz, 1H, pyridine H3); 8.68 (s, 1H, pyridine H6); 10.10 (s, 1H, aldehyde).

### 2b) Alternative synthesis of pyridine derivatives according to formula B

An alternative way to synthesize aldehyde derivatives according to formula B (n = 1, 2 or 3) can be done by oxidation of the appropriate alcohol precursors according to formula B1 using oxidizing reagents like IBX (iodoxybenzoic acid) (for n = 1,2 or 3) or MnO₂ (for n = 2 or 3).

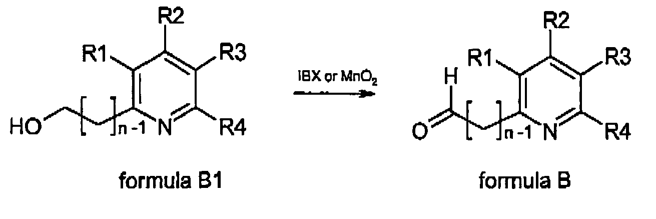

### 3. Acid derivatives according to formula C

Most of the applied acid derivatives according formula C (W = OH; Q variable) can be purchased from established suppliers of fine chemicals for synthesis. All acid derivatives of this invention were purchased from *Acros Organics, Geel (Belgium), Alfa Aesar, Karlsruhe (Germany), Maybridge, Tintagel, Cornwall (UK)* or *Sigma-Aldrich, Munich (Germany)* with the exception of the ones mentioned below.

### 3a) Specially purchased acid derivatives according to formula C

*4-Fluorothiophene-2-carboxylic acid* (C2), *5-bromofuran-3-carboxylic acid* (C34), *benzo[b]furan-3-carboxylic acid* (C36)

Derivatives according to formula C were purchased from:
*4-Fluorothiophene-2-carboxylic acid* (C2)
from *Chemstep, Carbon Blanc (France)* [Order number: 31578];
*5-Bromofuran-3-carboxylic acid* (C34)
from Akos, *Steinen (Germany)* [Order number: AKE-BBV-006108];
*Benzo[b]furan-3-carboxylic acid* (C36)
from *Aurora Fine Chemicals, Graz (Austria)* [Order number: kasi-045873];

### 3b) Individually synthesized acid derivatives according to formula C

*Pyrazolo[1,5-a]pyridine-2-carboxylic acid* (C42), *pyrazolo[1,5-a]pyridine-3-carboxylic acid* (C43),

The pyrazolo[1,5-a]pyridine carboxylic acid derivatives C42 and C43 were synthesized according to literature (Bettinetti et al., J. Med. Chem., (2002), 45, 4594).

### 4. Synthesis of embodiments according to formula I

The synthesis of the final embodiments was achieved by an amide coupling reaction utilizing the piperidine derivatives according formula A7 and acid derivatives of type formula C. The acid could be used as activated derivative such as acid chloride (W = Cl) or acid anhydride (W = alkylcarbonyloxy) or as free acid (W = OH) in the presence of an activating reagents specific for amide coupling like hydroxybenzotriazole, hydroxyazabenzotriazole, HATU (O-(7-azabenzotriazol-1-yl)-1,1,3,3-tetramethyluronium hexafluorophosphate) or TBTU (O-benzotriazolyl tetramethylisouronium tetrafluoroborate).

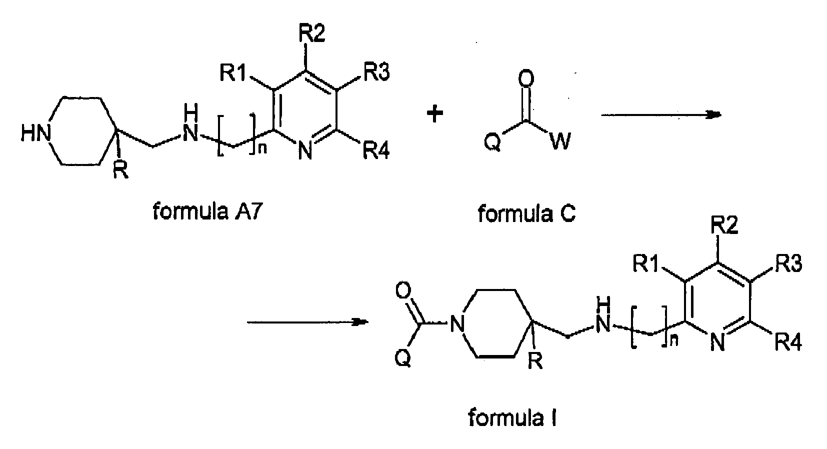

### Example E1:

### [4-Fluoro-4-[(5-methylpyridin-2-ylmethyl)amino]methyl]piperidin-1-yl]-thien-2-yl-methanone

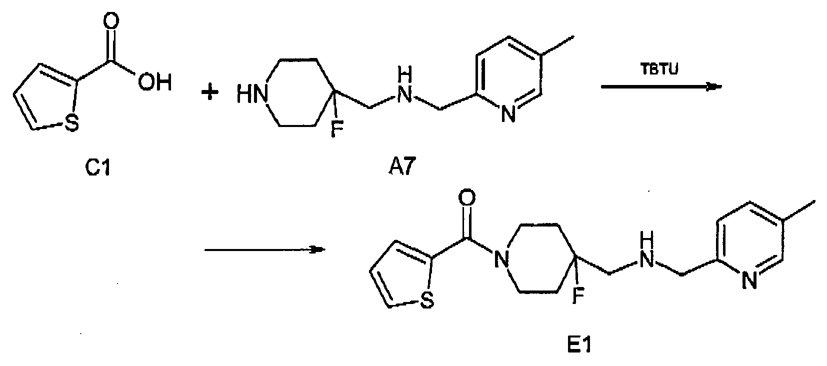

The synthesis of [4-fluoro-4-[(5-methylpyridin-2-ylmethyl)amino]methyl]piperidin-1-yl]-thien-2-yl-methanone (E1) was started by dissolving 20.5 mg (0.16 mmol) thiophene-2-carboxylic acid (C1) in 5.1 ml of dry dichloromethane. After adding 0.16 ml diisopropylethylamine the solution was cooled to 0°C and 50,4 mg (0.16 mmol) O-benzotriazolyl tetramethylisouronium tetrafluoroborate dissolved in 0.20 ml dry DMF was added to the reaction mixture, which was then allowed to warm up to ambient temperature. (4-Fluoropiperidin4-ylmethyl)(5-methylpyridin-2-ylmethyl)amine trihydrochloride (A7; n = 1; R = F; R1, R2, R4 = H; R3 = Me) was suspended in 0.50 ml dry dichloromethane, resolved by adding drops of diisopropylethylamine until complete solution and then added to the solution of activated acid derivative. After stirring for 2 hrs at room temperature the mixture was washed with brine for three times and dried with Na₂SO₄. The solvent was evaporated under reduced pressure and the residue was purified by flash-chromatography (CH₂Cl₂/MeOH 98:2).
Yield: 23.3 mg (42 %) light yellow oil.
MS (APCI): m/z 348 (M+1)⁺. IR (NaCl) v (cm⁻¹): 3660; 2923; 1619; 1438; 1276; 1095; 860; 736. ¹H NMR (CDCl₃, 360 MHz) δ (ppm): 1.62-1.88 (m, 2H, piperidine); 2.00-2.14 (m, 2H, piperidine); 2.35 (s, 3H, pyridine-CH₃); 2.82 (d, J=20.4 Hz, 2H, piperidine-CH₂NHCH₂); 3.28-3.48 (m, 2H, piperidine); 3.94 (s, 2H, piperidine-CH₂NHCH₂); 4.18-4.42 (m, 2H, piperidine); 7.07 (dd, J=5.0 Hz, 3.6 Hz, 1H, thiophene H4); 7.23 (d, J=8.0 Hz, 1H, pyridine H3); 7.32 (dd, J=3.6 Hz, 1.1 Hz, 1H, thiophene H3); 7.46 (dd, J=5.0 Hz, 1.1 Hz, 1H, thiophene H5); 7.49 (dd, J=7.4 Hz, 1.5 Hz, 1 H, pyridine H4); 8.39-8.43 (m, 1 H, pyridine H6).
HR-MS: C₁₆H₂₂FN₃O₈; calculated: 347.1468; found: 347.1467.

### Example E2:

### [4-Fluoro-4-[(5-methylpyridin-2-ylmethyl)amino]methyl]piperidin-1-yl]-5-fluorothien-2-yl-methanone

Synthesis worked according to the preparation of E1 when using 4-fluorothiophene-2-carboxylic acid (C2).
Yield: 7.8 mg (37%) light yellow oil.
MS (APCI): m/z 366 (M+1)⁺. IR (NaCl) v (cm⁻¹): 3332, 2923, 1616, 1477, 1430, 1276, 1199,1087, 964, 798, 740. ¹H NMR (CDCl₃, 360 MHz) δ (ppm): 1.60-1.84 (m, 2H, piperidine); 2.01-2.17 (m, 2H, piperidine); 2.35 (s, 3H, pyridine-CH₃); 2.82 (d, J=20.2 Hz, 2H, piperidine-CH₂NHCH₂); 3.31-3.46 (m, 2H, piperidine); 3.94 (s, 2H, piperidine-CH₂NHCH₂); 4.23-4.38 (m, 2H, piperidine); 6.47 (dd, J=4.2 Hz, 3.9 Hz, 1H, thiophene H4); 6.99 (dd, J=3.9 Hz, 3.9 Hz, ,1H, thiophene H3); 7.23 (d, J=7.7 Hz, 1H, pyridine H3); 7.49 (dd, J=7.8 Hz, 1.7 Hz, 1H, pyridine H4); 8.39-8.44 (m, 1H, pyridine H6).

### Example E3:

### 3-Chlorothien-2-yl-[4-fluoro-4-[[(5-methylpyridin-2-ylmethyl)amino]methyl]-piperidin-1-yl]-methanone

Synthesis worked according to the preparation of E1 when using 3-chlorothiophene-2-carboxylic acid (C3).
Yield: 12.8 mg (59%) light yellow oil.
MS (APCI): m/z 382 (M+1)⁺. IR (NaCl) v (cm⁻¹): 3745; 2919; 1635; 1446; 1276; 1095; 968; 898; 740. ¹H NMR (CDCl₃, 360 MHz) δ (ppm): 1.84-1.96 (m, 2H, piperidine); 2.00-2.16 (m, 2H, piperidine); 2.35 (s, 3H, pyridine-CH₃); 2.82 (d, J=20.7 Hz,2H, piperidine-CH₂NHCH₂); 3.17-3.52 (m, 2H, piperidine); 3.93 (s, 2H, piperidine-CH₂NHCH₂); 4.22-4.81 (m, 2H, piperidine); 6.94 (d, J=5.2 Hz,1H, chlorothiophene H4); 7.23 (d, J=7.9 Hz, 1H, pyridine H3); 7.39 (d, J=5.2 Hz,1H, chlorothiophene H5); 7.49 (dd, J=7.7 Hz, 1.8 Hz, 1H, pyridine H4); 8.41 (dd, J=1.4 Hz, 0.7 Hz, 1 H, pyridine H6). ¹³C NMR (CDCl₃, 360 MHz) δ (ppm): 18.1; 33.3; 38.8; 43.4; 55.2; 56.8; 57.0; 93.4; 95.3; 121.6; 124.0; 126.6; 127.7; 130.3;
139.4; 137.0; 149.7; 156.6; 161.5.
HR-MS: C₁₈H₂₁ClFN₃OS; calculated: 381.1078; found: 381.1075.

### Example E4:

### 5-Chlorothien-2-yl-[4-fluoro-4-[[(5-methylpyridin-2-ylmethyl)amino]methyl]-piperidin-1-yl]-methanone

Synthesis worked according to the preparation of E1 when using 5-chlorothiophene-2-carboxylic acid (C4).
Yield: 32.8 mg (60%) light yellow oil.
MS (APCI): m/z 382 (M+1)⁺, IR (NaCl) v (cm⁻¹): 3328; 2923; 1619; 1442; 1373; 1272; 1091; 806; 732. ¹H NMR (CDCl₃, 360 MHz) δ (ppm): 1.81-1.94 (m, 2H, piperidine); 2.02-2.15 (m, 2H, piperidine); 2.35 (s, 3H, pyridine-CH₃); 2.82 (d, J=20.2 Hz, 2H, piperidine-CH₂NHCH₂); 3.29-3.45 (m, 2H, piperidine); 3.93 (s, 2H, piperidine-CH₂NHCH₂); 4.22-4.38 (m, 2H, piperidine); 6.89 (d, J=4.1 Hz,1H, chlorothiophene H4); 7.10 (d, J=3.9 Hz,1H, chlorothiophene H3); 7.23 (d, J=7.7 Hz, 1H, pyridine H3); 7.49 (dd, J=8.0 Hz, 1.6 Hz, 1H, pyridine H4); 8.41 (dd, J=1.4 Hz, 0.7 Hz, 1H, pyridine H6). ¹³C NMR (CDCl₃, 360 MHz) δ (ppm): 18.1; 33.2; 33.4; 41.3; 55.17; 56.8; 570; 93.5; 95.4; 121.7; 125.9; 128.3; 131.4;
133.9; 135.9; 137.0; 149.7; 156.6; 162.3.
HR-MS: C18H21ClFN3OS; calculated: 381.1078; found: 381.1079.
CHN (%): C₁₈H₂₁ClFN₃OS; calculated (x 0.1 H₂O): C 56.41; H 5.58; N 10.97 S 8.35; found: C 56.18; H 5.65; N 10.67; S 8.10.

### Example E5:

### 3-Bromothien-2-yl-[4-fluoro-4-[[(5-mothylpyridin-2-ylmethyl)amino]mothyl]-piperidin-1-yl]-methanone

Synthesis worked according to the preparation of E1 when using 3-bromothiophene-2-carboxylic acid (C5).
Yield: 9.3 mg (38%) light yellow oil.
MS (APCI): m/z 428 (M+1)⁺. IR (NaCl) v (cm⁻¹): 3833; 2923; 1635; 1446; 1276; 875; 732. ¹H NMR (CDCl₃, 600 MHz) δ (ppm): 1.77-1.88 (m, 2H, piperidine); 1.98-2.15 (m, 2H, piperidine); 2.33 (s, 3H, pyridine-CH₃); 2.80 (d, J=20.5 Hz, 2H, piperidine-CH₂NHCH₂); 3.08-3.76 (m, 3H, piperidine); 3.91 (s, 2H, piperidine-CH₂NHCH₂); 4.37-4.80 (m, 1H, piperidine); 6.98 (d, J=5.3 Hz,1H, bromothiophene H4); 7.21 (d, J=7.6 Hz, 1H, pyridine H3); 7.35 (d, J=5.3 Hz,1H, bromothiophene H5); 7.47 (dd, J=7.9 Hz, 1.5 Hz, 1H, pyridine H4); 8.37-8.41 (m, 1H, pyridine H6). ¹³C NMR (CDCl₃, 360 MHz) δ (ppm): 18.1; 33.2; 55.2; 56.8; 57.0; 93.5; 95.4; 109.3; 121.7; 127.0; 130.2; 131.4; 132.3; 137.0; 149.7; 156.6;
162.0.
HR-MS: C₁₈H₂₁BrFN₃OS; calculated: 425.0573; found: 425.0573.

### Example E6:

### 4-Bromothien-2-yl-[4-fluoro-4-[[(5-methylpyridin-2-ylmethyl)amino]methyl]-piperidin-1-yl]-methanone

Synthesis worked according to the preparation of E1 when using 4-bromothiophene-2-carboxylic acid (C6).
Yield: 14.8 mg (61 %) light yellow oil.
MS (APCI): m/z 427 (M+1)⁺. IR (NaCl) v (cm⁻¹): 3324; 2923; 1619; 1434; 1373; 1272; 968; 812; 759; ¹H NMR (CDCl₃, 360 MHz) δ (ppm): 1.85-1.98 (m, 2H, piperidine): 2.03-2.17 (m, 2H, piperidine); 2.35 (s, 3H, pyridine-CH₃); 2.82 (d, J=20.2 Hz, 2H, piperidine-CH₂NHCH₂); 3.26-3.50 (m, 2H, piperidine); 3.93 (s, 2H, piperidine-CH₂NHCH₂); 4.15-4.46 (m, 2H, piperidine); 7.24 (d, J=1.2 Hz,1H, bromothiophene H5); 7.23 (d, J=8.0 Hz, 1H, pyridine H3); 7.37 (d, J=1.4 Hz,1H, bromothiophene H3); 7.49 (dd, J=7.9 Hz, 1.8 Hz, 1H, pyridine H4); 8.38-8.43 (m, 1H, pyridine H6). ¹³C NMR (CDCl₃, 600 MHz) δ (ppm): 18.1; 33.3; 55.1; 56.8; 57.0; 93.8; 95.0; 109.3; 121.7; 125.9; 130.6; 131.4; 137.0; 138.5; 149.7; 156.5;
162.0.
HR-MS: C₁₈H₂₁BrFN₃OS; calculated: 425.0573; found: 425.0573.
CHN (%): C₁₈H₂₁BrFN₃OS; calculated (x 0.6 H₂O): C 49.56; H 5.13; N 9.64 S 7.34; found: C 49.27; H 5.01; N 9.24; S 7.15.

### Example E7:

### 5-Bromothion-2-yl-[4-fluoro-4-[[(5-mothylpyridin-2-ylmethyl)amino]methyl]-piperidin-1-yl]-methanone

Synthesis worked according to the preparation of E1 when using 5-bromothiophene-2-carboxylic acid (C7).
Yield: 12.7 mg (52%) light yellow oil.
MS (APCI): m/z 428 (M+1)⁺. IR (NaCl) v (cm⁻¹): 3660; 2923; 1619; 1438; 1272; 1091; 971; 806; 732. ¹H NMR (CDCl₃, 360 MHz) δ (ppm):1.82-1.93 (m, 2H, piperidine); 2.03-2.15 (m, 2H, piperidine); 2.35 (s, 3H, pyridine-CH₃); 2.82 (d, J=20.2 Hz,2H, piperidine-CH₂NHCH₂); 3.27-3.45 (m, 2H, piperidine); 3.93 (s, 2H, piperidine-CH₂NHCH₂); 4.20-4.36 (m, 2H, piperidine); 7.03 (d, J=3.9 Hz,1H, bromothiophene H3); 7.08 (d, J=3.9 Hz, 1H, bromothiophene H4); 7.23 (d, J=8.0 Hz, 1H, pyridine H3); 7.49 (dd, J=8.0 Hz, 1.9 Hz, 1 H, pyridine H4); 8.39-8.43 (m, 1H, pyridine H6).¹³C NMR (CDCl₃, 360 MHz) δ (ppm): 18.1; 33.2; 33.4; 41.2; 55.2; 56.8; 57.0; 93.5; 95.4; 116.4; 121.7; 129.1; 129.6; 131.4; 137.1;
138.8; 149.7; 156.6; 162.3.
HR-MS: C₁₈H₂₁BrFN₃OS; calculated: 425.0573; found: 425.0576.

### Example E8:

### 4,5-Dibromothien-2-yl-[4-fluoro-4-[[(5-methylpyridin-2-ylmethyl)amino]methyl]-piperidin-1-yl]-methanone

Synthesis worked according to the preparation of E1 when using 4,5-dibromothiophene-2-carboxylic acid (C8).
Yield: 12.3 mg (44%) light yellow oil.
MS (APCI): m/z 490 (M+1)⁺. IR (NaCl) v (cm⁻¹): 3328, 2923, 1619, 1434, 1373, 1307, 1268, 1130, 964, 914, 802, 744. ¹H NMR(CDCl₃, 360 MHz) δ (ppm): 1.62-1.87 (m, 2H, piperidine); 2.05-2.19 (m, 2H, piperidine); 2.36 (s, 3H, pyridine-CH₃); 2.82 (d, J=20.2 Hz, 2H, piperidine-CH₂NHCH₂); 3.13-3.61 (m, 2H, piperidine); 3.94 (s, 2H, piperidine-CH₂NHCH₂); 4.16-4.58 (m, 2H, piperidine); 7.02 (s, 1H, thiophene H3); 7.23 (d, J=7.9 Hz, 1 H, pyridine H3); 7.48 (dd, J=8.0 Hz, 1.9 Hz, 1H, pyridine H4); 8.39-8.44 (m, 1 H, pyridine H6).

### Example E9:

### [4-Fluoro-4-[[(5-methylpyridin-2-ylmethyl)amino]methyl]piperidin-1-yl]-3-methylthien-2-yl-methanone

Synthesis worked according to the preparation of E1 when using 3-methylthiophene-2-carboxylic acid (C9).
Yield: 9.8 mg (48%) light yellow oil.
MS (APCI): m/z 362 (M+1)⁺. IR (NaCl) v (cm⁻¹): 3748; 2923; 1627; 1438; 1276; 1103; 968; 825; 721. ¹H NMR (CDCl₃, 600 MHz) δ (ppm): 1.75-1.89 (m, 2H, piperidine); 1.99-2.09 (m, 2H, piperidine); 2.27 (s, 1H, thiophone-CH₃); 2.33 (s, 3H, pyridine-CH₃); 2.79 (d, J=20.8 Hz, 2H, piperidine-CH₂NHCH₂); 3.23-3.37 (m, 2H, piperidine); 3.90 (s, 2H, piperidine-CH₂NHCH₂); 3.95-4.50 (m, 2H, piperidine); 6.84 (d, J=4.9 Hz, 1H, thiophene H4); 7.22 (d, J = 7.6 Hz, 1 H, pyridine H3); 7.25-7.27 (m, 1H, thiophene H5); 7.47 (dd, J=7.9 Hz, 1.5 Hz, 1H, pyridine H4); 8.37-8.41 (m, 1H, pyridine H6). ¹³C NMR (CDCl₃, 360 MHz) δ (ppm): 14.6; 18.1; 33.3; 33.5; 40.1; 55.2; 56.9; 57.1; 93.6; 95.5; 121.7; 125.7; 129.7; 130.4;
131.4; 137.0; 137.3; 149.7; 156.6; 164.6.
HR-MS: C₁₉H₂₄FN₃OS; calculated: 361.1624; found: 361.1624.

### Example E10:

### [4-Fluoro-4-[[(5-methylpyridin-2-ylmethyl)amino]methyl]piparidin-1-yl]-4-methylthien-2-yl-mothanone

Synthesis worked according to the preparation of E1 when using 4-methylthiophene-2-carboxylic acid (C10).
Yield: 8.5 mg (41%) light yellow oil.
MS (APCI): m/z 362 (M+1)⁺. IR (NaCl) v (cm⁻¹): 3332; 1923; 1619; 1442; 1272; 1130; 1079; 860; 825; 763. ¹H NMR (CDCl₃, 600 MHz) δ (ppm): 1.77-1.88 (m, 2H, piperidine); 2.01-2.10 (m, 2H, piperidine); 2.28 (s, 3H, thiophene-CH₃); 2.33 (s, 3H, pyridine-CH₃); 2.80 (d, J=20.4 Hz, 2H, piperidine-CH₂NHCH₂); 3.24-3.44 (m, 2H, piperidine); 3.92 (s, 2H, piperidine-CH₂NHCH₂); 4.21-4.42 (m, 2H, piperidine); 7.02 (s,1H, methylthiophene H5); 7.10 (s, 1H, methylthiophene H3); 7.21 (d, J=7.9 Hz, 1H, pyridine H3); 7.47 (dd, J=7.7 Hz, 1.3 Hz, 1H, pyridine H4); 8.37-8.41 (m, 1H, pyridine H6). ¹³C NMR (CDCl₃, 600 MHz) δ (ppm): 15.6; 18.1; 33.4; 55.2; 56.9; 57.0; 94.0; 95.1; 121.7; 123.9; 130.9; 131.4; 136.7;
137.0; 149.7; 156.6; 163.7.
HR-MS: C₁₉H₂₄FN₃OS; calculated: 361.1624; found: 361.1624.

### Example E11:

### [4-Fluoro-4-[[(5-methylpyridin-2-ylmethyl)amino]methyl]piperidin-1-yl]-5-mothylthiophon-2-yl-mothanone

Synthesis worked according to the preparation of E1 when using 5-methylthiophene-2-carboxylic acid (C11).
Yield: 5.9 mg (29%) light yellow oil.
MS (APCI): m/z 362 (M+1)⁺. IR (NaCl) v (cm⁻¹): 3567; 2923; 1616; 1461; 1427; 1276; 1091; 809; 732. ¹H NMR (CDCl₃, 360 MHz) δ (ppm): 1.69-1.86 (m, 2H, piperidine); 2.02-2.09 (m, 2H, piperidine); 2.33 (s, 3H, pyridine-CH₃); 2.51 (s, 3H, thiophene-CH₃); 2.79 (d, J=20.4 Hz, 2H, piperidine-CH₂NHCH₂); 3.27-3.42 (m, 2H, piperidine); 3.92 (s, 2H, piperidine-CH₂NHCH₂); 4.25-4.38 (m, 2H, piperidine); 6.70 (dd, J=3.6 Hz, 0.9 Hz, 1H, thiophene H4); 7.11 (d, J=3.8 Hz,1 H, thiophene H3); 7.22 (d, J=7.9 Hz, 1H, pyridine H3); 7.47 (dd, J=7.9 Hz, 1.8 Hz, 1H, pyridine H4); 8.37- 8.41 (m, 1H, pyridine H6). ¹³C NMR (CDCl₃, 360 MHz) δ (ppm): 15.3; 18.1; 33.3; 33.5; 41.5; 55.2; 56.9; 57.1; 93.7; 95.5; 121.7; 125.0; 129.2; 131.4; 134.7; 137.0; 143.7; 149.7; 156.6; 163.6.
HR-MS: C₁₉H₂₄FN₃OS; calculated: 361.1624; found: 361.1624.

### Example E12:

### [4-Fluoro-4-[[(5-methylpyridin-2-ylmethyl)amino]methyl]piperidin-1-yl]-5-mothylthiothien-2-yl-methanone

Synthesis worked according to the preparation of E1 when using 5-methylthiathiaphene-2-carboxylic acid (C12).
Yield: 15.5 mg (68 %) light yellow oil.
MS (APCI): m/z 394 (M+1)⁺ IR (NaCl) v (cm⁻¹): 3332; 2923; 1616; 1434; 1272; 1091; 968; 806; 748. ¹H NMR (CDCl₃, 360 MHz) δ (ppm): 1.61-1.75 (m, 2H, piperidine); 2.03-2.13 (m, 2H, piperidine); 2.36 (s, 3H, pyridine-CH₃); 2.57 (s, 3H, thiophene-SCH₃); 2.82 (d, J=20.4 Hz, 1H, piperidin-CH₂NHCH₂); 3.30-3.44 (m, 2H, piperidine); 3.94 (s, 2H, piperidine-CH₂NHCH₂); 4.24-4.36 (m, 2H, piperidine); 6.96 (d, J=3.6 Hz, 1H, thiophene H4); 7.16 (d, J=3.6 Hz, 1H, thiophene H3); 7.23 (d, J=7.9 Hz, 1H, pyridine H3); 7.48 (dd, J=7.8 Hz, 1.9 Hz, 1H, pyridine H4); 8.39-8.43 (m, 1H, pyridine H6). ¹³C NMR (CDCl₃, 600 MHz) δ (ppm): 18.1; 21.0; 33.3; 33.4; 41.0; 41.7; 55.1; 56.9; 57.0; 93.9; 95.1; 121.7; 128.5; 129.2; 131.4;
137.1; 137.9; 142.3; 149.7; 156.5; 162.8.
HR-MS: C₁₉H₂₄FN₃OS₂; calculated: 393.1345; found: 393.1343.

### Example E13:

### [4-Fluoro-4-[[(5-methylpyridin-2-ylmethyl)amino]methyl]piperidin-1-yl]-5-phenylthien-2-yl-mathanone

Synthesis worked according to the preparation of E1 when using 5-phenylthiophene-2-carboxylic acid (C13).
Yield: 15.4 mg (62 %) light yellow oil.
MS (APCI): m/z 424 (M+1)⁺. IR (NaCl) v (cm⁻¹): 3340; 2923; 1616; 1454; 1423; 1276; 1095; 968; 813; 755; 690. ¹H NMR (CDCl₃, 360 MHz) δ (ppm): 1.65-1.78 (m, 2H, piperidine); 2.05- 2.16 (m, 2H, piperidine); 2.36 (s, 3H, pyridine-CH₃); 2.84 (d, J=20.2 Hz, 2H, piperidine-CH₂NHCH₂); 3.31-3.48 (m, 2H, piperidine); 3.95 (s, 2H, piperidine-CH₂NHCH₂); 4.29-4.44 (m, 2H, piperidine); 7.24 (d, J=7.7 Hz, 1H, pyridine H3); 7.25 (d, J=3.6 Hz, 1H, thiophene H4); 7.30 (d, J=3.6 Hz, 1H, thiophene H3); 7.33-7.39 (m, 1 H, phenyl H4); 7.40-7.46 (m, 2H, phenyl H3, H5); 7.49 (dd, J=7.9 Hz, 1.8 Hz, 1H, pyridine H4); 7.61-7.67 (m, 2H, phenyl H2, H6); 8.40-8.43 (m, 1H, pyridine H6). ¹³C NMR (CDCl₃, 600 MHz) δ (ppm): 18.1; 33.3; 33.4; 40.8; 41.6; 55.1; 56.9; 57.0; 94.0; 95.1; 121.7; 122.6; 126.1; 128.3; 129.0; 129.8; 131.4; 133.5; 136.0; 137.1; 147.6; 149.7; 156.5; 163.4.

HR-MS: C₂₄H₂₆FN₃OS; calculated: 423.1781; found: 423.1781.

### Example E14:

### [4-Fluoro-4-[[(5-methylpyridin-2-ylmethyl)amino]methyl]piperidin-1-yl]-5-pyridin-2-ylthien-2-yl-methanone

Synthesis worked according to the preparation of E1 when using 5-pyridin-2-ylthiophene-2-carboxylic acid (C14).
Yield: 18.1 mg (74 %) light yellow oil.
MS (APCI): m/z 425 (M+1)⁺. IR (NaCl) v (cm⁻¹): 3324; 2923; 1616; 1454; 1430; 1276; 775; 748. ¹H NMR (CDCl₃, 360 MHz) δ (ppm): 1.64-1.82 (m, 2H, piperidine); 2.04-2.17 (m, 2H, piperidine); 2.36 (s, 3H, pyridlne-CH₃); 2.83 (d, J=20.4 Hz, 2H, piperidine-CH₂NHCH₂); 3.31-3.46 (m, 2H, piperidine); 3.95 (s, 2H, piperidine-CH₂NHCH₂); 4.30-4.45 (m, 2H, piperidine); 7.20-7.27 (m, 2H, pyridine H3, pyridine H5'); 7.36 (d, J=3.9 Hz, 1H, thiophene H4); 7.49 (dd, J=7.8 Hz, 1.7 Hz, 1H, pyridine H4); 7.53 (d, J=3.9 Hz, 1H, thiophene H3); 7.70 (ddd, J=8.0 Hz, 1.6 Hz, 0.9 Hz, 1H, pyridine H3'); 7.75 (ddd, J=8.0 Hz, 7.2 Hz, 1.8 Hz, 1H, pyridine H4'), 8.40- 8.43 (m, 1H, pyridine H6) 8.61 (ddd, J = 4.9 Hz, 1.8 Hz, 1.0 Hz, 1H, pyridine H6'). ¹³C NMR (CDCl₃, 600 MHz) δ (ppm): 18.1; 33.3; 33.5; 41.4; 55.1; 56.9; 57.0; 93.9; 95.1; 119.1; 121.7; 122.6; 123.9; 130.2; 131.4; 136.8; 137.1; 137.9;
147.4; 149.7; 149.7; 151.8; 156.5; 163.4.
HR-MS: C₂₃H₂₅FN₄OS; calculated: 424.1733; found: 424.1733.

### Example E15:

### [2,2']Bithien-5-yl-[4-fluoro-4-[[(5-methylpyridin-2-ylmethyl)amino]methyl]piperidin-1-yl]-methanone

Synthesis worked according to the preparation of E1 when using [2,2']bithiophenyl-5-carboxylic acid (C15).
Yield: 15.7 mg (63 %) light yellow oil.
MS (APCI): m/z 430 (M+1)⁺. IR (NaCl) v (cm⁻¹): 3340; 2923; 1616; 1457; 1427; 1272; 1091; 968; 806; 748; 701. ¹H NMR (CDCl₃, 360 MHz) δ(ppm): 1.64-1.77 (m, 2H, piperidine); 2.04-2.15 (m, 2H, piperidine); 2.36 (s, 3H, pyridine-CH₃); 2.83 (d, J=20.4 Hz, 2H, piperidine-CH₂NHCH₂); 3.33-3.46 (m, 2H, piperidine); 3.94 (s, 2H, piperidine-CH₂NHCH₂); 4.29-4.41 (m, 2H, piperidine); 7.07 (dd, J = 5.0 Hz, 3.6 Hz,1H, bithiophene H4'); 7.12 (d, J=3.9 Hz, 1H, bithiophene H3'); 7.23-7.27 (m, 3H, bithiophene H4, H5', pyridine H3); 7.30-7.32 (m, 1H, bithiophene H3); 7.49 (dd, J=7.8 Hz, 1.7 Hz, 1H, pyridine H4); 8.40-8.43 (m, 1H, pyridine H6). ¹³C NMR (CDCl₃, 360 MHz) δ (ppm): 18.1, 33.3; 33.5; 41.5; 55.2; 56.8; 57.1; 93.6; 95.5; 121.7; 123.1; 124.8; 125.5; 128.0; 129.6; 131.4; 135.5; 136.3; 137.1; 140.7; 149.7; 156.6; 163.1.
HR-MS: C₂₂H₂₄FN₃OS₂; calculated: 429.1345; found: 429.1346.

### Example E16:

### [4-Fluoro-4-[[(5-methylpyridin-2-ylmethyl)amino]methyl]piperidin-1-yl]-5-(2-methylthiazol-4-yl)thien-2-yl-methanone

Synthesis worked according to the preparation of E1 when using 5-(2-methylthiazol-4-yl)-thiophene-2-carboxylic acid (C16).
Yield: 16.7 mg (65 %) light yellow oil.
MS (APCI): m/z 445 (M+1)⁺. IR (NaCl) v (cm⁻¹): 3336; 2923; 1616; 1492; 1438; 1276; 1168; 1095; 968; 813; 748; 647. ¹H NMR (CDCl₃, 360 MHz) δ (ppm): 1.64-1.80 (m, 2H, piperidine); 2.04-2.15 (m, 2H, piperidine); 2.35 (s, 3H, pyridine-CH₃); 2.78 (s, 3H, thiazole-CH₃); 2.82 (d, J=20.4 Hz, 2H, piperidine-CH₂NHCH₂); 3.32-3.45 (m, 2H, piperidine); 3.94 (s, 2H, pipeddine-CH₂NHCH₂); 4.30-4.42 (m, 2H, piperidine); 7.23 (d, J=7.9 Hz, 1H, pyridine H3); 7.28-7.37 (m, 2H, thiophene H4, thiazole H5); 7.37 (d, J=3.8 Hz, 1H, thiophene H3); 7.49 (dd, J=7.8 Hz, 1.7 Hz, 1H, pyridine H4); 8.40-8.43 (m, 1H, pyridine H6). ¹³C NMR (CDCl₃, 360 MHz) δ (ppm): 18.1; 19.2; 33.3; 33.5; 41.5; 55.2; 56.8; 57.1; 93.6; 95.5; 112.6; 121.7; 123.3; 129.9; 131.4; 135.8; 137.1; 141.2; 148.6; 149.7; 156.6;
163.4; 166.5.
HR-MS: C₂₂H₂₅FN₄OS₂; calculated: 444.1454; found: 444,1455.

### Example E17:

### [4-Fluoro-4-[[(5-methylpyridin-2-ylmethyl)amino]methyl]piperidin-1-yl]-3-pyrrol-1-ylthion-2-yl-methanone

Synthesis worked according to the preparation of E1 when using 3-pyrrol-1-ylthiophene-2-carboxylic acid (C17).
Yield: 17.6 mg (74 %) light yellow oil.
MS (APCI): m/z 413 (M+1)⁺. IR (NaCl) v (cm⁻¹): 3340; 2923; 1627; 1558; 1488; 1450; 1272; 1083; 748. ¹H NMR (CDCl₃, 360 MHz) 6 (ppm): 1.42-1.70 (m, 2H, pipendine); 1.88-2.19 (m, 2H, piperidine); 2.36 (s, 3H, pyridine-CH₃); 2.64 (d, J=20.2 Hz, 1H, piperidine-CH₂NHCH₂); 2.96-3.13 (m, 2H, piperidine); 3.21-3.40 (m, 1H, piperidine); 3.88 (s, 2H, piperidine-CH₂NHCH₂); 4.41-4.68 (m, 1H, piperidine); 6.24-6.28 (m, 2H, pyrrole H3, H4); 6.90-6.93 (m, 2H, pyrrole H2, H5); 7.06 (d, J=5.2 Hz, 1H, thiophene H4); 7.21 (d, J=7.9 Hz,1H, pyridine H3); 7.44 (d, J=5.2 Hz, 1H, thiophene H5); 7.49 (dd, J=7.8 Hz, 1.7 Hz, 1H, pyridine H4); 8.39-8.43 (m, 1H, pyridine H6). ¹³C NMR (CDCl₃, 360 MHz) 6 (ppm): 18.1; 32.2; 32.4; 38.1; 42.7; 55.1; 56.6; 56.9; 93.4; 95.3; 110.3; 120.9; 121.6; 123.0; 123.7;
126.8; 131.4; 136.8; 137.0; 149.6; 156.7; 162.2.
HR-MS: C₂₂H₂₅FN₄OS; calculated: 412.1733; found: 412.1733.

### Example E18:

### [4-Fluoro-4-[[(5-methylpyridin-2-ylmethyl)amino]methyl]piperidin-1-yl]-thien-3-yl-methanone

Synthesis worked according to the preparation of E1 when using thiophene-3-carboxylic acid (C18).
Yield: 14.2 mg (72 %) light yellow oil.
MS (APCI): m/z 348 (M+1)⁺. IR (NaCl) v (cm⁻¹): 3513; 2923; 1627; 1438; 1276; 1103; 975; 813; 740. ¹H NMR (CDCl₃, 360 MHz) δ (ppm): 1.77-1.87 (m, 2H, piperidine); 1.98-2.14 (m, 2H, piperidine); 2.35 (s, 3H, pyridine-CH₃); 2.82 (d, J=20.2 Hz, 2H, piperidine-CH₂NHCH₂); 3.04-3.59 (m, 2H, piperidine); 3.94 (s, 2H, piperidine-CH₂NHCH₂); 4.22-4.92 (m, 2H, piperidine); 7.20 (dd, J=5.0 Hz, 1.1 Hz, 1H, thiophene H4); 7.23 (d, J=7.9 Hz, 1 H, pyridine H3); 7.36 (dd, J=5_{.}0 Hz, 3.0 Hz, 1H, thiophene H5); 7.49 (dd, J=8.0 Hz, 1.7 Hz, 1H, pyridine H4); 7.54 (dd, J=3.0 Hz, 1.1 Hz, 1H, thiophene H2); 8.39-8.43 (m, 1H, pyridine H5). ¹³C NMR (CDCl₃, 360 MHz) δ (ppm): 18.1; 33.5; 38.2; 43.2; 55.2; 56.8; 57.1; 93.6; 95.5; 121.7; 125.9; 126.2; 126.9; 131.4; 136.5; 137.1; 149.7; 156.6; 165.8.

HR-MS: C₁₈H₂₂FN₃OS; calculated: 347.1468; found: 347.1468.

### Example E19:

### 2-Bromothien-3-yl-[4-fluoro-4-[[(5-methylpyridin-2-ylmethyl)amino]methyl]piperidin-1-yl]-methanone

Synthesis worked according to the preparation of E1 when using 2-bromothiophene-3-carboxylic acid (C19).
Yield: 12.1 mg (49 %) light yellow oil.
MS (APCI): m/z 427 (M+1)⁺. IR (NaCl) v (cm⁻¹): 3332; 2923; 1635; 1442; 1276; 1037; 875; 752; 647. ¹H NMR (CE)Cl₃, 360 MHz) δ (ppm): 1.57-1.75 (m, 2H, piperidine); 1.96-2.19 (m, 2H, piperidine); 2.35 (s, 3H, pyridine-CH₃); 2.82 (dd, J=20.3 Hz, 1.7 Hz, 1H, piperidine-CH₂NHCH₂); 3.15-3.27 (m, 1H, piperidine); 3.37-3.55 (m, 2H, piperidine); 3.94 (s, 2H, pipeddine-CH₂NHCH₂); 4.54-4.64 (m, 1H, piperidine); 6.94 (d, J=5.7 Hz, 1H, thiophene H5); 7.23 (d, J=7.7 Hz, 1H, pyridine H3); 7.32 (d, J=5.7 Hz, 1H, thiophene H4); 7.49 (dd, J=7.9 Hz, 1.8 Hz, 1H, pyridine H4); 8.39-8.44 (m, 1H, pyridine H6). ¹³C NMR (CDCl₃, 360 MHz) δ (ppm): 18.1; 32.7; 32.9; 33.8; 34.0; 37.8; 42.9; 55.2; 56.8; 57.0; 93.5; 95.4; 110.6; 121.7; 127.0; 127.4; 131.4; 137.1; 137.1; 149.7; 156.5; 164.3.
HR-MS: C₁₈H₂₁BrFN₃OS; calculated: 425.0573; found: 425.0573.

### Example E20:

### [4-Fluoro-4-[[(5-mothylpyridin-2-ylmothyl)amino]methyl]piperidin-1-yl]-4-methoxythien-3-yl-methanone

Synthesis worked according to the preparation of E1 when using 4-methoxythiophene-3-carboxylic acid (C20).
Yield: 12.2 mg (59 %) light yellow oil.
MS (APCI): m/z 378 (M+1)⁺. IR (NaCl) v (cm⁻¹). 3328; 2927; 1631; 1554; 1418; 1434; 1376; 1276; 1245; 1207; 1091; 802; 759. ¹H NMR (CDCl₃, 360 MHz) δ (ppm): 1.52-1.76 (m, 2H, piperidine); 1.92-2.15 (m, 2H, piperidine); 2.35 (s, 3H, pyridine-CH₃); 2.82 (d, J=20.4 Hz, 1H, piperidine-CH₂NHCH₂); 3.10-3.26 (m, 1H, piperidine); 3.29-3.43 (m, 1H, piperidine); 3.48-3.62 (m, 1H, piperidine); 3.85 (s, 3H, thlophene-OCH₃); 3.94 (s, 2H, piperidine-CH₂NHCH₂); 4.47-4.64 (m, 1H, piperidine); 6.28 (d, J=3.4 Hz, 1H, thiophene H5); 7.23 (d, J=7.7Hz, 1H, pyridine H3); 7.37(d, J=3.1 Hz, 1H, thiophene H2); 7.49 (dd, J=7.8 Hz, 1.7 Hz, 1H, pyridine H4); 8.3-8.43 (m, 1H, pyridine H6). ¹³C NMR (CDCl₃, 600 MHz) δ(ppm): 18.1; 32.8; 32.9; 33.6; 33.8; 37.7; 42.9; 55.1; 56.9; 57.0; 94.0; 95.2; 97.3; 121.7; 125.2; 128.7; 131.4; 137.1; 149.7; 155.1; 156.5; 164.1.

HR-MS: C₁₉H₂₄FN₃O₂S; calculated: 377.1573; found: 377.1572.

### Example E21:

### 5-Chloro-4-methoxythien-3-yl-[4-fluoro-4-[[(5-methylpyridin-2-ylmethyl)amino]methyl]piperidin-1-yl]-methanone

Synthesis worked according to the preparation of E1 when using 5-chloro-4-methoxythiophene-3-carboxylic acid (C21).
Yield: 15.1 mg (64 %) light yellow oil.
MS (APCI): m/z 412 (M+1)⁺. IR (NaCl) v (cm⁻¹): 3328; 2927; 1635; 1550; 1481; 1434; 1361; 1272; 1245; 1033; 975; 790; 748. ¹H NMR (CDCl₃, 360 MHz) δ (ppm): 1.56-1.76 (m, 2H, piperidine); 1.94-2.03 (m, 1H, piperidine); 2.07-2.14 (m, 1H, piperidine); 2.33 (s, 3H, pyridine-CH₃); 2.80 (d, J=20.0 Hz, 1H, piperidine-CH₂NHCH₂); 3.10-3.20 (m, 1 H, piperidine); 3.33-3.41 (m, 1H, piperidine); 3.51-3.58 (m, 1H, piperidine); 3.90 (s, 3H, 0-CH3); 3.91 (s, 2H, piperidine-CH₂NHCH₂); 4.49- 4.56 (m, 1H, piperidine); 7.13 (s, 1H, thiophene H2); 7.21 (d, J=7.9 Hz, 1H, pyridine H3); 7.47 (dd, J=7.8 Hz, 1.8 Hz, 1H, pyridine H4); 8.38-8.40 (m, 1 H, pyridine H6). ¹³C NMR (CDCl₃, 360 MHz) δ (ppm): 18.1; 32.7; 32.9; 33.6; 33.8; 37.9; 43.1; 55.2; 56.8; 57.0; 61.5; 93.5; 95.4; 114.6; 120.5; 121.7; 130.9; 131.4; 137.0; 149.7; 150.1; 156.6; 163.6.
HR-MS: C₁₉H₂₃CIFN₃O₂S; calculated: 411.1183; found: 411.1182.

### Example E22:

### 5-Bromo-4-methoxythion-3-yl-[4-fluoro-4-[[(5-methylpyridin-2-ylmethyl)amino]methyl]piperidin-1-yl]-methanone

Synthesis worked according to the preparation of E1 when using 5-bromo-4-methoxythiophene-3-carhoxylic acid (C22).
Yield: 14.5 mg (56 %) light yellow oil.
MS (APCI): m/z 457 (M+1)⁺. IR (NaCl) v (cm⁻¹): 3324; 2927; 1635; 1546; 1481; 1434; 1353; 1272; 1091; 1025; 752. ¹H NMR (CDCl₃, 360 MHz) δ (ppm): 1.56-1.81 (m, 2H, piperidine); 2.02-2.19 (m, 2H, piperidine); 2.35 (s, 3H, py(idine-CH₃); 2.82 (d, J=20.2 Hz, 2H, piperidine-CH₂NHCH₂); 3.12-3.24 (m, 1H, piperidine); 3.33-3.4 (m, 1H, piperidine); 3.51-3.61 (m, 1 H, piperidine); 3.90 (s, 3H, O-CH3); 3.94 (s, 2H, piperidine-CH₂NHCₜl₂); 4.50-4.60 (m, 1H, piperidine); 7.23 (d, J=7.9 Hz, 1H, pyridine H3); 7.33 (s, 1H, thiophene H2); 7.49 (dd, J=7.8 Hz, 1.7 Hz, 1 H, pyridine H4); 8.39-8.42 (m, 1 H, pyridine H6). ¹³C NMR (CDCl₃, 600 MHz) δ (ppm): 18.1; 32.7; 32.9; 33.6; 33.8; 37.9; 43.1; 55.1; 56.8; 57.0; 61.2; 93.8; 95.0; 98.4; 121.7; 124.0; 131.2; 131.5; 137.1; 149.6; 152.4; 156.4; 163.6.
HR-MS: C₁₉H₂₃BrFN₃O₂S; calculated: 455.0678; found: 455.0675.

### Example E23:

### Benzo[b]thien-2-yl-[4-fluoro-4-[[(5-methyl-pyridin-2-ylmethyl)amino]methyl]-piperidin-1-yl]-methanone

Synthesis worked according to the preparation of E1 when using benzolb]thlophene-2-carboxylic acid (C23).
Yield: 18.2 mg (80 %) light yellow oil.
MS (APCI): m/z 398 (M+1)⁺. IR (NaCl) v (cm⁻¹): 2923; 2360; 2333; 1623; 1523; 1442; 1272; 752. ¹H NMR (CDCl₃, 360 MHz) δ (ppm): 1.78-1.84 (m, 2H, piperidine); 2.04-2.16 (m, 2H, piperidine); 2.36 (s, 3H, pyridine-CH₃); 2.84 (d, J=20.2 Hz, 2H, piperidine-CH₂NHCH₂); 3.28-3.52 (m, 2H, piperidine); 3.95 (s, 2H, piperidine-CH₂NHCH₂); 4.18-4.59 (m, 2H, piperidine); 7.24 (d, J=7.9 Hz, 1H, pyridine H3); 7.39-7.46 (m, 2H, benzothiophene H5, H6); 7.46-7.53 (m, 2H, pyridine H4, benzothiophene H3); 7.81-7.92 (m, 2H, benzothiophene H4, H7); 8.39-8.45 (m, 1H, pyridine H6). ¹³C NMR (CDCl₃, 360 MHz) δ (ppm): 18.1; 33.3; 33.5; 55.2; 56.8; 57.1; 93.5; 95.4; 121.7; 122.4; 124.6; 124.8; 125.0; 125.8; 131.4; 136.7; 137.1; 138.7; 140.1; 149.7; 156.5; 163.8.
HR-MS: C₂₂H₂₄FN₃OS; calculated: 397.1624; found: 397.1626.

### Example E24:

### 5-Bromobenzo[b]thien-2-yl-[4-fluoro-4-[[(5-methylpyridin-2-ylmothyl)amino]methyl]piporidin-1-yl]-methanone

Synthesis worked according to the preparation of E1 when using 5-bromo-benzo[b]thiophene-2-carboxylic acid (C24).
Yield: 4.0 mg (15%) light yellow oil.
MS (APCI): m/z 477 (M+1)⁺. ¹H NMR (CDCl₃, 600 MHz) δ (ppm): 1.72-1.81 (m, 2H, piperidine); 2.05-2.10 (m, 2H, piperidine); 2.34 (s, 3H, pyridine-CH₃); 2.82 (d, J=20.6 Hz, 2H, piperidine-CH₂NHCH₂); 3.09-3.63 (m, 2H, piperidine); 3.93 (s, 2H, piperidine-CH₂NHCH₂); 3.99-4.76 (m, 2H, piperidine); 7.22 (d, J=7.9 Hz, 1H, pyridine H3); 7.40 (s, 1H, benzothiophene H4); 7.47 (dd, J=7.7 Hz, 1.7 Hz, 1 H, pyridine H4); 7.51 (dd, J=8.7 Hz, 1.9 Hz, 1H, benzothiophene H6); 7.72 (d, J=8.7 Hz, 1H, benzothiophene H7); 7.97 (d, J=1.9 Hz, 1 H, benzothiophene H3); 8.39-8.41 (m, 1H, pyridine H6).

### Example E25:

### Benzo[b]thien-3-yl-[4-fluoro-4-[[(5-methylpyridin-2-ylmethyl)amino]methyl]piperidin-1-yl]-methanone

Synthesis worked according to the preparation of E1 when using benzo[b]thiophene-3-carboxylic acid (C25).
Yield: 19.4 mg (86 %) light yellow oil.
MS (APCI): m/z 398 (M+1)⁺. IR (NaCl) v (cm⁻¹): 3332; 2923; 1631; 1515; 1434; 1272; 1238; 1126; 763; 748. ¹H NMR (CDCl₃, 360 MHz) δ (ppm): 1.50-1.81 (m, 2H, piperidine); 1.95-2.20 (m, 2H, piperidine); 2.35 (s, 3H, pyridine-CH₃); 2.83 (d, J=20.2 Hz,2H, piperidine-CH₂NHCH₂); 3.26-3.45 (m, 2H, piperidine); 3.56-3.83 (m, 1H, piperidine); 3.93 (s, 2H, piperidine-CH₂NHCH₂); 4.21-5.06 (m, 1H, piperidine); 7.23 (d, J=7.7 Hz, 1H, pyridine H3); 7.39-7.47 (m, 2H, benzothiophene H5, H6); 7.49 (dd, J=7.8 Hz, 1.7 Hz, 1H, pyridine H4); 7.59 (s, 1H, benzothiophene H2); 7.80-7.86 (m, 1H, benzothiophene H7); 7.88-7.93 (m, 1H, benzothiophene H4); 8.39-8.43 (m, 1H, pyridine H6). ¹³C NMR (CDCl₃, 360 MHz) δ (ppm): 18.1; 33.1; 33.9; 38.0; 43.3; 55.1; 56.8; 57.0; 93.9; 95.0; 121.6; 122.6; 122.9; 124.9; 125.0; 126.4; 131.4; 131.8; 137.1; 139.8; 149.6; 156.5; 164.4.
HR-MS: CH₂₂H₂₄FN₃OS; calculated: 397.1624; found: 397.1624.

### Example E26:

### [4-Fluoro-4-[[(5-methylpyridin-2-ylmethyl)amino]methyl]piperidin-1-yl]-thieno[3,2-b]thien-2-yl-mothanone

Synthesis worked according to the preparation of E1 when using thieno[3,2-b]thiophene-2-carboxylic acid (C26).
Yield: 7.5 mg (33 %) light yellow oil.
MS (APCI): m/z 404 (M+1)⁺. ¹H NMR (CDCl₃, 360 MHz) δ(ppm): 1.76-1.85 (m, 2H, piperidine); 2.05-2.16 (m, 2H, piperidine); 2.36 (s, 3H, pyridine-CH₃); 2.83 (d, J=20.2 Hz, 2H, piperidine-CH₂NHCH2); 3.34-3.48 (m, 2H, piperidine); 3.94 (s, 2H, piperidine-CH₂NHCH₂); 4.32-4.44 (m, 2H, piperidine); 7.24 (d, J=7.9 Hz, 1H, pyridine H3); 7.30-7.32 (m, 1H, thienothiophene H6); 7.46-7.54 (m, 3H, thienothiophene H3, H5, pyridine H4); 8.39-8.43 (m, 1H, pyridine H6). ¹³C NMR (CDCl₃, 360 MHz) δ (ppm): 18.1; 33.3; 33.5; 55.2; 56.9; 57.1; 93.6; 95.5; 119.5; 121.1; 121.7; 129.7; 131.4; 137.1; 138.4; 138.5; 141.1; 149.7; 156.6; 163.5.
HR-MS: C₂₀H₂₂FN₃OS₂; calculated: 403.1188; found: 403.1186.

### Example E27:

### [4-Fluoro-4-[[(5-methylpyridin-2-ylmethyl)amino]methyl]piperidin-1-yl]-thieno[2,3-b]thien-2-yl-methanone

Synthesis worked according to the preparation of E1 when using thieno[2,3-b]thiophene-2-carboxylic acid (C27).
Yield: 16.4 mg (69 %) light yellow oil.
MS (APCI): m/z 404 (M+1)⁺. IR (NaCl) v (cm⁻¹): 3390; 2360; 2337; 1616; 1523; 1438; 1272; 1095; 968; 748; 647. ¹H NMR (CDCl₃, 360 MHz) δ (ppm): 1.64-1.78 (m, 2H, piperidine); 2.05-2.16 (m, 2H, piperidine); 2.35 (s, 3H, pyridine-CH₃); 2.83 (d, J=20.2 Hz, 2H, piperidin-CH₂NHCH₂); 3.34-3.48 (m, 2H, piperidine); 3.94 (s, 2H, piperidin-CH₂NHCH₂); 4.32-4.44 (m, 2H, piperidine): 7.23 (d, J=8.0 Hz,1H, pyridine H3); 7.26 (d, J=5.4 Hz, 1H, thienothiophene H4); 7.41 (d, J=5.2 Hz, 1H, thienothiophene H5); 7.45 (s, 1H, thienothiophene H3); 7.48 (dd, J=7.9 Hz, 1.6 Hz, 1H, pyridine H4); 8.39-8.43 (m, 1H, pyridine H6). ¹³C NMR (CDCl₃, 360 MHz) δ (ppm): 18.1; 33.3; 33.5; 41.5; 55.2; 56.8; 57.1; 93.6; 95.5; 120.2; 121.3; 121.7; 128.8; 131,4; 137.1; 139.9; 140.0; 145.8; 149.7; 156.6; 163.4.
HR-MS: C₂₀H₂₂FN₃OS₂; calculated: 403.1188; found: 403.1187.

### Example E28:

### 3-Chlorothieno[2,3-b]thion-2-yl-[4-fluoro-4-[[(5-methylpyridin-2-ylmothyl)amino]methyl]piperidin-1-yl]-mothanone

Synthesis worked according to the preparation of E1 when using 3-chlorothieno[2,3-b]thlophene-2-carboxylic acid (C28).
Yield: 20.6 mg (81 %) light yellow oil.
MS (APCI): m/z 438 (M+1)⁺. IR (NaCl) v (cm⁻¹): 3340; 2923; 1631; 1488; 1438; 1380; 1276; 1110; 748; 667. ¹H NMR (CDCl₃, 360 MHz) δ (ppm): 1.65-1.86 (m, 2H, piperidine); 2.03-2.16 (m, 2H, piperidine); 2.35 (s, 3H, pyridine-CH₃); 2.83 (d, J=20.4 Hz, 2H, piperidin-CH₂NHCH₂); 3.25-3.56 (m, 2H, piperidine); 3.94 (s, 2H, piperidin-CH₂NHCH₂); 4.00-4.61 (m, 2H, piperidine); 7.23 (d, J=7.9 Hz,1H, pyridine H3); 7.24 (d, J=5.4 Hz, 1 H, thienothiophene H4); 7.45 (d, J=5.2 Hz, 1H, thienothiophene H5); 7.48 (dd, J=7.8 Hz, 1.9 Hz, 1H, pyridine H4); 8.39-8.43 (m, 1H, pyridine H6). ¹³C NMR (CDCl₃, 600 MHz) δ (ppm): 18.1; 29.7; 33.3; 38.6; 43.3; 55.2; 56.8; 57.0; 93.8; 95.0; 116.6; 119.1; 121.7; 129.5; 131.4; 132.2; 136.6; 137.1; 143.9; 149.7; 156.6; 161.2.
HR-MS: C₂₀H₂₁FN₃OS₂; calculated: 437.0799; found: 437.0799.

### Example E29:

### [4-Fluoro-4-[[(5-methylpyridin-2-ylmethyl)amino]methyl]pipendin-1-yl]-4,5,6,7-tetrahydrobenzo[c]thien-1-yl-methanone

Synthesis worked according to the preparation of E1 when using 4,5,6,7-tetrahydrobenzo[c]thiophene-1-carboxylic acid (C29).
Yield: 16.7 mg (70 %) light yellow oil.
MS (APCI): m/z 402 (M+1)⁺. IR (NaCl) v (cm⁻¹): 3328; 2927; 2857; 1627; 1454; 1276; 1118; 752. ¹H NMR (CDCl₃, 360 MHz) δ (ppm): 1.57-1.71 (m, 2H, piperidine); 1.71-7.83 (m, 4H, tetrahydrobenzothiophene H5, H6); 1.99-2.11 (m, 2H, pipeddine); 2.35 (s, 3H, pyridine-CH₃); 2.67-2.76 (m, 4H, tetrahydrobenzothiophene H4, H7); 2.81 (d, J=20.6 Hz, 2H, piperidine-CH₂NHCH₂); 3.25-3.37 (m, 2H, piperidine); 3.93 (s, 2H, piperidine-CH₂NHCH₂); 4.08-4.25 (m, 2H, piperidine); 6.94 (s, 1H, tetrahydrobenzothiophene H3); 7.23 (d, J=7.9 Hz,1H, pyridine H3); 7.48 (dd, J=7.8 Hz, 1.7 Hz, 1H, pyridine H4); 8.40-8.43 (m, 1H, pyridine H6). ¹³C NMR (CDCl₃, 360 MHz) δ (ppm): 18.1; 23.0; 23.1; 25.6; 26.1; 33.3; 33.6; 40.9; 55.2; 56.2; 57.1; 120.2; 121.7; 128.8; 131.4; 137.1; 138.6; 138.6; 149.7; 156.6; 164.8.
HR-MS: C₂₂H₂₆FN₃OS; calculated: 401.1937; found: 401.1937.

### Example E30:

### 7-Bromo-2,3-dihydrothieno[3,4-b][1,4]dioxin-5-yl-[4-fluoro-4-[[5-methylpyridin-2-ylmethyl)aminolmothyl]piperidin-1-yl]-methanone

Synthesis worked according to the preparation of E1 when using 7-bromo-2,3-dihydrothieno[3,4-b][1,4]dioxine-5-carboxylic acid (C30).
Yield: 9.7mg (35 %) light yellow oil.
MS (APCI): m/z 486 (M+1)⁺. IR (NaCl) v (cm⁻¹): 3324; 2923; 2873; 1612; 1496; 1442; 1361; 1276; 1087; 914; 748. ¹H NMR (CDCl₃, 360 MHz) δ (ppm): 1.75-1.87 (m, 2H, piperidine); 2.01-2.13 (m, 2H, piperidine); 2.36 (s, 3H, pyridine-CH₃); 2.82 (d, J=20.7 Hz, 2H, piperidine-CH₂NHCH₂); 3.25- 3.39 (m, 2H, piperidine); 3.94 (s, 2H, piperidine-CH₂NHCH₂); 4.03-4.21 (m, 2H, piperidine); 4.27-4.35 (m, 4H, dihydrothienodioxine H2, H3); 7.23 (d, J=7.9 Hz, 1 H, pyridine H3); 7.49 (dd, J=7.8 Hz, 1.7 Hz, 1H, pyridine H4); 8.39-8.43 (m, 1H, pyridine H6). ¹³C NMR (CDCl₃, 360 MHz) δ (ppm): 18.1; 33.3; 33.5; 41.3; 55.2; 56.9; 57.1; 64.6; 64.9; 92.0; 93.7; 95.5; 113.0; 121.7; 131.4; 137.0; 138.7; 138.9; 149.7; 156.6; 161.2.
HR-MS: C₂₀H₂₂BrFN₃O₃S; calculated: 483.0627; found: 483.0621.

### Example E31:

### [4-Fluoro-4-[[(5-methylpyridin-2-ylmethyl)amino]methyl]piperidin-1-yl]-furan-2-yl-methanone

Synthesis worked according to the preparation of E1 when using furan-2-carboxylic acid (C31 ).
Yield: 13.2 mg (28 %) light yellow oil.
MS (APCI): m/z 332 (M+1)⁺ IR (NaCl) v (cm⁻¹): 3490; 2923; 1627; 1488; 1434; 1373; 1284; 1110; 929; 825; 755. ¹H NMR (CDCl₃, 360 MHz) 6 (ppm): 1.63-1.91 (m, 2H, piperidine); 2.03-2.16 (m, 2H, piperidine); 2.35 (s, 3H, pyridine-CH₃); 2.82 (d, J=20.6 Hz, 2H, piperidine-CH₂NHCH₂); 3.09-3.59 (m, 2H, piperidine); 3.94 (s, 2H, piperidine-CH₂NHCH₂); 4.29-4.54 (m, 2H, piperidine); 6.50 (dd, J=3.4 Hz, 1.8 Hz, 1H, furan H4); 7.01 (dd, J=3.5 Hz, 0.8 Hz, 1H, furan H3); 7.24 (d, J=8.0 Hz, 1H, pyridine H3); 7.46-7.52 (m, 2H, pyridine H4, furan H5); 8.39-8.43 (m, 1 H, pyridine H6).
HR-MS: C₁₈H₂₂FN₃O₂; calculated: 331.1696; found: 331.1698.

### Example E32:

### 4,5-Dibromofuran-2-yl-[4-fluoro-4-[[(5-mothylpyridin-2-ylmethyl)amino]methyl]piperidin-1-yl]-methanone

Synthesis worked according to the preparation of E1 when using 4,5-dibromofuran-2-carboxylic acid (C32).
Yield: 13.3 mg (46%) light yellow oil.
MS (APCI): m/z 506 (M+1)⁺. IR (NaCl) v (cm⁻¹): 2923, 1631, 1488, 1434, 1280, 1218, 979, 771. ¹H NMR (CDCl₃, 360 MHz) δ (ppm): 1.60-1.82 (m, 2H, piperidine); 2.03-2.17 (m, 2H, piperidine); 2.36 (s, 3H, pyridine-CH₃); 2.82 (d, J=20.2 Hz, 2H, piperidine-CH₂NHCH₂); 3.28-3.49 (m, 2H, piperidine); 3.94 (s, 2H, piperidine-CH₂NHCH₂); 4.17-4.38 (m, 2H, piperidine); 7.11 (s, 1H, bromofuran H3); 7.23 (d, J=7.9 Hz, 1 H, pyridine H3); 7.49 (dd, J=7.8 Hz, 1.7 Hz, 1H, pyridine H4); 8.39-8.44 (m, 1H, pyridine H6).

### Example E33:

### [4-Fluoro-4-[[(5-methylpyridin-2-ylmethyl)amino]methyl]piperidin-1-yl]-furan-3-yl-methanone

Synthesis worked according to the preparation of E1 when using furan-3-carboxylic acid (C33).
Yield: 16.0 mg (85 %) light yellow oil.
MS (APCI): m/z 332 (M+1)⁺. IR (NaCl) v (cm⁻¹): 3405; 2927; 2360; 1623; 1442; 1373; 1292; 1072; 875; 748. ¹H NMR (CDCl₃, 360 MHz) δ (ppm): 1.59-1.84 (m, 2H, piperidine); 2.00-2.13 (m, 2H, piperidine); 2.36 (s, 3H, pyridine-CH₃); 2.91 (d, J=20.2 Hz,2H, piperidine-CH₂NHCH₂); 3.11-3.55 (m, 2H, piperidine); 4.03 (s, 2H, piperidine-CH₂NHCH₂); 4.11-4.81 (m, 2H, piperidine); 6.36 (dd, J=1.9 Hz, 0.8 Hz, 1H, furan H4); 7.25 (d, J=7.9 Hz, 1H, pyridine H3); 7.45 (dd, J=1.7 Hz, 1.7 Hz, 1H, furan H5); 7.52 (dd, J=7.8 Hz, 1.7 Hz, 1H, pyridine H4); 7.73 (dd, J=1.5 Hz, 1.0 Hz, 1 H, furan H2); 8.39-8.43 (m, 1H, pyridine H6). ¹³C NMR (CDCl₃, 360 MHz) δ (ppm): 18.1; 33.3; 38.4; 43.2; 54.7; 56.6;56.8; 93.3; 95.2; 110.0; 121.0; 121.8; 131.8; 137.3; 142.9; 143.3; 149.6; 155.4; 163.8.
HR-MS: C₁₈H₂₂FN₃O₂; calculated: 331.1696; found: 331.1696.

### Example E34:

### 5-Bromofuran-3-yl-[4.fluoro-4-[[(5-methylpyridin-2-ylmethyl)amino]methyl]piperidin-1-yl]-methanone

Synthesis worked according to the preparation of E1 when using 5-bromofuran-3-carboxylic acid (C34).
Yield: 14.7 mg (63 %) light yellow oil.
MS (APCI): m/z 412 (M+1)⁺. ¹H NMR (CDCl₃, 600 MHz) δ (ppm): 1.56-1.77 (m, 2H, piperidine); 1.99-2.16 (m, 2H, piperidine); 2.36 (s, 3H, pyridine-CH₃); 2.82 (d, J=20.0 Hz, 2H, piperidine-CH₂NHCH₂); 3.02-3.64 (m, 3H, piperidine); 3.94 (s, 2H, piperidine-CH₂NHCH₂); 4.08-4.71 (m, 1H, piperidine); 6.49 (d, J=1.1 Hz, 1H, furane H4); 7.23 (d, J=7.9 Hz, 1H, pyridine H3); 7.49 (dd, J=7.9 Hz, 1.8 Hz, 1H, pyridine H4); 7.68 (d, J=1.1 Hz, 1 H, furane H2); 8.38-8.43 (m, 1H, pyridine H6). ¹³C NMR (CDCl₃, 360 MHz) δ (ppm): 18.1; 32.8; 33.5; 38.0; 43.0; 55.1; 56.8; 57.0; 93.8; 95.0;111.5; 121.7; 123.3; 131.5; 137.2;
144.4; 149.7; 156.4; 162.3.

### Example E35:

### Benzo[b]furan-2-yl-[4-fluoro-4-[[(5-methylpyridin-2-ylmothyl)amino]methyl]piperidin-1-yl]-methanone

Synthesis worked according to the preparation of E1 when using benzo[b]furan-2-carboxylic acid (C35).
Yield: 30.1 mg (49 %) light yellow oil.
MS (APCI): m/z 382 (M+1)⁺. IR (NaCl) v (cm⁻¹): 2923; 2850; 1331; 1565; 1434; 1373: 1288; 1253; 1099; 971; 786; 740. ¹H NMR (CDCl₃, 360 MHz) δ (ppm): 1.69-1.90 (m, 2H, piperidine); 2.08-2.19 (m, 2H, piperidine); 2.36 (s, 3H, pyridine-CH₃); 2.84 (d, J=20.2 Hz, 2H, piperidine-CH₂NHCH₂); 3.04-3.72 (m, 2H, pipeddine); 3.95 (s, 2H, piperidine-CH₂NHCH₂); 4.15-4.77 (m, 2H, piperidine); 7.24 (d, J=7.9 Hz, 1H, pyridine H3); 7.30-7.36 (m, 2H, benzofuran H3, H5); 7.43 (ddd, J=8.3 Hz, 7.2 Hz, 1.2 Hz, 1 H, benzofuran H6); 7.49 (dd, J=7.8 Hz, 1.7 Hz, 1 H, pyridine H4); 7.56 (ddd, J=7.3 Hz, 1.6 Hz, 0.8 Hz, 1H, benzofuran H7); 7.68 (ddd, J=7.7 Hz, 1.3 Hz, 0.6 Hz, 1H, benzofuran H4); 8.39-8.43 (m, 1 H, pyridine H6).
HR-MS: C₂₂H₂₄FN₃O2; calculated: 381.1853; found: 381.1852.

### Example E36:

### Benzo[b]furan-3-yl-[fluoro-4-[[(5-methylpyridin-2-ylmethyl)amino)methyl]piperidin-1-yl]-methanone

Synthesis worked according to the preparation of E1 when using benzo[b]furan-3-carboxylic acid (C36).
Yield: 15.5 mg (71 %) light yellow oil.
MS (APCI): m/z 382 (M+1)⁺. IR (NaCl) v (cm⁻¹): 3332; 2923; 1631; 1565; 1446; 1103; 752. ¹H NMR (CDCl₃, 600 MHz) δ (ppm): 1.52-1.89 (m, 2H, piperidine); 1.97-2.17 (m, 2H, piperidine); 2.34 (s, 3H, pyridine-CH₃); 2.81 (d, J=20.6 Hz, 2H, piperidine-CH₂NHCH₂); 3.23-3.57 (m, 2H, piperidine); 3.92 (s, 2H, piperidine-CH₂NHCH₂); 4.08-4.93 (m, 2H, piperidine); 7.21 (d, J=7.9 Hz, 1H, pyridine H3); 7.33 (ddd, J=7.4 Hz, 7.6 Hz, 1.0 Hz, 1H, benzothiophene H5); 7.37 (ddd, J=8.1 Hz, 7.2 Hz, 1.0 Hz, 1H, benzothiophene H6); 7.47 (dd, J=7.9 Hz, 1.5 Hz, 1 H, pyridine H4); 7.55 (d, J=8.3 Hz, 1H, benzothiophene H7); 7.65 (d, J=7.6 Hz, 1H, benzothiophene H4); 7.88 (s, 1H, benzothiophene H2); 8.38-8.41 (m, 1H, pyridine H6).
HR-MS: C₂₂H₂₄FN₃O₂; calculated: 381.1853; found: 381.1853.

### Example E37:

### [4-Fluoro-4-[[(5-methylpyridin-2-ylmethyl)amino]methyl]piperidin-1-yl]-pyrrol-2-yl-methanone

Synthesis worked according to the preparation of E1 when using pyrrol-2-carboxylic acid (C37).
Yield: 5.8 mg (31 %) light yellow oil.
MS (APCI): m/z 331 (M+1)⁺. IR (NaCl) v (cm⁻¹): 3733; 3216; 2923; 1670; 1600; 1442; 1292; 1922; 744. ¹H NMR (CDCl₃, 360 MHz) δ (ppm): 1.50-1.84 (m, 2H, piperidine); 2.00-2.19 (m, 2H, piperidine); 2.36 (s, 3H, pyridine-CH₃); 2.83 (d, J=20.4 Hz, 2H, piperidine-CH₂NHCH₂); 3.23-3.58 (m, 2H, piperidine); 3.95 (s, 2H, piperidine-CH₂NHCH₂); 4.23-4.52 (m, 2H, piperidine); 6.28 (ddd, J=3.6 Hz, 2.7 Hz, 3.7 Hz, 1H, pyrrole H4); 6.56 (ddd, J=3.7 Hz, 2.4 Hz, 1.3 Hz, 1H, pyrrole H3); 6.95 (ddd, J=2.6 Hz, 2.6 Hz, 1.2 Hz, 1H, pyrrole H5); 7.24 (d, J=7.9 Hz, 1H, pyridine H3); 7.49 (dd, J=8.0 Hz, 2.0 Hz, 1H, pyridine H4); 8.39-8.45 (m, 1 H, pyridine H6); 9.38 (bs, 1 H, pyrrole NH).

### Example E38:

### [4-Fluoro-4-[[(5-methylpyridin-2-ylmethyl)aminolmethyl]piperidin-1-yl)-pyrazol-3-yl-methanone

Synthesis worked according to the preparation of E1 when using pyrazol-3-carboxylic acid (C38).
Yield: 8.1mg (43%).
MS (APCI): m/z 332 (M+1)⁺. IR (NaCl) v (cm⁻¹): 3736; 3124; 2927; 1616; 1492; 1454; 1292; 1218; 1130; 1045; 914; 771; 732. ¹H NMR (CDCl₃, 360 MHz) δ (ppm): 1.65-1.87 (m, 2H, piperidine); 2.05-2.16 (m, 2H, piperidine); 2.36 (s, 3H, pyridine-CH₃); 2.86 (d, J=20.2 Hz, 2H, piperidine-CH₂NHCH₂); 3.17-3.61 (m, 2H, piperidine); 3.98 (s, 2H, piperidine-CH₂NHCH₂); 4.48-4.63 (m, 2H, piperidine); 6.67 (d, J=2.3 Hz, 1H, pyrazole H4); 7.24 (d, J=8.0 Hz, 1H, pyridine H3); 7.50 (dd, J=8.0 Hz, 1.7 Hz, 1H, pyridine H4); 7.61 (d, J=2.2 Hz, 1H, pyrazole H5); 8.39-8.44 (m, 1H, pyridine H6); 11.27 (bs, 1H, pyrazole NH).

### Example E41:

### [4-Fluoro-4-[[(5-methylpyridin-2-ylmethyl)amino]methyl]piperidin-1-yl]-indol-2-yl-methanone

Synthesis worked according to the preparation of E1 when using indol-2-carboxylic acid (C41 ).
Yield: 3.4 mg (6.1 %) light yellow oil.
MS (APCI): m/z 389 (M+1)⁺. ¹H NMR (CDCl₃, 360 MHz) δ (ppm): 1.72-1.84 (m, 2H, piperidine); 2.09-2.17 (m, 2H, piperidine); 2.34 (s, 3H, pyridine-CH₃); 2.82 (d, J=20.2 Hz,2H, pipeddine-CH₂NHCH₂); 3.27-3.67 (m, 2H, piperidine); 3.93 (s, 2H, piperidine-CH₂NHCH₂); 4.53-4.60 (m, 2H, piperidine); 6.79 (dd, J=2.3 Hz, 0.9 Hz, 1H, indole H3); 7.16 (ddd, J=8.1 Hz, 6.9 Hz. 1.0 Hz, 1 H, indole H5); 7.23 (d, J=7.9 Hz, 1H, pyridine H3); 7.30 (ddd, J=8.3 Hz, 6.9 Hz, 1.0 Hz; 1H, indole H6); 7.44 (ddd, J=8.3 Hz, 1.0 Hz; 1H, indole H7); 7.47 (dd, J=7.7 Hz, 1.7 Hz, 1H, pyridine H4); 7.66 (dd, J=8.0 Hz, 1.0 Hz, 1H, indole H4); 8.39-8.52 (m, 1H, pyridine H6); 9.16 (bs, 1 H, indole NH).

### Example E42:

### [4-Fluoro-4-[[(5-mothylpyridin-2-ylmethyl)aminolmothyl]piperldin-1-yl)-pyrazolo[1,5-a]pyridin-2-yl-methanone

Synthesis worked according to the preparation of E1 when using pyrazolo[1,5-a]pyridine-2-carboxylic acid (C42).
Yield: 47.9 mg (79 %) light yellow oil.
MS (APCI): m/z 382 (M+1)⁺. IR (NaCl) v (cm⁻¹): 3498; 2923; 1631; 1504; 1241; 1103; 883; 763. ¹H NMR (CDCl₃, 360 MHz) δ (ppm): 1.69-1.90 (m, 2H, piperidine); 2.00-2.20 (m, 2H, piperidine); 2.35 (s, 3H, pyridine-CH₃); 2.83 (d, J=20.6 Hz, 2H, piperidine-CH₂NHCH₂); 3.17-3.31 (m, 1 H, piperidine); 3.47-3.60 (m, 1H, piperidine); 3.95 (s, 2H, piperidine-CH₂NHCH₂); 4.45-4.70 (m, 2H, piperidine); 6.80-6.90 (m, 2H, pyrazolopyridine H3, H6); 7.17 (ddd, J=8.9 Hz, 6.8 Hz, 1.1 Hz, 1 H, pyrazolopyridine H5); 7.24 (d, J=7.9 Hz, 1H, pyridine H3); 7.49 (dd, J=7.8 Hz, 1.7 Hz, 1 H, pyridine H4); 7.59 (ddd, J=8.9 Hz, 1.2 Hz, 1.2 Hz, 1 H, pyrazolopyridine H4); 8.40-8.42 (m, 1H, pyridine H6); 8.44 (ddd, J=7.0 Hz, 2.0 Hz, 0.9 Hz, 1H, pyrazolopyridine H7).
HR-MS: C₂₁H₂₄FN₅O; calculated: 381.1965; found: 381.1965.

### Example E43:

### [4-Fluoro-4-[[(5-methylpyridin-2-ylmethyl)amino]methyl]piperidin-1-yl]-pyrazolo[1,5-a]pyridin-3-yl-methanone

Synthesis worked according to the preparation of E1 when using pyrazolo[1,5-a]pyridine-3-carboxylic acid (C43).
Yield: 21.5 mg (98 %) lighte yellow oil.
MS (APCI): m/z 382 (M+1)⁺. ¹H NMR (CDCl₃, 360 MHz) δ (ppm): 1.65-1.83 (m, 2H, piperidine); 2.05-2.16 (m, 2H, piperidine); 2.35 (s, 3H, pyridine-CH₃); 2.85 (d, J=20.2 Hz, 2H, piperidine-CH₂NHCH₂); 3.35-3.51 (m, 2H, piperidine); 3.96 (s, 2H, piperidine-CH₂NHCH₂); 4.27-4.43 (m, 2H, piperidine); 6.93 (ddd, J=6.9 Hz, 6.9 Hz, 1.3 Hz, 1H, pyrazolopyridine H6); 7.24 (d, J=7.7 Hz, 1H, pyridine H3); 7.34 (ddd, J=8.7 Hz, 7.0 Hz, 1.2 Hz, 1H, pyrazolopyridine H5); 7.49 (dd, J=7.8 Hz, 1.7 Hz, 1H, pyridine H4); 7.98 (ddd, J=8.8 Hz, 1.0 Hz, 1.0 Hz, 1 H, pyrazolopyridine H4); 8.08 (s, 1 H, pyrazolopyridine H2); 8.39-8.43 (m, 1H, pyridine H6); 8.51 (ddd, J=7.0 Hz, 1.0 Hz, 1.0 Hz, 1H, pyrazolopyridine H7). ¹³C NMR (CDCl₃, 360 MHz) δ (ppm): 18.1; 33.4; 33.5; 40.2; 55.0; 56.9; 57.0; 94.0; 95.2; 106.0; 113.3; 119.1; 121.7; 125.9; 128.8; 131.5; 137.1; 141.7; 149.7; 156.3; 164.1. HR-MS: C₂₁H₂₄FN₅O; calculated: 381.1965; found: 381.1964.

### 5. Synthesis of exemplary compounds

### 5a) Acid derivatives according to formula C

Derivatives according to formula C as precursors for exemplary embodiments are available from:
*5-Bromobenzo[b]thiophene-3-carboxylic acid* (C44)
   from *Maybridge, Tintagel, Comwall (UK)* [Order number: CC 33501];
*5-Chlorobenzo[b]thiophene-3-carboxylic acid* (C45)
   from *Maybridgs, Tintagel, Cornwall (UK)* [Order number: CC 00115]
   or from *Rare Chemicals, Kiel (Germany)* [Order number: AL BO 0996];
*5-Ethoxy-2-methylbenzo[b]furan-3-carboxylic acid* (C46)
   from Akos, *Steinen (Germany)* [Order number: STT-00013526]
   or from *Matrix Scientific, Columbia, SC* (USA) [Order number: 18524];
*5-Methoxybenzo[b]furan-3-carboxylic acid* (C47)
   from Akos, *Steinen (Germany)* [Order number: STT-00137631]
*5-Phonylsulfonyloxybenzo[b]thiophene-3-carboxylic acid* (C48)
   from *Sunshine Chemlab, Monmouth Junction, NJ (USA)* [Order number: SC-T09-0005];
*Indole-3-carboxylic acid* (C49)
from *Aldrich, Karlsruhe (Germany)* [Order number: 284734];
*4-Bromoindoie-3-carboxylic acid* (C50)
   from *Maybridge, Tintagel, Comwall (UK)* [Order number: MO 08275];
*5-Chloroindole-3-carboxylic acid* (C51)
   from *Rare Chemicals, Kiel (Germany)* [Order number: AL BE 0763];
*5-Bromoindole-3-carboxylic acid* (C52)
   from *Rare Chemicals, Kiel (Germany)* [Order number: AL BE 0374];
*5-Methylindole-3-carboxylic acid* (C53)
   from *Rare Chemicals, Kiel (Germany)* [Order number: AL BE 0707];
*5-Methoxylindole-3-carboxylic acid* (C54)
   from *Rare Chemicals, Kiel (Germany)* [Order number: AL BE 0096]
   or from *ABCR, Karlsruhe (Germany)* [Order number: AB169216];
*6-Fluoroindole-3-carboxylic acid* (C55)
   from *Rare Chemicals, Kiel (Germany)* [Order number: AL BE 0955];
*7-Bromoindole-3-carboxylic acid* (C56)
   from *ABCR, Karlsruhe (Germany)* [Order number: AB169202];
*7-Methylindole-3-carboxylic acid* (C57)
   from *Rare Chemicals, Kiel (Germany)* [Order number: AL BE 1499];
*7-Azaindole-3-carboxylic acid* (C58)
   from *Sinova, Bethesda, MD (USA)* [Order number: SL-02000]
   or from *3B Scientific, Libertyville, IL (USA)* [Order number: 3B3-080420];
*Indolizine-1-carboxylic acid* (C59)
   can be prepared starting from indolizine-1-carboxylic acid methyl ester synthesized according to literature (Zhang, L. et al., *Synthesis* 2000, 1733) and subsequent hydrolysis with 2n NaOH;
*Indolizine-2-carboxylic acid* (C60)
   from *Sinova, Bethesda, MD (USA)* [Order number: SL-02000]
   or from *Anichem, North Brunswick, NJ (USA)* [Order number: H13108];
*Imidazole-2-carboxylic acid* (C61)
   from *Maybridge, Tintangel, Cornwall (UK)* [Order number: CC 08901]
   or from *ABCR, Karlsruhe (Germany)* [Order number: AB172745];
*4-Methyl-1H-imidazole-2-carboxylic acid* (C62)
   from *Greenchem Institute, Haverford, PA (USA)* [Order number: IM-1051];
*Imidazole-4-carboxylic acid* (C63)
   from *Aldrich, Karlsruhe (Germany)* [Order number: 425842];
*6-Chloroimidazo[1,2-a]pyridin-2-carboxylic acid* (C64)
   from *Ambinter, Paris (France)* [Order number: BB_SC-4679];
*6-Chloro-2-methylimidazo[1,2-a]pyridin-3-carboxylic acid* (C65)
   from *Butt Park, Camelford (UK)* [Order number: 13\\05-83];
*Oxazole-2-carboxylic acid* (C66)
   from *J & W PharmLab, Morrisville, PA (USA)* [Order number: 56-0014];
*4-Bromooxazole-2-carboxylic acid* (C67)
   from *Anichem, North Brunswick, NJ (USA)* [Order number: H12359];
*Oxazole-5-carboxylic acid* (C68)
   from *Chemstep, Carbon Blanc (France)* [Order number: 1945]
   or from *J & W PharmLab, Morrisville, PA (USA)* [Order number: 56-0014];
*4-Methyloxazo*/*e-5-carboxylic acid* (C69)
   from *Aldrich, Karlsruhe (Germany)* [Order number: 425842];
*Thiazole-2-carboxylic acid* (C70)
   from *Ark Pharm, Libertyville, IL (USA)* [Order number: AK-21895]
   or from *Rare Chemicals, Kiel (Germany)* [Order number: AL BE 0750];
*4-Methylthiazole-2-carboxylic acid* (C71)
   from *Anichem, North Brunswick, NJ (USA)* [Order number: H12350];
*4-Isopropylthiazole-2-carboxylic acid* (C72)
   from *Anichem, North Brunswick, NJ (USA)* [Order number: H12387];
*4-Trifluoromethylthiazole-2-carboxylic acid* (C73)
   from *Anichem, North Brunswick, NJ (USA)* [Order number: H12389];
*Thiazole-5-carboxylic acid* (C74)
   from *ABCR, Karlsruhe (Germany)* [Order number: AB169620]
   orfrom *Rare Chemicals, Kiel (Germany)* [Order number: AL BE 1261];
*2-Bromo-4-methylthiazole-5-carboxylic acid* (C75)
   from *Aldrich, Karlsruhe (Germany)* [Order number: H12389];
*5-Cyanoisothiazole-3-carboxylic acid* (C76)
   from *Chemstep, Carbon Blanc (France)* [Order number: 24595];
*1,2-Benzisothiazole-3-carboxy*/*ic acid* (C77)
   from *Aurora, Graz (Austria)* [Order number: kcd-556319];
*Isoxazole-5-carboxylic acid* (C40)
   from *Acros, Geel (Belgium)* [Order number: 21169-71-1];
*1,2-Benzisoxazole-3-carboxylic acid* (C78)
   from *Chemstep, Carbon Blanc (France)* [Order number 69627];
*1,2,5-Thiadiazo*/*e-3-carboxylic acid* (C79)
   from *J* & *W PharmLab, Morrisville, PA (USA)* [Order number: 90-0084]; *4-Methyl-1,2,5-Thiadiazole-3-carboxylic acid* (C80)
   from *Chemstep, Carbon Blanc (France)* [Order number: 1958];
*1,2,5-Oxadiazole-3-carboxylic acid* (C81)
   from *Maybridge, Tintagel, Cornwall (UK)* [Order number CC 07901];
*4-Methylfurazan-3-carboxylic acid* (C82)
   from *ChemBridge, San Diego, CA (USA)* [Order number: 4401891];
*1,3,4-Oxadiazole-2-carboxylic acid* (C83)
   from *Anichem, North Brunswick, NJ (USA)* [Order number: H10685];
*5-Methyl-1,3,4-oxadiazole-2-carboxylic acid* (C84)
   from *Anichem, North Brunswick, NJ (USA)* [Order number: A69224];
*5-Trifluoromethyl-1,3,4-oxadiazole-2-carboxylic acid* (C85)
   from *Anichem, North Brunswick, NJ (USA)* [Order number: H10688];
*5-Methyl-1,3,4-thiadiazole-2-carboxylic acid* (C86)
   from *Chemstep, Carbon Blanc (France)* [Order number: 1898];
*3H-1,2,3-Triazole-4-carboxylic acid* (C87)
   from *Pfaltz & Bauer, Waterbury, CT (USA)* [Order number: T17880]
   or from *Matrix Scientific, Columbia, SC (USA)* [Order number: 19672];
*5-Methyl-2H-1,2,3-triazole-4-carboxylic* acid (C88)
   from *Ryan Scientific, Mt. Pleasant, SC (USA)* [Order number: B017733];
*4H-1,2,4-triazole-3-carboxylic acid* (C39)
   from *Aldrich, Kerlsruhe (Germany)* [Order number: 668472];

### 5b) Synthesis of embodiments according to formula I

### Example E44:

**5-Bromobenzoibithien-3-yl-[4-fluoro-4-[[(5-methylpyridin-2-ylmethyl)amino]methyl]piperidin-1-yl]-methanone**

Synthesis works according to the preparation of E1 when using 5-bromobenzo[b]thiophene-3-carboxylic acid (C44).

### Example E45:

**5-Chlorobenzo[b]thien-3-yl-[4-fluoro-4-[[(5-methylpyridin-2-ylmethyl)amino]mothyl]piperidin-1-yl]-methanone**

Synthesis works according to the preparation of E1 when using 5-chlorobenzo[b]thiophene-3-carboxylic acid (C45).

### Example E46:

**5-Ethoxy-2-mothylbenzo[b]furan-3-yl-[4-fluoro-4-[[(5-methylpyridin-2-ylmethyl)amino]methyl]piperidin-1-yl]-methanone**

Synthesis works according to the preparation of E1 when using 5-ethoxy-2-methylbenzo[b]furan-3-carboxylic acid (C46).

### Example E47:

**[4-Fluoro-4-[[(5-methylpyridin-2-ylmethyl)amino]methyl]piperidin-1-yl]-5-mothoxybenzo[b]furan-3-yl-methanone**
Synthesis works according to the preparation of E1 when using 5-methoxybenzo[b]furan-3-carboxylic acid (C47).

### Example E48:

**[4-Fluoro-4-[[(5-methylpyridin-2-ylmethyl)amino]methyl]piperidin-1-yl]-5-phenylsulfonyloxybenzo[b]thiophene-3-yl-methanone**

Synthesis works according to the preparation of E1 when using 5-phenylsulfonyloxybenzo[b]thiophene-3-carboxylic acid (C48).

### Example E49:

**[4-Fluoro-4-[[(5-methylpyridin-2-ylmethyl)amino]methyl]piperidin-1-yl]-indol-3-yl-methanone**

Synthesis works according to the preparation of E1 when using indole-3-carboxylic acid (C49).

### Example E50:

**4-Bromoindol-3-yl-[4-fluoro-4-[[(5-methylpyridin-2-ylmethyl)amino]methyl]piperidin-7-yl]-methanone**

Synthesis works according to the preparation of E1 when using 4-bromoindole-3-carboxylic acid (C50).

### Example E51:

**5-Chloroindol-3-yl-[4-fluoro-4-[[(5-methylpyridin-2-ylmethyl)amino]methyl]piperidin-1-yl]-methanone**

Synthesis works according to the preparation of E1 when using 5-chloroindole-3-carboxylic acid (C51).

### Example E52:

**5-Bromoindol-3-yl-[4-fluoro-4-[[(5-methylpyridin-2-ylmethyl)amino]methyl]piperidin-1-yl]-methanone**

Synthesis works according to the preparation of E1 when using 5-bromoindole-3-carboxylic acid (C52).

### Example E53:

**[4-Fluoro-4-[[(5-methylpyridin-2-ylmethyl)amino]methyl]piperidin-1-yl]-5-methylindol-3-yl-methanone**
Synthesis works according to the preparation of E1 when using 5-methylindole-3-carboxylic acid (C50).

### Example E54:

**[4-Fluoro-4-[[(5-methylpyridin-2-ylmethyl)amino]methyl]piperidin-1-yl]-5-methoxylindol-3-yl-methanone**

Synthesis works according to the preparation of E1 when using 5-methoxylindole-3-carboxylic acid (C50).

### Example E55:

**6-Fluoroindol-3-yl-[4-fluoro-4-[[(5-methylpyridin-2-ylmethyl)amino]methyl]piperidin-1-yl]-methanone**

Synthesis works according to the preparation of E1 when using 6-fluoroindole-3-carboxylic acid (C55).

### Example E56:

**7-Bromoindol-3-yl-[4-fluoro-4-[[(5-methylpyridin-2-ylmethyl)amino]methyl]piperidin-1-yl]-methanone**
Synthesis works according to the preparation of E1 when using 7-bromoindole-3-carboxylic acid (C56).

### Example E57:

**[4-Fluoro4-[[(5-methylpyridin-2-ylmethyl)amino]methyl]piperidin-1-yl]-7-methylindol-3-yl-methanone**

Synthesis works according to the preparation of E1 when using 7-methylindole-3-carboxylic acid (C57).

### Example E58:

**7-Azaindol-3-yl-[4-fluoro-4-[[(5-methylpyridin-2-ylmethyl)amino]methyl]piperidin-1-yl]-methanone**
Synthesis works according to the preparation of E1 when using 7-azaindole-3-carboxylic acid (C58).

### Example E59:

**[4-Fluoro-4-[[(5-methylpyridin-2-ylmethyl)amino]methyl]piperidin-1-yl]-indolizin-1-yl-methanone**
Synthesis works according to the preparation of E1 when using indolizine-1-carboxylic acid (C59).

### Example E60:

**[4-Fluoro-4-[[(5-methylpyridin-2-ylmethyl)amino]methyl]piperidin-1-yl]-indolizin-2-yl-methanone**

Synthesis works according to the preparation of E1 when using indolizine-2-carboxylic acid (C60).

### Example E61:

**[4-Fluoro-4-[[(5-methylpyridin-2-ylmethyl)amino]methyl]piperidin-1-yl]-imidazol-2-yl-methanone**

Synthesis works according to the preparation of E1 when using imidazol-2-carboxylic acid (C61).

### Example E62:

**[4-Fluoro-4-[[(5-methylpyridin-2-ylmethyl)amino]methyl]piperidin-1-yl]-4-methyl-1H-**imidazol-2-yl-methanone

Synthesis works according to the preparation of E1 when using 4-methyl-1 H-imidazole-2-carboxylic acid (C62).

### Example E63:

**[4-Fluoro-4-[[(5-mothylpyridin-2-ylmethyl)amino]methyl]piperidin-1-yl]-imidazol-4-yl-methanone**

Synthesis works according to the preparation of E1 when using imidazol-4-carboxylic acid (C63).

### Example E64:

**6-Chloroimidazo[1,2-a]pyridin-2-yl-[4-fluoro-4-[[(5-methylpyridin-2-ylmethyl)amino]methyl]piperldin-1-yl]-methanone**

Synthesis works according to the preparation of E1 when using 6-chloroimidazo[1,2-a]pyridin-2-carboxylic acid (C64).

### Example E65:

**6-Chloro-2-methylimidazo[1,2-a]pyridin-3-yl-[4-fluoro-4-[[(5-methylpyridin-2-ylmethyl)amino]methyl]piperidin-1-yl]-methanone**
Synthesis works according to the preparation of E1 when using 6-chloro-2-methylimidazo[1,2-a]pyridin-3-carboxylic acid (C65).

### Example E66:

**[4-Fluoro-4-[[(5-methylpyridin-2-ylmethyl)amino]methyl]piperidin-1-yl]-oxazol-2-yl-methanone**
Synthesis works according to the preparation of E1 when using oxazole-2-carboxylic acid (C66).

### Example E67:

**4-Bromooxazol-2-yl-[4-fluoro-4-[[(5-methylpyridin-2-ylmethyl)amino]methyl]piperidin-1-yl]-methanone**
Synthesis works according to the preparation of E1 when using 4-bromooxazole-2-carboxylic acid (C67).

### Example E68:

**[4-Fluoro-4-[[(5-methylpyridin-2-ylmethyl)amino]methyl]piperidin-1-yl]-oxazol-5-yl-methanone**

Synthesis works according to the preparation of E1 when using oxazole-5-carboxylic acid (68).

### Example E69:

**[4-Fluoro-4-[[(5-methylpyridin-2-ylmethyl)amino]methyl]piperidin-1-yl]-4-methyloxazol-5-yl-methanone**

Synthesis works according to the preparation of E1 when using 4-methyloxazole-5-carboxylic acid (C69).

### Example E70:

**[4-Fluoro-4-[[(5-methylpyridin-2-ylmethyl)amino]methyl]piperidin-1-yl]-thiazol-2-yl-methanone**
Synthesis works according to the preparation of E1 when using thiazole-2-carboxylic acid (C70).

### Example E71:

**[4-Fluoro-4-[[(5-methylpyridin-2-ylmethyl)amino]methyl]piperidin-1-yl]-4-methylthiazol-2-yl-methanone**

Synthesis works according to the preparation of E1 when using 4-methylthiazole-2-carboxylic acid (C71).

### Example E72:

**[4-Fluoro-4-[[(5-methylpyridin-2-ylmethyl)amino]methyl]piperidin-1-yl]-4-isopropylthiazol-2-yl-methanone**

Synthesis works according to the preparation of E1 when using 4-isopropyllthiazole-2-carboxylic acid (C72).

### Example E73:

**[4-Fluoro-4-[[(5-methylpyridin-2-ylmethyl)amino]methyl]piperidin-1-yl]-4-trifluoromethylthiazol-2-yl-methanone**
Synthesis works according to the preparation of E1 when using 4-trifluoromethylthiazole-2-carboxylic acid (C73).

### Example E74:

**[4-Fluoro-4-[[(5-methylpyridin-2-ylmethyl)amino]methyl]piporidin-1-yl]-thiazol-5-yl-methanone**
Synthesis works according to the preparation of E1 when using thiazole-5-carboxylic acid (C74).

### Example E75:

**2-Bromo-4-methylthiazol-5-yl-[4-fluoro-4-[[(5-methylpyridin-2-ylmethyl)amino]methyl]piperidin-1-yl]-methanone**
Synthesis works according to the preparation of E1 when using 2-bromo-4-methylthiazole-5-carboxylic acid (C75).

### Example E76:

**5-cyanoisothiazol-3-yl-[4-fluoro-4-[[(5-methylpyridin-2-ylmethyl)amino]methyl]piperidin-1-yl]-methanone**
Synthesis works according to the preparation of E1 when using 5-cyanoisothiazole-3-carboxylic acid (C76).

### Example E77:

**1,2-Benzisothiazol-3-yl-[4-fluoro4-[[(5-methylpyridin-2-ylmethyl)amino]methyl]piperidin-1-yl]-methanone**
Synthesis works according to the preparation of E1 when using 1,2-benzisothiazole-3-carboxylic acid (C77).

### Example E40:

**[4-Fluoro-4-[[(5-methylpyridin-2-ylmethyl)amino]methyl]piperidin-1-yl]-isoxazol-5-yl-methanone**

Synthesis works according to the preparation of E1 when using isoxazole-5-carboxylic acid (C40).

### Example E78:

**1,2-Benzisoxazol-3-yl-[4-fluoro-4-[[(5-methylpyridin-2-ylmethyl)amino]methyl]piperidin-1-yl]-methanone**

Synthesis works according to the preparation of E1 when using 1,2-benzisoxazole-3-carboxylic acid (C78).

### Example E79:

**[4-Fluoro-4-[[(5-methylpyridin-2-ylmethyl)amino]methyl]piperidin-1-yl]-1,2,5-**thiadiazol-3-yl-methanone

Synthesis works according to the preparation of E1 when using 1,2,5-thiadiazole-3-carboxylic acid (C79).

### Example E80:

**[4-Fluoro-4-[[(5-methylpyridin-2-ylmethyl)amino]methyl]piperidin-1-yl]-4-methyl-1,2,5-thiadiazol-3-yl-methanone**

Synthesis works according to the preparation of E1 when using 4-methyl-1,2,5-thiadiazole-3-carboxylic acid (C80).

### Example E81:

**[4-Fluoro-4-[[(5-methylpyridin-2-ylmethyl)amino]methyl]piperidin-1-yl)-1,2,5-oxadiazol-3-yl-methanone**

Synthesis works according to the preparation of E1 when using 1,2,5-oxadiazole-3-carboxylic acid (C81).

### Example E82:

**[4-Fluoro-4-([(5-methylpyridin-2-ylmethyl)amino]methyl]piperidin-1-yl]4-methylfurazan-3-yl-methanone**

Synthesis works according to the preparation of E1 when using 4-methylfurazan-3-carboxylic acid (C82).

### Example E83:

**[4-Fluoro-4-[[(5-methylpyridin-2-ylmethyl)amino]methyl]piperidin-1-yl]-1,3,4-oxadiazol-2-yl-methanone**

Synthesis works according to the preparation of E1 when using 1,3,4-oxadiazole-2-carboxylic acid (C83).

### Example E84:

**[4-Fluoro-4-[[(5-methylpyridin-2-ylmethyl)amino]methyl]piperidin-1-yl]-5-methyl-1,3,4-oxadiazol-2-yl-methanone**

Synthesis works according to the preparation of E1 when using 5-methyl-1,3,4-oxadiazole-2-carboxylic acid (C84).

### Example E85:

**[4-Fluoro-4-[[(5-methylpyridin-2-ylmethyl)amino]methyl]piperidin-1-yl]-5-trifluoromethyl-1,3,4-oxadiazol-2-yl-methanone**

Synthesis works according to the preparation of E1 when using 5-trifluoromethyl-1,3,4-oxadiazole-2-carboxylic acid (C85).

### Example E86:

**[4-Fluoro4-[[(5-methylpyridin-2-ylmethyl)amino]methyl]piperidin-1-yl]-5-methyl-1,3,4-thiadiazol-2-yl-methanone**

Synthesis works according to the preparation of E1 when using 5-methyl-1,3,4-thiadiazole-2-carboxylic acid (C86).

### Example E87:

**[4-Fluoro4-[[(5-methylpyridin-2-ylmethyl)amino]methyl]piperidin-1-yl]-3H-1,2,3-triazol-4-yl-methanone**
Synthesis works according to the preparation of E1 when using 3H-1,2,3-triazole-4-carboxylic acid (C87).

### Example E88:

**[4-Fluoro-4-[[(5-methylpyridin-2-ylmethyl)amino]methyl]piperidin-1-yl]-5-methyl-2H-1,2,3-triazol-4-yl-methanone**

Synthesis works according to the preparation of E1 when using 5-methyl-2H-1,2,3-triazole-4-carboxylic acid (C88).

### Example E39:

**[4-Fluoro-4-[[(5-methylpyridin-2-ylmethyl)amino]methyl]piperidin-1-yl]-4H-1,2,4-triazol-3-yl-methanone**

Synthesis works according to the preparation of E1 when using 4H-1,2,4-triazole-3-carboxylic acid (C39).

### 6. Biological Activity

Biological activity of the compounds has been determined in radioligand binding assays. These experiments have been done according to methods which are described in literature (Hübner, H. et al., J. Med. Chem. (2000), 43, 7562). For the determination of binding affinity to the receptors of the dopamine D2 family membranes were established carrying the human D2long, D2short, D3 or D4.4 receptor subtype stably expressed in Chinese hamster ovary cell lines. In principle, the binding experiments started when incubating an appropriate amount of membrane with the radioligand [³H]spiperone and the particular test compounds used in a wide range of different concentrations from subnanomolar up to micromolar ones. In the same way, binding affinities of the test compounds to the dopamine D1 receptor were determined when using native membrane preparations from porcine striatum and the D1 selective radioligand [³H]SCH 23390.

The investigation of receptor binding to the serotonin and adrenergic receptors was done according to a published procedure (Heindl, C. et al., Tetrahedron: Asymmetry (2003), 14, 3141-3152). In detail, for the determination of the binding properties to serotonin receptors membrane preparations from porcine cortex was incubated with the 5-HT1A selective radioligand [³H]WAY 100635 or with the 5-HT2 selective compound [³H]ketanserin. The measurement of alpha-1 and alpha-2 receptor binding was achieved when utilizing porcine cortical membranes and the selective radioligands [³H]prazosin (for alpha-1) and [³H]RX821002 in an assay similar to that described above.

The results of the binding experiments are summarized in Table 1 as mean values of two to five individual experiments each done in triplicate and expressed as Ki values in nM.

**Table 1: Results of the binding assays with dopamine, serotonin and adrenergic receptors Ki-values given: A ≤50nM: B = 50-500 nM; C = 501-5000 nM; D > 5uM**

| cpd | R₁ | R₂ | R₃ | D1 | D2 | D3 | D4.4 | 5-HT1a | 5-HT2 | alpha1 |
|---|---|---|---|---|---|---|---|---|---|---|
| | 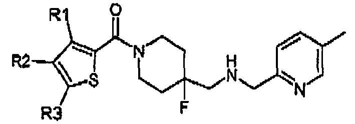 | | | | | | | | | |
| E1E15 | H | H | H | D | D | D | D | A | D | D |
| E2 | H | H | F | nd | nd | nd | nd | A | D | and |
| E3 | Cl | H | H | D | D | D | C | A | D | C |
| E4 | H | H | Cl | D | D | D | C | A | D | C |
| E5 | Br | H | H | D | D | D | C | A | D | C |
| E6 | H | Br | H | D | D | D | C | A | D | C |
| E7 | H | H | Br | D | D | D | C | A | D | C |
| E8 | H | Br | Br | nd | nd | nd | nd | A | D | nd |
| E9 | Me | H | H | D | D | D | D | A | D | D |
| E10 | H | Me | H | D | D | D | C | A | D | C |
| E11 | H | H | Me | D | D | D | C | A | D | D |
| E12 | H | H | MeS | D | D | D | C | A | D | C |
| E13 | H | H | Ph | D | C | C | D | B | C | C |
| E14 | H | H | 2-pyridyl | D | D | D | D | B | C | C |
| E15 | H | H | 2-thienyl | D | D | C | D | B | C | C |
| E16 | H | H | 2-methyl-3-thiazolyl | D | D | C | D | B | D | C |
| E17 | 1-pyrroly | H | H | D | D | D | C | B | D | C |
| | 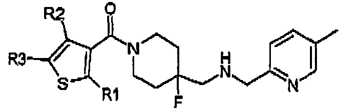 | | | | | | | | | |
| E18 | H | H | H | D | D | D | D | A | D | D |
| E19 | Br | H | H | D | D | D | C | A | D | C |
| E20 | H | OMe | H | D | D | D | C | B | D | D |
| E21 | H | OMe | Cl | D | D | D | C | A | D | C |
| E22 | H | OMe | Br | D | D | D | C | A | D | C |
| | 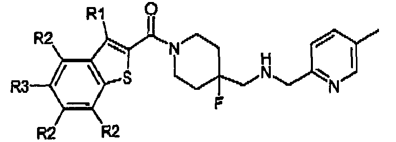 | | | | | | | | | |
| E23 | H | H | H | D | D | D | C | B | D | C |
| E24 | H | H | Br | D | D | C | C | B | D | B |
| | 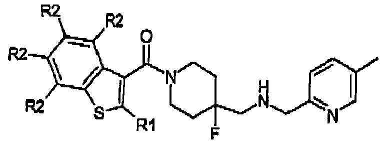 | | | | | | | | | |
| E25 | H | H | - | D | D | D | B | A | D | C |
| | 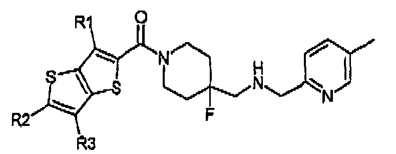 | | | | | | | | | |
| E26 | H | H | H | D | D | D | C | A | D | C |
| | 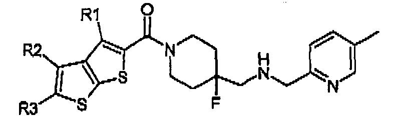 | | | | | | | | | |
| E27 | H | H | H | D | D | D | B | A | D | C |
| E28 | Cl | H | H | D | C | C | B | A | D | C |
| | 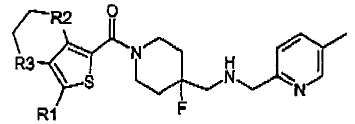 | | | | | | | | | |
| E29 | H | -CH₂- | -CH₂- | D | D | D | C | A | D | C |
| E30 | Br | -O- | -O- | D | D | C | C | A | D | C |
| | 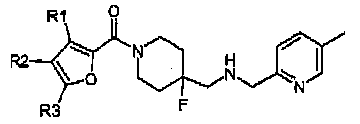 | | | | | | | | | |
| E31 | H | H | H | D | D | D | D | B | D | D |
| E32 | H | Br | Br | nd | nd | nd | nd | A | D | nd |
| | 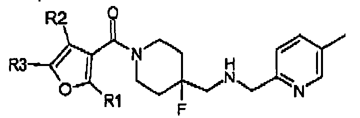 | | | | | | | | | |
| E33 | H | H | H | D | D | D | D | B | D | D |
| E34 | H | H | Br | D | D | D | C | A | D | C |
| | 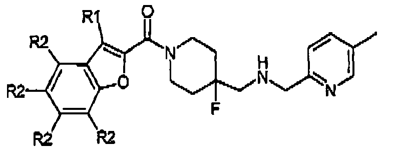 | | | | | | | | | |
| E35 | H | H | - | D | D | D | C | B | D | C |
| | 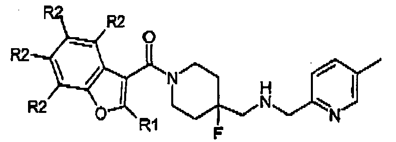 | | | | | | | | | |
| E36 | H | H | - | D | D | D | C | A | D | C |
| | 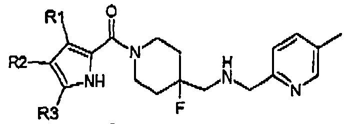 | | | | | | | | | |
| E37 | H | H | H | nd | nd | nd | nd | C | D | nd |
| | 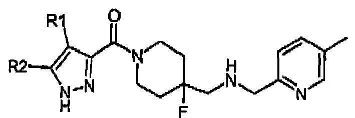 | | | | | | | | | |
| E38 | H | H | - | nd | nd | nd | nd | C | D | nd |
| | 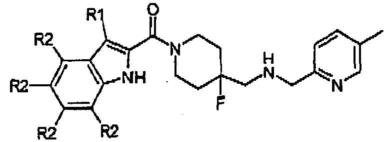 | | | | | | | | | |
| E41 | H | H | - | D | D | C | C | B | D | C |
| | 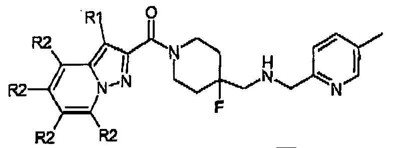 | | | | | | | | | |
| E42 | H | H | - | D | D | D | D | B | D | D |
| | 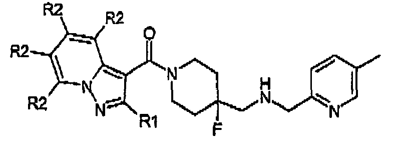 | | | | | | | | | |
| E43 | H | H | - | D | D | D | D | B | D | C |

nd = values were not determined

Moreover, most of the compounds of the present invention exhibit a remarkable affinity to the serotonin 5-HT1A receptor subtype, and a pronounced selectivity for the 5-HT1A receptor over the different dopamine receptor subtypes as well as over the 5-HT2 and the alpha-1 receptor.

The investigation of functional activity at the 5-HT1A receptor was carried out by a [³⁵S]GTPγS assay indicating the stimulation of receptor by irreversible binding of radioactive [³⁵S]GTPγS to the appropriate alpha subunit of the G-proteins which are located in the membrane (Schlotter, K. et al, J. Med. Chem. (2005), 48, 3696). In detail, membranes from CHO (Chinese hamster ovary) cells stably expressing the human 5-HT1A receptor were stimulated with an increasing concentration of test compounds used in a range of subnanomolar to micromolar concentrations in the presence of radioactive [³⁵S]GTPγ. The amount of bound radioactivity which indicates the amount of stimulation of the receptor was determined after transferring the membranes to filters by scintillation counting. Dose response correlations could be derived and the particular stimulation of the receptor was compared to the maximum effect of the reference compound and physiological ligand serotonin.

Table 2 shows the functional (intrinsic) activity of some compounds according to the present disclosure at the serotonin 5-HT1A receptor as the major target molecule. The intrinsic activity is given based on % activity of serotonin: A=Full antagonist (<15%); B=weak partial agonist (15-40%) C=strong partial agonist (40-75%) D=full agonist (>75%). The EC₅₀ values are presented as follows: A ≤50nM; B = 50-500 nM; C = 501-5000 nM; D > 5µM.

**Table 2: Intrinsic activities of the compounds of this invention at the serotonin 5-HT1A receptor determined in a [³⁵S]GTPγS assay**

| Intrinsic effect considering: A=full antagonist (<15%); B=weak partial agonist (15-40%) C=strong partial agonist (40-75%) D=full agonist (>75%). EC₅₀ values: A ≤50nM; B = 50- 500 nM; C = 501-5000 nM; D > 5µM | | | | | |
|---|---|---|---|---|---|
| compound | R1 | R2 | R3 | 5-HT1A stimulated | [³⁵S]GTP□S binding |
| | | | | intrinsic effect | EC₅₀ value |
| F13640 | - | - | - | D | A |
| | 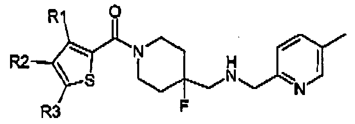 | | | | |
| E1E15 | H | H | H | D | A |
| E3 | Cl | H | H | D | A |
| E4 | H | H | Cl | D | A |
| E5 | Br | H | H | D | A |
| E6 | H | Br | H | D | A |
| E7 | H | H | Br | D | A |
| E9 | Me | H | H | D | A |
| E10 | H | Me | H | D | A |
| E11 | H | H | Me | D | A |
| | 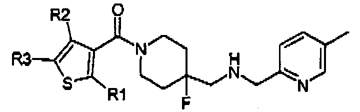 | | | | |
| E18 | H | H | H | D | A |

All the compounds of the present invention which were tested for intrinsic activity act as a full agonist.

## Claims

1. A compound having the general formula I 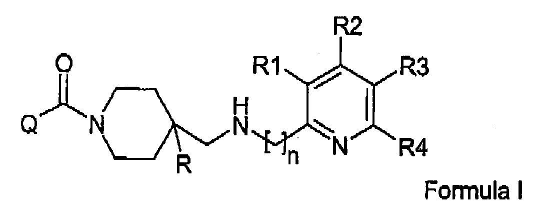 wherein:
n is 1,2 or 3;
R is hydrogen, fluoro, chloro, hydroxyl, cyano;
R1 and R2 are independently hydrogen, fluoro, chloro, methyl;
R3 is hydrogen, C1-C3 alkyl, fluoromethyl, cyano, hydroxyl, methoxyl, fluoro, chloro;
R4 is selected from hydrogen; (C1-C5)alkyl; fluoro(C1- C5)alkyl; cyclo(C3- C5)alkyl;
(C1-C5)alkoxyl; (C1-C5)alkylthio; (C3-C5)cycloalkyloxy; (C3-C5)cycloalkylthio; (C1-C5)alkoxyl, which may contain one or more fluoro substituents; (C1-C5)alkylthio, which may contain one or more fluoro substituents; a group -NRxRy, wherein Rx and Ry are independently hydrogen, (C1-C5)alkyl, (C5-C6)cycloalkyl or (C1-C3)alkyloxycarbonyl;, or wherein Rx and Ry are alkyles, which together with the nitrogen to which they are attached form a four to six membered saturated ring;
or a five membered heteroaryl group chosen from furanyl, pyrrolyl, thienyl, pyrazolyl, imidazolyl, oxazolyl, isoxazolyl, thiazolyl, isothiazolyl, triazolyl, oxadiazolyl, tetrazolyl;
Q represents a
five membered heteroaryl group, which is
(ii) substituted with one, two or three residues R5, R6 and R7; or
(iii) substituted with one residue R5, and annelated to a second five or six membered ring, which is aromatic or heteroarylic, wherein said second ring is substituted with one or two residues R8 and R9; or
(iv) substituted with one residue R5, and is annelated to a second five, six or seven membered ring, which is non-aromatic, may contain one or two heteroatoms, and is substituted with one or two residues R10 and R11;
wherein every substituent R5, R6, R7, R8, R9, R10 and R11 is bound to a ring forming carbon atom;
and wherein any heterarylic or non-aromatic ring which contains one or more ring forming nitrogens, may be substituted at one or more of said ring forming nitrogens with a residue R12.
R5, R6, R7, R8 and R9 are independently of each other selected from the group consisting of hydrogen, hydroxyl, formyl, oxime, cyano, nitro, NR13R14, carboxy, carbamoyl, (C1-C6)alkyl, (C3-C6)cycloalkyl, (C1-C6)alkyloxy, (C1-C6)alkylthio, (C2-C6)alkenyl, (C2-C6)alkynyl, phenyl, heteroaryl, phenoxy, halogen, trifluoromethyl, (C1-C6)alkylcarbonyl, (C1-C6)alkylaminocarbonyl, di(C1-C6)alkylaminocarbonyl, phenylcarbonyl, phenyl(C1-C6)alkyl, phenyl(C1-C6)alkyloxy, phenyl(C1-C6)alkylcarbonyl, phenyl(C1-C6)alkyloxycarbonyl, (C1-C6)alkyloxycarbonyl, (C1-C6)alkylsulfonyl, phenylsulfonyl, sulfamoyl, (C1-C6)alkylaminosulfonyl, di(C1-C6)alkylaminosulfonyl, phenylsulfonylamino, and (C1-C6)alkylsulfonylamino; wherein each alkyl, alkenyl, or alkynyl may be unsubstituted or substituted with one or more residues selected from among hydroxyl, (C1-C3)alkyloxy, halogen, and NR13R14; and wherein each heteroaryl is a monocyclic ring and wherein each phenyl or heteroaryl can be unsubstituted or substituted with one or more residues selected from among hydroxyl, (C1-C3)alkyloxy, halogen, (C1-C3)alkyl, (C3-C6)cycloalkyl, carboxy, NR13R14, cyano, trifluoromethyl and nitro;
R10 and R11 are independently selected from the group consisting of hydrogen, hydroxyl, (C1-C6)alkyl, phenyl, halogen, NR13R14 and oxo, wherein each alkyl may be unsubstituted or substituted with one or more residues selected from among hydroxyl, (C1-C3)alkyloxy, halogen, and NR13R14;
R12 is hydrogen, (C1-C6)alkyl, (C3-C6)cycloalkyl, phenyl, phenyl(C1-C3)alkyl, (C1-C5)alkylcarbonyl, phenylcarbonyl, phenyl(C1-C3)alkylcarbonyl and phenylsulfonyl, wherein each phenyl ring may be substituted with one or two substituents selected from among fluoro, chloro, methyl and methoxy;
R13 and R14 are independently selected from hydrogen, (C1-C5)alkyl, (C5-C6)cycloalkyl and (C1-C3)alkyloxycarbonyl or R13 and R14 are alkylenes, which together with the nitrogen to which they are attached form a four to six membered saturated ring;
and pharmaceutically acceptable salts and/or isomers or racemates thereof.

2. A compound according to claim 1 wherein R is fluoro.

3. A compound according to claim 1 wherein R1, R2 and R4 are all hydrogen.

4. A compound according to claim 1 wherein R3 is methyl or chloro.

5. A compound according to claim 1 wherein R is fluoro, R1 and R2 are both hydrogen, and R3 is methyl or chloro, and R4 is hydrogen or (C1-C3)alkyl.

6. A compound according to claim 1, having the general, formula Ib 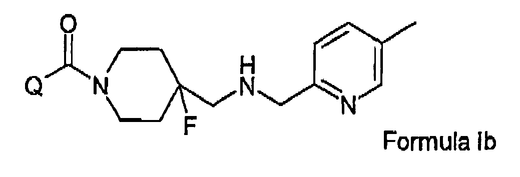 wherein Q is as defined in claim 1.

7. A compound according to anyone of the preceding claims, wherein Q is a five membered heteroaryl selected from among furanyl, pyrrolyl, thienyl, pyrazolyl, imidazolyl, oxazolyl, isoxazolyl, thiazolyl, isothiazolyl, triazolyl, oxadiazolyl, thiadiazolyl and tetrazolyl, which is substituted with one or two residues R5 and R6 which are independently selected from hydrogen, hydroxyl, formyl, cyano, oxime, (C1-C4)alkyl, (C1-C4)alkyloxy, (C1-C4)alkylthio, carboxy, (C1-C3)alkyloarbonyl, (C1-C3)alkyloxycarbonyl, chloro, bromo, iodo, hydroxymethyl, trifluoromethyl, (C1-C4)alkylaminomrbonyl, di(C1-C4)alkylaminocarbonyl, nitro, NR13R14; and wherein in the case of pyrrolyl, pyrazolyl, imidazolyl and triazolyl one nitrogen atom is substituted with R12 as defined in claim 1, and wherein R13 and R14 are independently selected from hydrogen, (C1-C3)alkyl, (C5-C6)cycloalkyl and (C1-C3)alkyloxycarbonyl or R13 and R14 are alkylenes, which together with the nitrogen to which they are attached form a four to six membered saturated ring.

8. A compound according to claim 7, wherein R12 is hydrogen.

9. A compound according to claim 7, wherein Q represents a group Q2: 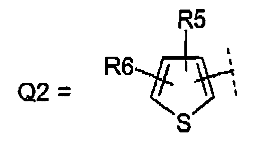 wherein:
R5 and R6 are as defined in claim 1;
and wherein the bond crossed by the dotted line indicates the attachment position.

10. A compound according to claim 9, wherein R5 and R6 are independently selected from hydrogen, fluoro, chloro, bromo, iodo, (C1-C3)alkyl, (C1-C3)alkyloxy, methylthio, phenyl.

11. A compound according to anyone of the preceding claims, wherein Q is a five membered heteroaryl selected from among furanyl, pyrrolyl, thienyl, pyrazolyl, imidazolyl, oxazolyl, isoxazolyl, thiazolyl, isothiazolyl and triazolyl, which is annelated as described in claim 1.

12. A compound according to anyone of claims 1 or 11, wherein Q is a thienyl, furanyl, pyrrolyl, or pyrazolyl, which carries one substituent R5, and which is annelated to a second ring selected from the group phenyl, thienyl, pyridyl, which is substituted with one or two residues R8 and R9, wherein R5, R8 and R9 are independently selected from hydrogen, hydroxyl, formyl, cyano, oxime, (C1-C4)alkyl, (C2-C4)alkenyl, (C2-C4)alkynyl, (C1-C4)alkyloxy, (C1-C4)alkylthio, carboxy, (C1-C3)alkylcarbonyl, (C1-C3)alkyloxycarbonyl, chloro, bromo, iodo, hydroxymethyl, trifluoromethyl, (C1-C4)alkylaminocarbonyl, di(C1-C5)alkylaminocarbonyl, nitro, and NR13R14, wherein R13 and R14 are independently selected from hydrogen, (C1-C3)alkyl, (C5-C6)cycloalkyl and (C1-C3)alkyloxycarbonyl or R13 and R14 are alkylenes, which together with the nitrogen to which they are attached form a four to six membered saturated ring.

13. A compound according to anyone of claims 1, 6, 11 or 12, wherein Q is thienyl or furanyl which is annelated to a second aromatic or heteroarylic ring, and forming a ring system selected from one of the formula Q3-Q5: 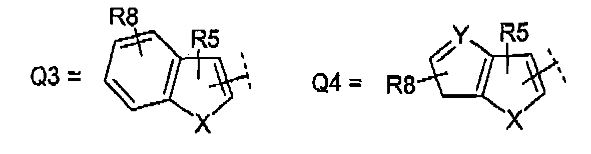 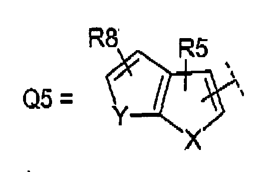 wherein:
X is S or O;
R5 and R8 are as described in claim 1 or 12; and
Y is S.

14. A compound according to claim 12, wherein Q is a thienyl or furanyl which is annelated with a phenyl and in which Q is attached in position 3 of the heteroarene, thus forming a ring system according to formula Q6: 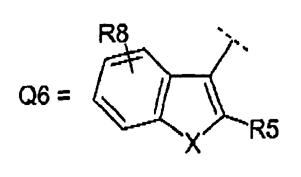 wherein:
X is S or O;
R5 and R8 are as described in claim 1 or 12.

15. A compound, according to anyone of claims 13 or 14, wherein R5 and R8 are independently selected from hydrogen, fluoro, chloro, bromo.

16. A compound according to anyone of claims 1 or 11, wherein Q is a thienyl or furanyl, which carries one substituent R5, and which is annelated to a second ring selected from the group cyclopentyl, cyclohexyl, or a saturated five or six membered ring which contains one or more heteroatoms selected from N, S and O, wherein said N is substituted with R12, and wherein
said second ring carries one substituent R10;
R5 is selected from hydrogen, hydroxyl, formyl, cyano, oxime, (C1-C4)alkyl, (C2-C4)alkenyl, (C2-C4)alkynyl, (C1-C4)alkyloxy, (C1-C4)alkylthio; carboxy, (C1-C3)alkyloarbonyl, (C1-C3)alkyloxycarbonyl, chloro, bromo, iodo, hydroxyethyl, trifluoromethyl, (C1-C4)alkylaminocarbonyl, di(C1-C4)alkylaminocarbonyl, nitro, and NR13R14;
R10 is selected from the group consisting of hydrogen, hydroxyl, (C1-C3)alkyl, phenyl, halogen, NR13R14 and oxo, wherein each alkyl may be unsustituted or substituted with one or more residues selected from among hydroxyl, (C1-C3)alkyloxy, halogen, and NR13R14
R12 is selected from hydrogen, (C1-C5)alkyl, phenyl(C1-C3)alkyl, (C1-C5)alkylcarbonyl, phonylcarbonyl and phenylsulfonyl; and
R13 and R14 are independently selected from hydrogen, (C1-C3)alkyl, (C5-C6)cycloalkyl and (C1-C3)alkyloxycarbonyl or R13 and R14 are alkylenes, which together with the nitrogen to which they are attached form a four to five membered saturated ring.

17. A compound according to anyone of claims 1, 6 or 16, wherein Q is a thienyl or furanyl which is annelated to a second ring, thus forming a ring system according to formula Q7. 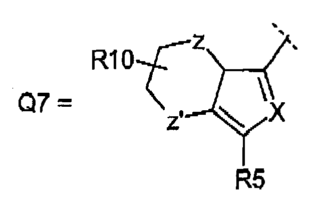 wherein:
X is S or O;
R5 and R10 are independently substituted as described in claim 1 or 16;
Z and Z' are independently CH₂ or O.

18. A compound according to claim 1, selected from:
- [4-Fluoro-4-[(6-methylpyridin-2-ylmethyl)amino]methyl]piperidin-1-yl]-thien-2-yl-methanone
- [4-Fluoro-4-[(5-methylpyridin-2-ylmethyl)amino]methyl]piperidin-1-yl]-5-fluorothien-2-yl-methanone
- 3-Chlorothion-2-yl-[4-fluoro-4-[[(5-methylpyridin-2-ylmethyl)amino]methyl]-piperidin-1-yl]methanone
- 6-Chlorothien-2-yl-[4-fluoro-4-[[(5-methylpyridin-2-ylmethyl)amino]methyl]-piperidin-1-yl]-methanone
- 3-Bromothien-2-yl-[4-fluoro-4-[[(5-methylpyridin-2-ylmethyl)amino]methyl]-piperidin-1-yl]-methanone
- 4-Bromothien-2-yl-[4-fluoro-4-[[(5-methylpyridin-2-ylmethyl)amino]methyl]-piperidin-1-yl]-methanone
- 5-Bromothien-2-yl-[4-fluoro-4-[[(5-methylpyridin-2-ylmethyl)amino]methyl]-piperidin-1-yl]-methanons
- 4,5-Dibromothien-2-yl-[4-fluoro-4-[[(5-methylpyridin-2-ylmethyl)amino]methyl]-piperidin-1-yl]-methanone
- [4-Fluoro-4-[[(5-methylpyridin-2-ylmethyl)amino]methyl]piperidin-1-yl]-3-methylthien-2-yl-methanone
- [4-Fluoro-4-[[(5-methylpyridin-2-ylmethyl)amino]methyl]piperidin-1-yl]-4-methylthien-2-yl-methanone
- [4-Fluoro-4-[[(5-methylpyridin-2-ylmethyl)amino]methyl]piperidin-1-yl]-5-methylthiophen-2-yl-methanone
- [4-Fluoro-4-[[(5-methylpyridin-2-ylmethyl)amino]methyl]piperidin-1-yl]-5-methylthiothien-2-yl-methanone
- [4-Fluoro-4-[[(5-methylpyridin-2-ylmethyl)amino]methyl]piperidin-1-yl]-5-phenylthien-2-yl-methanone
- [4-Fluoro-4-[[(5-methylpyridin-2-ylmethyl)amino]methyl]piperidin-1-yl]-5-pyridin-2-ylthien-2-yl-methanone
- [2,2']Bithien-5-yl-[4-fluoro-4-[[(5-methylpyridin-2-ylmethyl)amino]methyl]-piperidin-1-yl]-methanone
- [4-Fluoro-4-[[(5-methylpyridin-2-ylmethyl)amino]methyl]piperidin-1-yl]-[5-(2-methylthiazol-4-yl)thien-2-yl]-methanone
- [4-Fluoro-4-[[(5-methylpyridin-2-ylmethyl)amino]methyl]piperidin-1-yl]-3-pyrrol-1-ylthien-2-yl-methanone
- [4-Fluoro-4-[[(5-methylpyridin-2-ylmethyl)amino]methyl]piperidin-1-yl]-thien-3-yl-methanone
- 2-Bromothien-3-yl-[4-fluoro-4-[[(5-methylpyridin-2-ylmethyl)amino]methyl]-piperidin-1-yl]-methanone
- [4-Fluoro-4-[[(5-methylpyridin-2-ylmethyl)amino]methyl]piperidin-1-yl]-4-methoxythien-3-yl-methanone
- 5-Chloro-4-methoxythien-3-yl-[4-fluoro-4-[[(5-methylpyridin-2-ylmethyl)amino]methyl]piperidin-1-yl]-methanone
- 5-Bromo-4-methoxythien-3-yl-[4-fluoro-4-[[(5-methylpyridin-2-ylmethyl)amino]methyl]piperidin-1-yl]-methanone
- Benzo[b]thien-2-yl-[4-fluoro-4-[[(5-methylpyridin-2-ylmethyl)amino]methyl]-piperidin-1-yl]-methanone
- 5-Bromobenzo[b]thien-2-yl-[4-fluoro-[[(5-methylpyridin-2-ylmethyl)amino]methyl]piperidin-1-yl]-methanone
- Benzo[b]thien-3-yl-[4-fluoro-4-[[(5-methylpyridin-2-ylmethyl)amino]methyl]piperidin-1-yl]-methanone
- [4-Fluoro-4-[[(5-methylpyridin-2-ylmethyl)amino]methyl]piperidin-1-yl]-thieno[3,2-b]thien-2-yl-methanone
- [4-Fluoro-4-[[(5-methylpyridin-2-ylmethyl)amino]methyl]piperidin-1-yl]-thieno[2,3-b]thien-2-yl-methanone
- 3-Chlorothieno[2,3-b]thien-2-yl-[4-fluoro-4-[[(5-methylpyridin-2-ylmethyl)amino]methyl]piperidin-1-yl]-methanone
- [4-Fluoro-4-[[(5-methylpyridin-2-ylmethyl)amino]methyl]piperidin-1-yl]-(4,5,6,7-tetrahydrobenzo[c]thien-1-yl)-methanone
- 7-Bromo-2,3-dihydrothieno[3,4-b][1,4]dioxin-5-yl-[4-fluoro-4-[[(5-methylpylidin-2-ylmethyl)amino]methyl]piperidin-1-yl]-methanone
- [4-Fluoro-4-[[(5-methylpyridin-2-ylmethyl)amino]methyl]piperidin-1-yl]-furan-2-yl-methanone
- 4,5-Dibromofuran-2-yl-[4-fluoro-4-[[5-methylpyridin-2-ylmethyl)aminolmethyl]piperidin-1-yl]-methanone
- [4-Fluoro-4-[[5-methylpyridin-2-ylmethyl)amino]methyl]piperidin-1-yl]-furan-3-yl-methanone
- 5-Bromofuran-3-yl-[4-fluoro-4-[[(5-methylpyridin-2-ylmethyl)amino]methyl]piperidin-1-yl]-methanone
- Benzo[b]furan-2-yl-[4-fluoro-4-[[(5-methylpyridin-2-ylmethyl)amino]methyl]piperidin-1-yl]-methanone
- Benzo[b]furan-3-yl-[4-fluoro-4-[[(5-methylpyridin-2-ylmethyl)amino]methyl]piperidin-1-yl]-methanone
- [4-Fluoro-4-[[(5-methylpyridin-2-ylmethyl)amino]methyl]piperidin-1-yl]-pyrrol-2-yl-methanone
- [4-Fluoro-4-[[(5-methylpyridin-2-ylmethyl)amino]methyl]piperidin-1-yl]-pyrazol-3-yl-methanone
- [4-Fluoro-4-[[(5-methylpyridin-2-ylmethyl)amino]methyl]piperidin-1-yl]-indol-2-yl-methanone
- [4-Fluoro-4-[[(5-methylpyridin-2-ylmethyl)amino]methyl]piperidin-1-yl]-pyrazolo[1,5-a]pyridin-2-yl-methanone
- [4-Fluoro4-[[(6-methylpyridin-2-ylmethyl)amino]methyl]piperidin-1-yl]-pyrazolo[1,5-a]pyridin-3-yl-methanone

19. A compound according to any one of the preceding claims as a medicine.

20. Pharmaceutical composition comprising at least one of the compounds according to anyone of claims 1-18, and a pharmaceutically acceptable carrier.

21. Use of a compound according to any one of claims 1-18 for the production of a medicament for the prevention and/or treatment of pain, depression, or anxiety disorders.

22. A compound according to any one of claims 1-19 as a medicine for the prevention and/or treatment of pain, depression, or anxiety disorders.
